(19) Europäisches Patentamt
European Patent Office
Office européen des brevets

(11) Publication number: 0 335 369 A2

# (12) EUROPEAN PATENT APPLICATION

(21) Application number: 89105540.2

(51) Int. Cl.4: C07H 15/252 , A61K 31/70

(22) Date of filing: 29.03.89

(30) Priority: 29.03.88 JP 73212/88
26.05.88 JP 126961/88

(43) Date of publication of application:
04.10.89 Bulletin 89/40

(84) Designated Contracting States:
DE FR GB IT

(71) Applicant: ZAIDAN HOJIN BISEIBUTSU KAGAKU KENKYU KAI
14-23, Kami Ohsaki 3-chome
Shinagawa-ku Tokyo(JP)

(72) Inventor: Takeuchi, Tomio
1-11, Higashi Gotanda 5-Chome
Shinagawa-Ku Tokyo(JP)
Inventor: Umezawa, Sumio
1-1-709, Shinjuku 5-Chome
Shinjuku-Ku Tokyo(JP)
Inventor: Tsuchiya, Tsutomu
1-17-201, Eda Higashi 3-Chome Midori-ku
Yokohama-shi Kanagawa-ken(JP)
Inventor: Umezawa, Kazuo
1-2-505, Hiroo 3-Chome Shibuya-ku
Tokyo(JP)
Inventor: Takagi, Yasushi Cl Mansion Higashi Totsuka
No. 512, 412-1, Kawakami-cho Totsuka-ku
Yokohama-shi Kanagawa-ken(JP)
Inventor: Miyake, Toshiaki
5-1-207, Shimoda-cho 3-Chome Kohoku-ku
Yokohama-shi Kanagawa-ken(JP)

(74) Representative: Popp, Eugen, Dr. et al
MEISSNER, BOLTE & PARTNER
Widenmayerstrasse 48 Postfach 86 06 24
D-8000 München 86(DE)

## (54) New anthracycline derivatives and processes for the preparation of the same.

(57) As new anthracycline derivatives are now produced 7-O-(2,6-dideoxy-2-fluoro-α-L-mannopyranosyl)-daunomycinone and -adriamycinone, their 4-demethoxy derivatives and 14-O-ester derivatives of the adriamycinone compounds of them, as well as 4-demethoxy-7-O-(2,6-dideoxy-2-fluoro-α-L-talopyranosyl)-daunomycinone and -adriamycinone and 14-O-ester derivatives of the adriamycinone compound thereof which exhibit excellent antitumor activities and are useful as antitumor agents. These new anthracycline derivatives may be produced by various processes.

EP 0 335 369 A2

# New anthracycline derivatives and processes for the preparation of the same

## SUMMARY OF THE INVENTION

This invention relates to new anthracycline derivatives having antitumor activities and pharmaceutcal antitumor composition containing a new anthracycline derivative as the active ingredient. More particularly, this invention relates to new compounds having antitumor activities which include 7-O-(2,6-dideoxy-2-fluoro-α-L-mannopyranosyl)-daunomycinone and -adriamycinone, their 4-demethoxy derivatives and 14-O-alkanoic acid esters thereof, as well as 4-demethoxy-7-O-(2,6-dideoxy-2-fluoro-α-L-talopyranosyl)-daunomycinone and -adriamycinone and their 14-O-alkanoic acid esters thereof. This invention also relates to pharmaceutical compositions containing at least one of these new anthracycline derivatives as an active ingredient. This invention further relates to processes for the preparation of these new anthracycline derivatives. This invention also includes a method of inhibitingly treating tumor cells with at least one of these new anthracycline derivatives.

## BACKGROUND OF THE INVENTION

Known examples of antibiotics of the anthracycline type include daunomycin which is referred to in the same of daunorubicin in U.S. Patent No. 3,616,242, and adriamycin which is referred to in the name of doxorubicin in U.S. Patent No. 3,590,028. These particular compounds exhibit a broad antitumor spectrum against experimental tumors and have found wide-spread clinical utility as chemotherapeutic antitumor agents. Daunomycin and adriamycin are compounds of the following formula

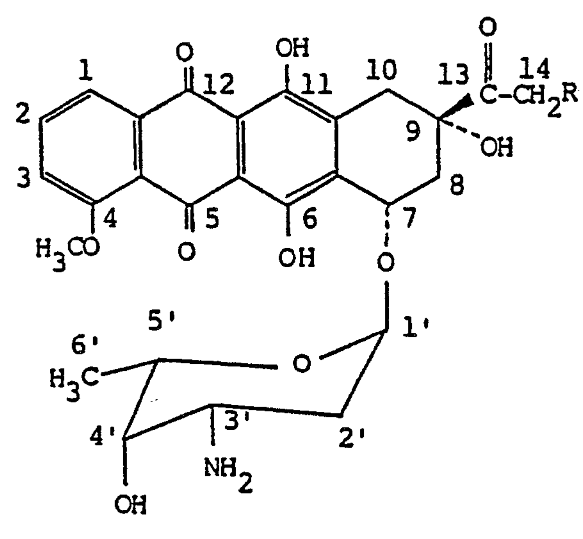

(a)

wherein R is a hydrogen atom or a hydroxyl group.

Daunomycin which is the compound of the formula (a) where R is a hydrogen atom, and adriamycin which is the compound of the formula (a) where R is a hydroxyl group show relatively high antitumor activities against various kinds of tumors, although they are not fully satisfactory. Namely, daunomycin and adriamycin have a broad antitumor spectrum against experimental tumors and have been used widely as chemotherapeutic agents for the treatment of tumor-bearing patients. However, they are also known to bring about serious adverse side-effects such as leukocytopenia, alopecia and myocardiopathy, in many instances. It has hence been attempted to prepare various compounds analogous to daunomycin for the purpose of providing such novel daunomycin analogues which possess higher anticancer activities and lower toxicity. These attempts have already lead to the proposal of some compounds, including aclacinomycins A and B (F. Arcamone, "Topics in Antibiotic Chemistry", Vol. 2, pp. 102-279, published from Elis Horwood Limited, U.S.A., and U.S. Patent No. 3,988,315), 4′-O-tetrahydropyranyl-adriamycin (West German Patent No. 2,831,579 and Japanese Patent Publication No. 47194/81) and N-mono-benzyl- or N-dibenzyl-adriamycin (U.S. Patent No. 4,177,264).

Further, U.S. Patent No. 4,427,664 discloses 7-O-(3,4-di-O-acetyl-2,6-dideoxy-2-iodo-α-L-mannohex-

opyranosyl)daunomycinone (NSC 331,962) and 7-O-(3,4-di-O-acetyl-2,6-dideoxy-2-iodo-α-L-talohexopyranosyl )daunomycinone (NSC 327,472). Besides, European patent application publications No. 116,222 Al (corresponding to U.S. patent application SN. 450,863 of which a continuation-in-part application is U.S. patent application SN. 623,741 whose continuation application is U.S. patent application SN. 42,624, now granted under U.S. patent No. 4,772,688 of Horton et al issued 20 September 1988) discloses the following compounds:-

14-O-Acetyl-7-O-(3,4-di-O-acetyl-2,6-dideoxy-2-iodo-α-L-manno-hexopyranosyl)adriamycinone;
14-O-Acetyl-7-O-(3,4-di-O-acetyl-2-bromo-2,6-dideoxy-α-L-talo-hexopyranosyl)adriamycinone;
14-O-Acetyl-7-O-(3,4-di-O-acetyl-2,6-dideoxy-2-iodo-α-L-talo-hexopyranosyl)adriamycinone;
14-O-Acetyl-7-O-(3,4-di-O-acetyl-2-bromo-2,6-dideoxy-α-L-manno-hexopyranosyl)adriamycinone;
14-O-Acetyl-7-O-(3,4-di-O-acetyl-2-chloro-2,6-dideoxy-α-L-manno-hexopyranosyl)adriamycinone;
7-O-(3,4-di-O-acetyl-2,6-dideoxy-2-iodo-α-L-manno-hexopyranosyl)-14-O-(5-carboxypentanoyl)-adriamycinone.

We, the present inventors, have proceeded with investigations in an attempt to provide new daunomycin or adriamycin derivatives which have better antitumor activities and lower toxicity than daunomycin or adriamycin. As a part of the outcome of our investigations, we have already succeeded in synthesizing some daunomycin and adriamycin derivatives having antitumor activities by chemically modifying the sugar moiety of daunomycin and adriamycin. For example, the present inventors have already reported 4′-O-tetrahydropyranyl-daunomycins and -adriamycins in Japanese Patent Publication No. 47194/81 as well as 3′-deamino-3′-morpholino-daunomycins and -adriamycins in Japanese Patent Application First Publication "Kokai" No. 163393/82.

In the meantime, the present inventors succeeded in synthesizing, as compounds having antitumor activities, certain anthracycline derivatives represented by the general formula

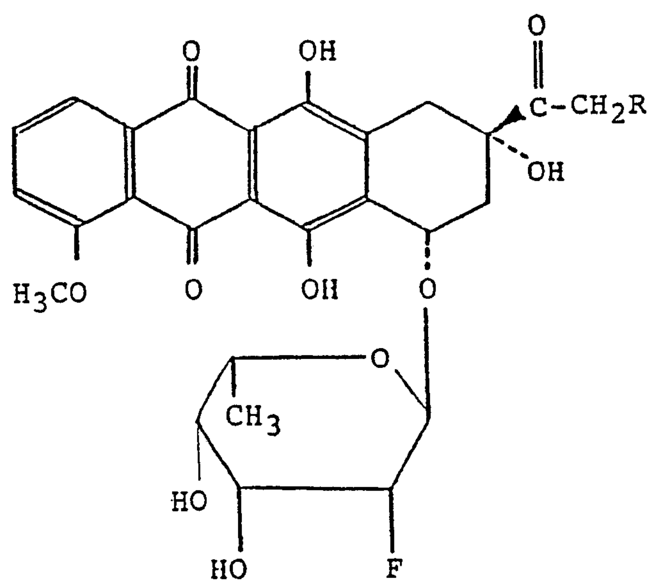

(b)

wherein R means a hydrogen atom or a hydroxyl group, for example, 7-O-(2,6-dideoxy-2-fluoro-α-L-talopyranosyl)daunomycinone and 7-O-(2,6-dideoxy-2-fluoro-α-L-talopyranosyl)adriamycinone (Japanese Patent Application No. 282798/85; Japanese Patent Application First Publication "Kokai" No. 145097/87; European Patent Application No. 86.117662.6; U.S. Patent Application Serial No. 942,773 of which a continuation application is U.S. Patent Application SN. 244,652).

The present inventors also succeeded in synthesizing, as novel adriamycin analogues having antitumor activities, anthracycline derivatives represented by the general formula

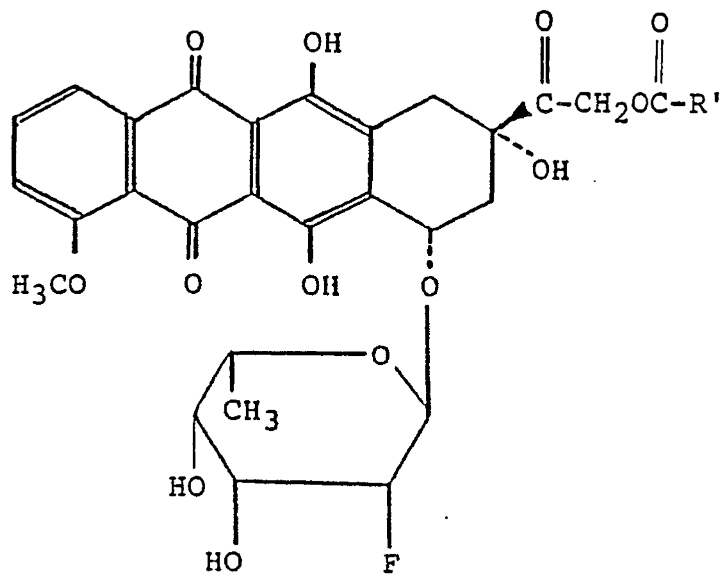

(c)

wherein R′ means a group $-(CH_2)_mH$, where m is an integer of 1-6, or another group $-(CH_2)_n-COOH$, where n is an integer of 1-10 (Japanese Patent Application No. 288993/86; U.S. Patent Application Serial No. 128,173 filed December 3, 1987 and European Patent Application No. 87.117791).

However, the above anthracycline derivatives which have been synthesized by the present inventors and are represented by the formula (b) or (c) are somewhat complicated in their methods of synthesis. The present inventors therefore conducted different investigations in an attempt to produce novel anthracycline derivatives which have superior antitumor activities to the compounds of the formula (b) or (c) and which can be prepared by a simpler synthetic process.

The antitumor activities of the novel compounds of the formula (b) or (c) are remarkedly superior to those of daunomycin and adriamycin (see Japanese Patent Application No. 282798/85; Japanese Patent Application First Publication "Kokai" No. 145097/87). The present inventors proceeded with an investigation in order to provide such anthracycline derivatives which can exhibit antitumor activities higher than those of the compounds of the formula (b) or (c) and can be synthesized more easily than such compounds of the formula (b) or (c). As a result, we have succeeded in synthesizing anthracycline derivatives represented by the general formual

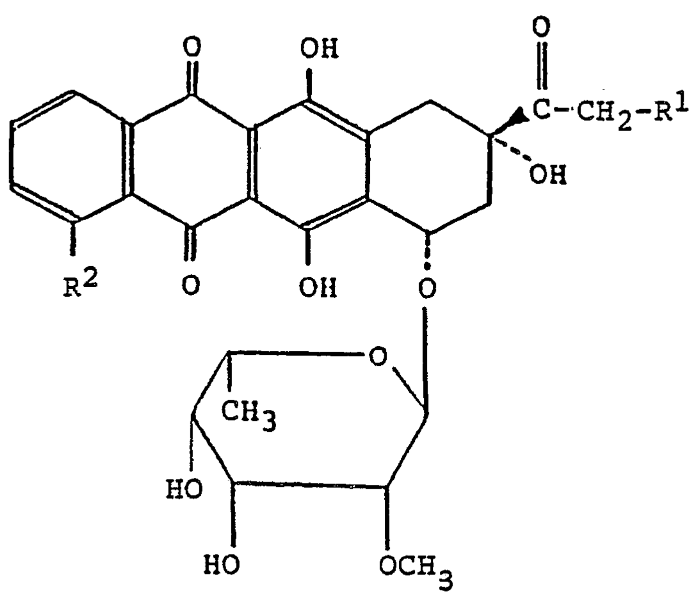

(d)

wherein $R^1$ is a hydrogen atoms or a hydroxyl group or a group of the formula

$-O\overset{O}{\overset{\|}{C}}-(CH_2)_n-COOH$

where n is an integer of 1-6, and $R^2$ is a methoxy group or a hydrogen atom, and their salts (Japanese Patent Application No. 15783/88 filed January 28, 1988 and assigned commonly to the present assignee).

In addition to the investigations mentioned above, the present inventors also conducted a still further investigation in an attempt to provide novel anthracycline derivatives having excellent carcinostatic effects and improved solubility in water by modifying the compounds of the above formula (b). As a result, the present inventors succeeded in synthesizing certain novel anthracycline derivatives having such a formula (e) as described below. It has also been found that when the novel compounds of the formula (e) are converted into their acid addition salts, these acid addition salts show an enhanced solubility in water and thus may be formulated readily into injectionable solutions and find advantageous clinical applications and that the compounds of formula (e) themselves exhibit high carcinostatic effects for various kinds of human cancer cells.

Thus, the present inventors also provided anthracycline derivatives represented by the general formula

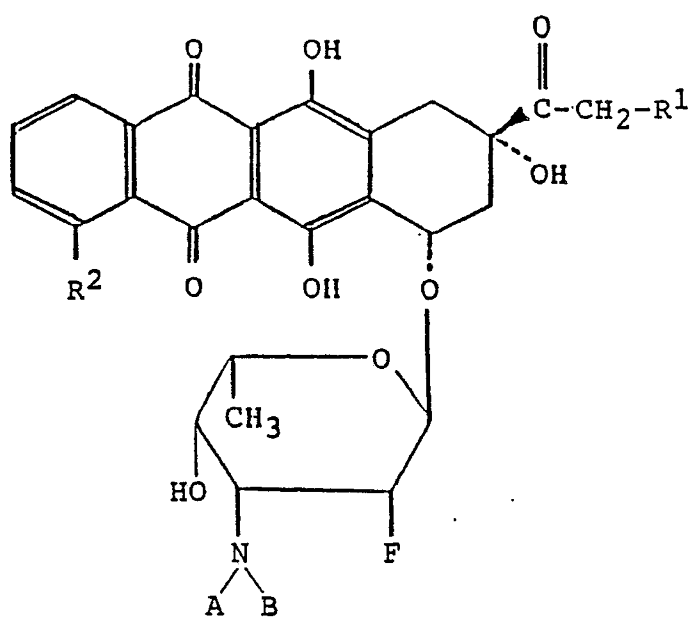

(e)

wherein $R^1$ is a hydrogen atom or a hydroxyl group, $R^2$ is a methoxy group or a hydrogen atom, and A and B are individually a hydrogen atom, or A and B taken together form a chain of the formula $-CH_2-CH_2-O-CH_2-CH_2-$, and acid addition salts thereof (Japanese Patent Application No. 27443/88 filed February 10, 1988).

Further investigations have now been conducted by us for the purpose of providing another, excellent novel anthracycline derivatives, in addition to the above-mentioned anthracycline derivatives having any one of the formulae (b)-(e) which have been synthesized by the present inventors.

## DETAILED DESCRIPTION OF THE INVENTION

The antitumor activities of the above-mentioned novel compounds of the formula (b)-(e) are remarkably higher than those of daunomucin and adriamycin (Japanese Patent Application No. 282798/85; Japanese Patent Application First Publication "Kokai" No. 145097/87). Now, we have made extensive investigations with an object to provide such novel anthracycline derivatives which can maintain and exhibit antitumor activities as high as those of the compounds of the formula (b)-(e) even when they are administered at low doses. As a result, we have now succeeded in producing novel anthracycline derivative compounds having a general formula (I) as given below. We have also now found that the novel anthracycline derivatives of the general formula (I) according to this invention can achieve the objects desired in this invention.

In a first aspect of this invention, there is thus provided anthracycline derivatives represented by the general formula

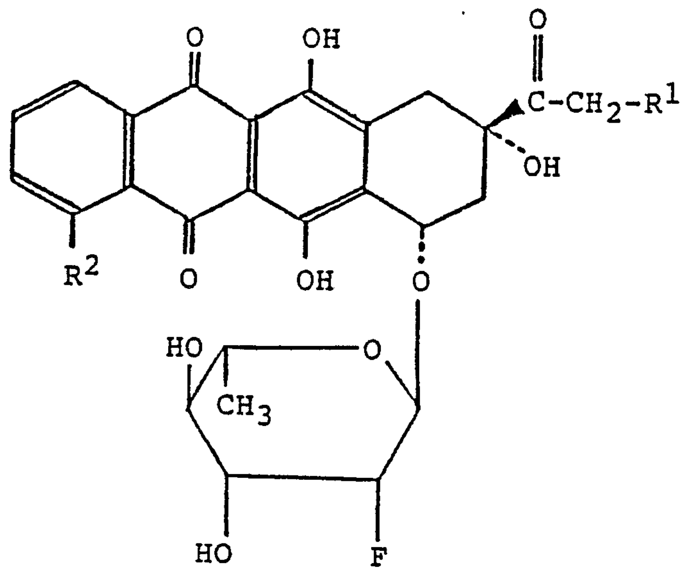

(I)

wherein $R^1$ is a hydrogen atom or a hydroxyl group or is a group having the formula

$$-O\overset{\displaystyle O}{\overset{\|}{C}}-(CH_2)_n-COOH$$

where n denotes an integer of 1 to 10, and $R^2$ is a methoxy group or a hydrogen atom, or a salt thereof.

The compounds of the general formula (I) according to this invention may largely be classified into the following three classes (A), (B) and (C):

(A) 7-O-(2,6-dideoxy-2-fluoro-α-L-mannopyranosyl)daunomucinone and 7-O-(2,6-dideoxy-2-fluoro-α-L-mannopyranosyl)-4-demethoxydaunomycinone represented by the general formula

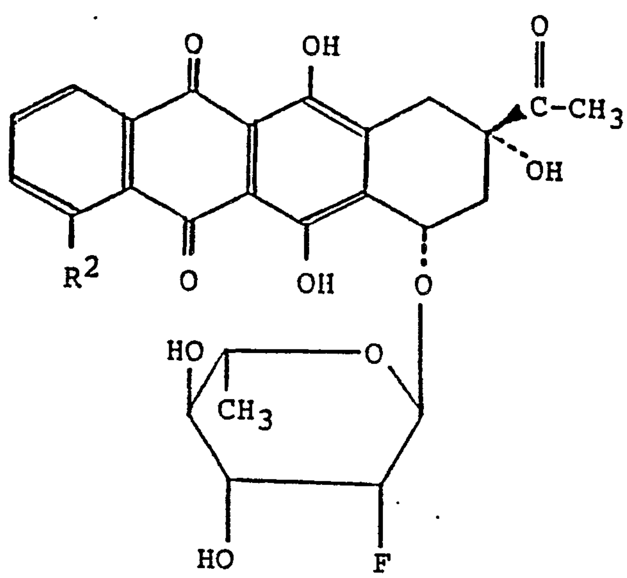

(Ia)

where $R^2$ has the same meaning as defined above;

(B) 7-O-(2,6-dideoxy-2-fluoro-α-L-mannopyranosyl)adriamycinone and 7-O-(2,6-dideoxy-2-fluoro-α-L-mannopyranosyl)-4-demethoxyadriamycinone represented by the general formula

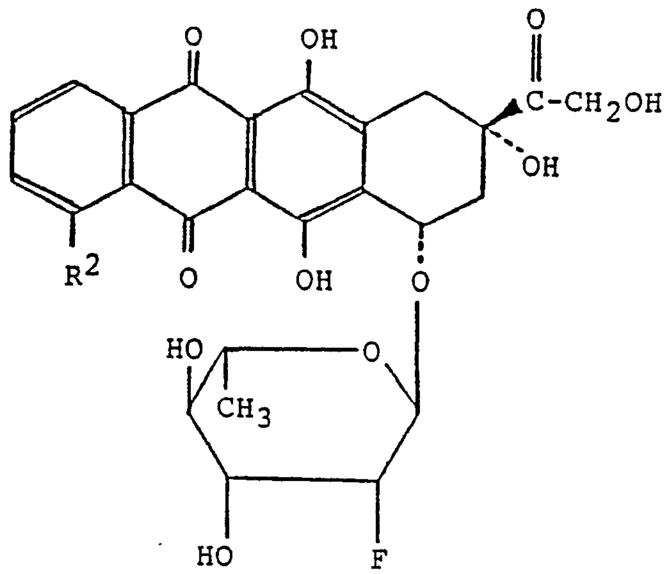

(Ib)

where $R^2$ has the same meaning as defined above; and

(C) Alkanoic acid half-ester derivatives of adriamycin analogues represented by the general formula

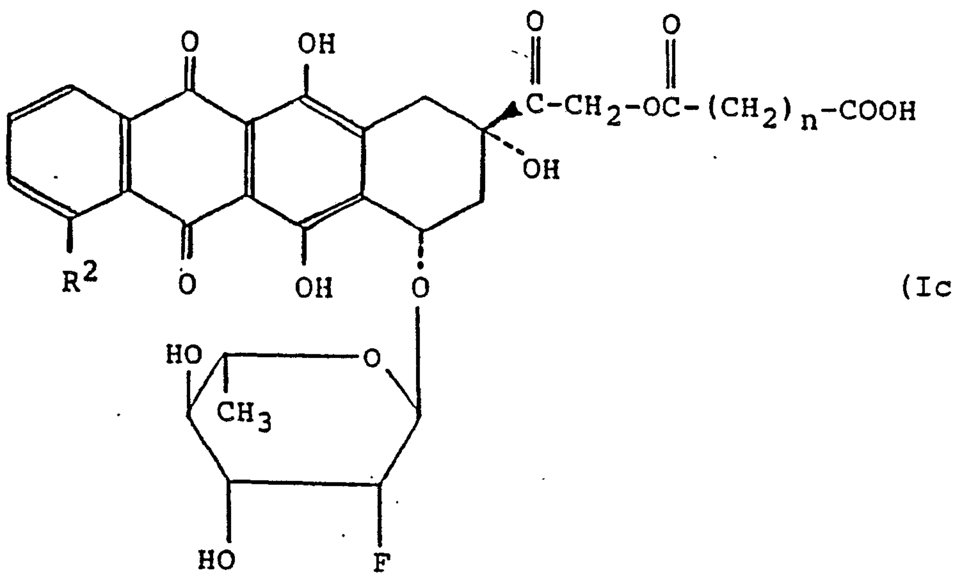

(Ic)

wherein $R^2$ is a methoxy group or a hydrogen atom and n is an integer of 1 to 10, or a salt thereof.

The names of specific examples of the compounds having the general formula (I) according to this invention and their physical properties will be described later in Examples 1 to 11 given hereinafter.

It has been confirmed through some tests that the compounds of the general formula (I) of the present invention have markedly high antitumor activities against experimental tumors in animals and their antitumor activities are remarkably higher than those of daunomycin and adriamycin. It has also been found that the compounds of the formula (I) exhibit high antitumor activities even at their low doses. In addition, the compounds of the general formula (I) have high antibacterial activities, too and are also useful as antibacterial agent. A description will next be made of tests on the antitumor activities of some particular compounds of the general formula (I).

Test 1:

Antitumor activities against leukemia in $CDF_1$ mice as induced by mouse leukemia, Leukemia L-1210 cells

To evaluate the antitumor effects of the compounds of this invention against experimental tumors in animals, $CDF_1$ mice were intraperitoneally transplanted with cells of Leukemia L-1210 (at a does of 1 x $10^5$ cells/mouse). After an elapsed time of 24 hours, a compounds of the formula (I) of this invention to be tested was administered intraperitoneally for 9 consecutive days once per day. Observation was made for 60 days, where survival days of each mouse tested were recorded. In comparison with the control test wherein mice were treated with the administration of physiological saline alone, the increase (%) in the life-span of the treated mice was calculated as T/C %, where T is the mean survival days of the treated animals and C is the mean survival days of the untreated control animals. For the comparison, daunomycin and adriamycin were also tested in the same manner. The results are summarized in Table 1.

Table 1

| Compound tested | % Increase in life-span (T/C, %) | | | | | | | | | | |
|---|---|---|---|---|---|---|---|---|---|---|---|
| | Dose (mg/kg/day) | | | | | | | | | | |
| | 10 | 5 | 2.5 | 1.25 | 0.6 | 0.3 | 0.15 | 0.08 | 0.05 | 0.025 | 0.013 |
| 7-0-(2,6-Dideoxy-2-fluoro-α-L-mannopyranosyl) daunomycinone (Compound of Example 1) | | 160* | 280 | >309 | 189 | 123 | 109 | | | | |
| 7-0-(2,6-Dideoxy-2-fluoro-α-L-mannopyranosyl)-4-demethoxy-daunomycinone (Compound of Example 2) | | 73* | 134* | >686* | 309 | 163 | 113 | | | | |
| 7-0-(2,6-Dideoxy-2-fluoro-α-L-mannopyranosyl) adriamycinone (Compound of Example 3) | | 211* | 231 | >426 | >363 | >440 | 157 | | | | |
| 7-0-(2,6-Dideoxy-2-fluoro-α-L-mannopyranosyl)-4-demethoxy-adriamycinone (Compound of Example 4) | | 22* | 53* | 141* | >686 | >686 | >534 | | | | |
| 7-0-(2,6-Dideoxy-2-fluoro-α-L-mannopyranosyl) adriamycinone 14-0-hemipimelate (Compound of Example 9) | | >463 | >686 | >346 | >329 | 274 | 123 | | | | |
| 7-0-(2,6-Dideoxy-2-fluoro-α-L-mannopyranosyl)-4-demethoxy-adriamycinone 14-0-hemipimelate (Compound of Example 11) | | - | - | 111* | 175 | >363 | 121 | | | | |
| Daunomycin (comparative drug) | | 117* | 151* | 193 | 166 | 133 | 130 | | | | |
| Adriamycin (comparative drug) | | 191* | 228 | 222 | 142 | 136 | 123 | | | | |
| 7-0-(2,6-Dideoxy-2-fluoro-α-L-talopyranosyl) daunomycinone (Reference) | | 184 | 217 | 171 | 118 | 105 | 105 | | | | |
| 7-0-(2,6-Dideoxy-2-fluoro-α-L-talopyranosyl) adriamycinone (Reference) | | >750 | >350 | 275 | 185 | 182 | 127 | | | | |

In Table 1, asterisks (*) indicate that the development of toxicity such as toxicity-related death or a weight loss was observed on the corresponding mice tested.

Adriamycin employed as a comparative drug in Test 1 above is a carcinostatic agent which is actually used in clinical treatments. It is administered to men at doses in a range of from 0.4 mg/kg to 2 mg/kg depending on the types of cancers to be treated. When adriamycin is administered at a does of from 2.5mg/kg/day to 5 mg/kg/day to the mice which has been inoculated with L-1210 cells, adriamycin as a conventional carcinostatic agent exhibits the antitumor effects such that the rate (%) of increase in life-span (T/C, %) amounts to about 228-191%, and adriamycin can then be accompanied by development of toxicity (see the results of Table 1 above). In contrast, it should be worthy to note that the compounds of this invention as administered at a suitable low dose in a range of from 0.3 mg/kg/day to 2.5 mg/kg/day is able to bring about equal or even better rate (%) of increases in life-span (T/C, %) without development of toxicity and also that the compounds of the present invention can exhibit the antitumor effects at the very much low dose of 0.3 mg/kg/day. The compounds of this invention therefore have an advantage that the antitumor effects can be expected in clinical treatments also when they are administered not at a large dose to cancer-bearing patients.

Test 2:

Tumor cells of mouse leukemia P 388/S (sensitive) or P388/ADR (resistant) as the experimental tumor cells of mice were cultured in testing tubes. 7-O-(2,6-Dideoxy-2-fluoro-α-L-mannopyranosyl)adriamycinone 14-O-hemipimelate, which is a compounds according to this invention, was then added at varying concentrations. The incubation of the cells was thereafter continued for 72 hours to measure the 50% inhibitory concentration ($IC_{50}$, ng/mℓ) of the test compound against the growth of the tunmor cells. A similar test was also conducted using 7-O-(2,6-dideoxy-2-fluoro-α-L-talopyranosyl)-adriamycinone 14-O-hemipimelate as a referential compound. The test results are summarized in Table 2.

Table 2

| Compound tested | $IC_{50}$ (ng/mℓ) | |
|---|---|---|
| | P 388/S | P 388/ADR |
| The above indicated compound of this invention | 0.3 | 39.8 |
| Referential compound | 3.3 | 485 |

From the foregoing results, the novel anthracycline compound of the general formula (I) is considered to have excellent antitumor activities. Therefore, the compounds of this invention are expected to be extremely useful as an antitumor agent usable in actual clinical applications and also to be useful for the treatment of various tumors, like adriamycin.

As will be envisaged clearly from the results of the tests described above, the compounds of the general formula (I) provided by the present invention show excellent antitumor activities against experimental tumors such as leukemia cells L-1210 and leukemia cells P 388.

The compounds of the general formula (I) can therefore be used as therapeutic agents for malignant tumors, namely, for the treatment of solid cancers and ascitic cancers.

In a second aspect of this invention, there is also provided a pharmaceutical antitumor composition which comprises as an active ingredient an anthracycline derivative having the general formula (I), in association with a pharmaceutically acceptable liquid or solid carrier for the active ingredient.

Upon actual administration of the new compound of the general formula (I) according to this invention, the compound may generally be administered parenterally. However, it is also possible to administer the compound of this invention orally after the compound is formulated together with a pharmacologically-acceptable conventional solid or liquid carrier into a dosable preparation form such as powder, granules, tablets, syrup or injectionable solutions or suspensions.

For usual administration methods, the compound may be administered in the form of an injectionable solution or suspension by intraperitoneal injection, subcutaneous injection, blood vessel injection, either intravenous or intra-arterial, or local injection upon administration to animals, and in the form of an injectionable solution or suspension by blood vessel injection, either intravenous or intra-arterial, or local injection upon administration to men. The new compound of this invention may be administered either continuously or intermettently to an extent that the total dosage would not exceed a certain level as determined in view of results of animal tests and various circumstances. Needless to say, its administration

should be effected by varying the dosage of the compound of this invention in accordance with the manner of administration and the conditions of each patient or animal to be treated, for example, age, body weight, sex, sensitivity, foods, administration time, administration route, drugs to be administered in combination and the seriousness of disease, etc. As a guideline, the compound of this invention may be administered at substantially the same dose as adriamycin when used as an antitumor agent, namely, once a day at a dose in a range of from 0.3 mg/kg to 2.5 mg/kg. Optimum dosage and frequency of administration under certain specific conditions must be determined by medicinal experts through preliminary tests in view of the above-mentioned quideline. These requirements for administration are equally applied to oral administration of the new compound of this invention.

The new compounds of the general formula (I) according to this invention also exhibit antibacterial activities against Gram-positive bacteria. As therapeutic agnets for infections caused by Gram-positive bacteria, the compound of this invention can be administered in one of the preparation forms mentioned above and at the above-described dosage. Further, the frequency of administration, preparation form and the like can be determined by an artisan while paying the pre-cautions as described above.

Description will now be made in respect of the processes for the preparation of the compounds of the general formula (I) according to this invention.

Among the compounds of the general formula (I) according to this invention, such a compound of the formula (Ia), which is a compound of the formula (I) where $R^1$ is a hydrogen atom, including 7-O-(2,6-dideoxy-2-fluoro-α-L-mannopyranosyl(daunomycinone and 7-O(2,6-dideoxy-2-fluoro-α-L-mannopyranosyl)-4-demethoxydaunomycinone, can be synthesized by reacting the 7-hydroxyl group of daunomycinone or 4-demethoxydaunomycinone having the formula (II) given below with a glycosyl compound having the formula (III) given below, namely, 2,6-dideoxy-2-fluoro-α-L-mannopyranosyl halide or its hydroxyl-protected derivative, and where the resulting reaction product contains any remaining hydroxyl-protecting groups, then removing the hydroxyl-protecting groups from the reaction product.

In a third aspect of this invention, therefore, there is provided a process for the preparation of a daunomycin derivative represented by the formula

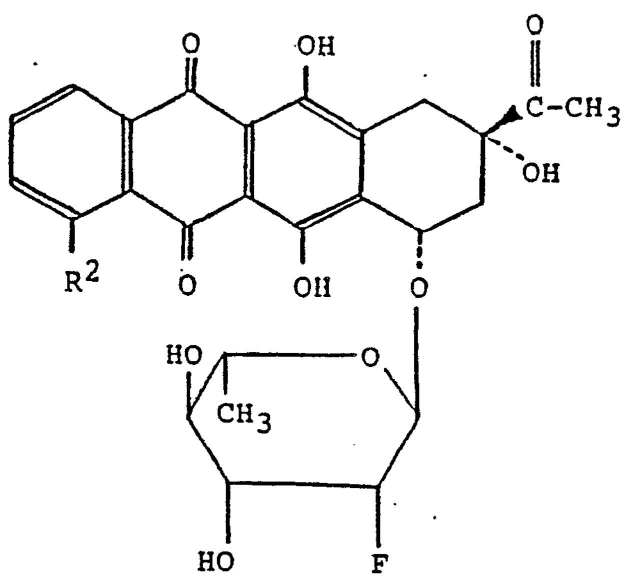

(Ia)

wherein $R^2$ is a methoxy group or a hydrogen atom, which comprises condensing daunomycinone or 4-demothoxy-daunomycinone represented by the formula

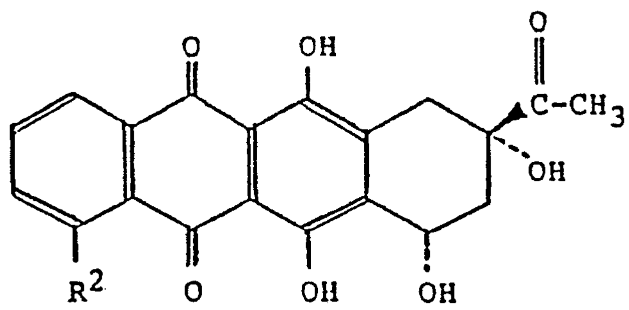

(II)

wherein $R^2$ has the same meaning as defined above, with a 2,6-dideoxy-2-fluoro-α-L-mannopyranosyl halide or a hydroxyl-protected derivative thereof represented by the formula

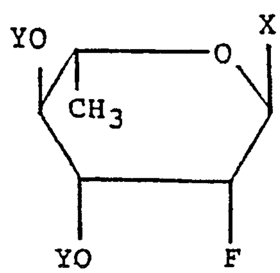

(III)

wherein X is a bromine, chlorine or iodine atom and Y is a hydrogen atom or a hydroxyl-protecting group, to form a compound represented by the formula

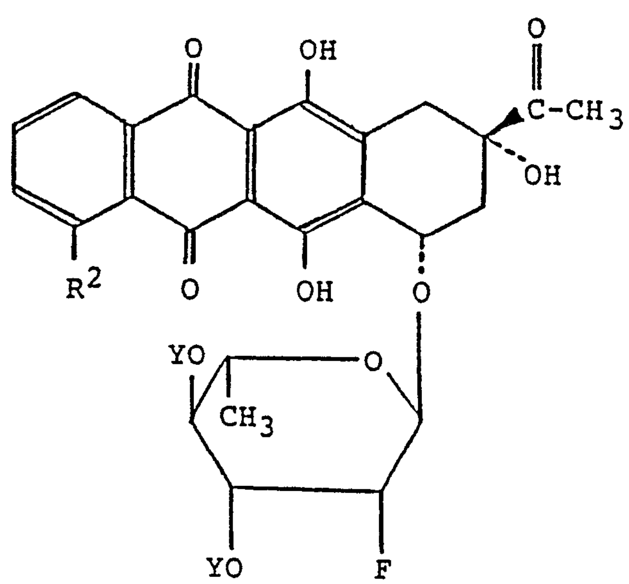

(Ia-1)

where $R^2$ and Y have the same meanings as defined above, and where the compound of the formula (Ia-1) contains the remaining hydroxyl-protecting groups (Y), then removing the hydroxyl-protecting groups (Y) from the compound of the formula (Ia) in a known manner.

In the process according to the third aspect of this invention, the reaction between daunomycinone or 4-demethoxydaunomycinone of the formula (II) and the 2,6-dideoxy-2-fluro-α-L-mannopyranose derivative of the formula (III) may be effected according to any known technique by which a sugar can be condensed with an aglycone through a glycoside linkage.

In the process according to the third aspect of this invention, the condensation reaction between the compound of the formula (II) and the compound of formula (III) may be effected usually in an aprotic organic solvent. Exemplary aprotic organic solvents useful in the practice of the condensation reaction may

include N,N-dimethylformamide (DMF), dimethylsulfoxide (DMSO), hexamethylphosphoryl triamide, glyme, tetrahydrofuran (THF), dioxane, diethyl ether, and various halogenated hydrocarbons, e.g., dichloromethane, trichloromethane and tetrachloroethane. Although these solvents may contain a little water, it is desirable to dehydrate them beforehand.

It is generally desirable to conduct the condensation reaction in the presence of a dehydrohalogenation agent which acts as a condensation catalyst, for example, a tertiary alkylamine such as triethylamine, a tertiary amine such as dimethylaniline, silver oxide, silver trifluoromethanesulfonate, silver carbonate, mercury oxide, mercury bromide or mercury cyanide.

Such a dehydrohaloganation agent may be used generally in an amount of at least 1 mole, preferably 2.5-4.0 moles per mole of the compound of the formula (III).

On the other hand, the compound of the formula (III) may desirably be used in an amount of 1 mole or a little excessively, for example, 1.5 moles per mole of the compound of the formula (II).

Although no particular limitation is imposed on the reaction temperature for the condensation, the reaction temperature generally may range from the freezing point of a solvent to be used to 80°C. The reaction can thus be conducted at a temperature in the vicinity of room temperature. It is preferable to conduct the condensation reaction between the compound of the formula (II) and that of the formula (III) in an aprotic organic solvent such as one of the aforementioned halogenated hydrocarbons, under anhydrous conditions, and in the presence of a condensation catalyst, for example, mercuric oxide or mercuric bromide and also in the presence of a molecular sieve as a dehydrating agent. The reaction product of the formula (Ia-1) as formed can be recovered from the reaction mixture by a method known per se in the art. The reaction product of the formula (Ia-1) thus recovered can be purified by chromatography on a silica gel column, using a toluene-ethyl acetate mixture or another mixed solvents as a developing solvent.

When the 3- and 4-hydroxyl groups of the mannose moiety of the glycosyl compound of the formula (III) used in the process according to the third aspect of this invention have been blocked by hydroxyl-protecting groups (Y), the hydroxyl-protecting groups (Y) remain in the resulting condensation product of the formula (Ia-1). Removal of the hydroxyl-protecting groups (Y) by a usual method can result in the formation of the target compound of the formula (Ia).

The hydroxyl-protecting groups (Y) which may optionally be present in the glycosyl compound of the formula (III) may be any conventional acyl groups, for example, acetyl or benzoyl groups. Such hydroxyl-protecting groups can be removed through a hydrolysis reaction which makes use of an acid or alkali. As the acid, a lower fatty acid such as formic acid or trifluoroacetic acid or an inorganic acid such as hydrochloric acid or sulfuric acid may be used, for example. On the other hand, illustrative examples of the alkali available for the above purpose may include NaOH, KOH, $Ba(OH)_2$ and the like. A solvent useful in the practice of this deprotecting reaction may be water or an alcohol, or mixed solvents of an aprotic solvent such as DMF, DMSO, dioxane or tetrahydrofuran with water or an alcohol. An aqueous alcohol may be employed usually. The reaction temperature for the deprotection may be at 0-100°C, usually, at 0-50°C.

The compound of the formula (Ia) obtained in the above way is in the form of a red solid and can be purified by reprecipitation or recrystallization from mixed organic solvents such as chloroform-hexane.

Among the glycosyl compounds of the formula (III) usable in the process according to the third aspect of this invention, 3,4-di-O-acetyl-2,6-dideoxy-2-fluoro-α-L-mannopyranosyl bromide having the below-described formula (j) [the compound of the formula (III) where Y = acetyl, and X = Br ] can be prepared in accordance with the under-mentioned reaction scheme, starting from the known compound of the formula (f), namely di-O-acetyl-6-deoxy-L-glucal and proceeding via 1,3,4-tri-O-acetyl-2,6-dideoxy-2-fluoro-α- and β-L-mannopyranoses of the below-described formula (i).

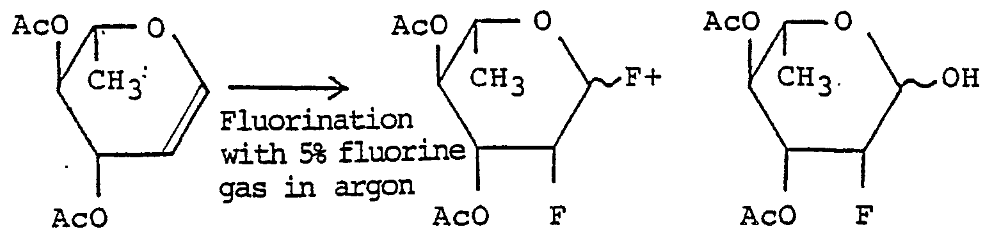


(f) (g) (h)

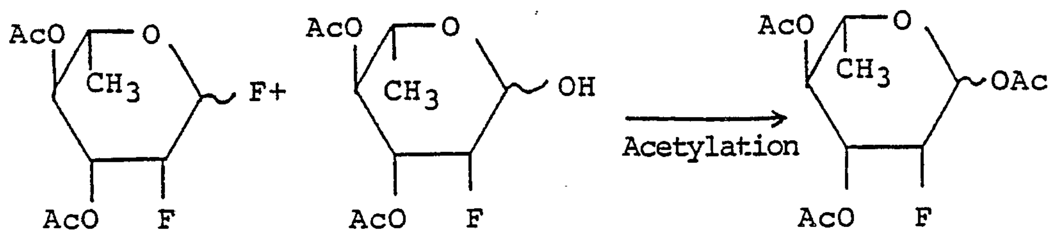


(g) (h) (i)

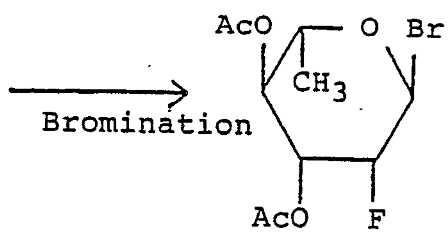


(j)

In the above reaction scheme, Ac stands for an acetyl group. Details of the series of reactions for preparing the compound of the formula (j) from the compound of the formula (f) are described in Referential Examples 1, (1)-(3) which will be described hereinafter. Incidentally, when the tri-O-acetyl compound of the formula (i) is reacted with a titanium halide, for example, titanium tetrabromide, titanium tetrachloride or titanium tetraiodide at room temperature or under heating, and under anhydrous conditions, in an inert organic solvent, e.g., dichloromethane or ethyl acetate or mixed solvents thereof or is reacted with hydrogen bromide, hydrogen chloride or hydrogen iodide dissolved in acetic acid, a halide compound having the following formula:

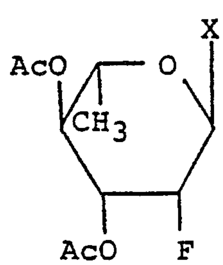

(k)

wherein each Ac is an acetyl group and X is a bromine, chlorine or iodine atom may be formed, in general.

Further treatment of the compound of the formula (k) as dissolved in an inert solvent, for example, with an aqeuous solution of hydrogen bromide can provide a 2,6-dideoxy-2-fluoro-α-L-mannopyranosyl halide of the following formula:

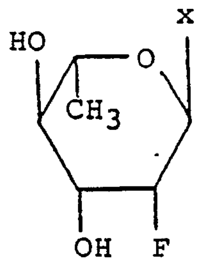

(III')

wherein X has the same meaning as defined above. To protect the 3- and 4-hydroxyl groups of the compound of the formula (III′) with hydroxyl-protecting groups (Y) of the acyl type, the compound of the formula (III′) may be reacted with an acetic anhydride or an anhydride or chloride of another lower alkanoic acid, or with the anhydride or chloride of an aromatic carboxylic acid, e.g., benzoic acid. This protecting reaction provides the compound of the formula (III) where each Y is the hydroxyl-protecting group of the acyl type.

Among the compounds of the formula (I) according to the first aspect of this invention, such a compound of the formula (Ib) which is a compound of the formula (I) where $R^1$ is a hydroxyl group, including 7-O-(2,6-dideoxy-2-fluoro-α-L-mannopyranosyl)adriamycinone or -4-demethoxyadriamycinone, can be synthesized when the 14-methyl group of the compound of the formula (Ia-1) which is obtained as an intermediate or the 14-methyl group of the compound of the formula (Ia) which is obtained as the final product in the process according to the third aspect of this invention is converted into a halomethyl group, followed by converting the halomethyl group into a hydroxymethyl group ($-CH_2OH$) by hydrolysis, and where the resulting conversion product from the hydrolysis still contains any hydroxyl-protecting groups (Y), then removing the hydroxyl-protecting groups therefrom. It is however not essential to protect the hydroxyl groups of the glycoside moiety upon the aforesaid hydrolysis of the halomethyl group of the compound of the formula (Ia-1) or (Ia).

In the fourth aspect of this invention, therefore, there is provided a process for the preparation of an adriamycin derivative represented by the formula

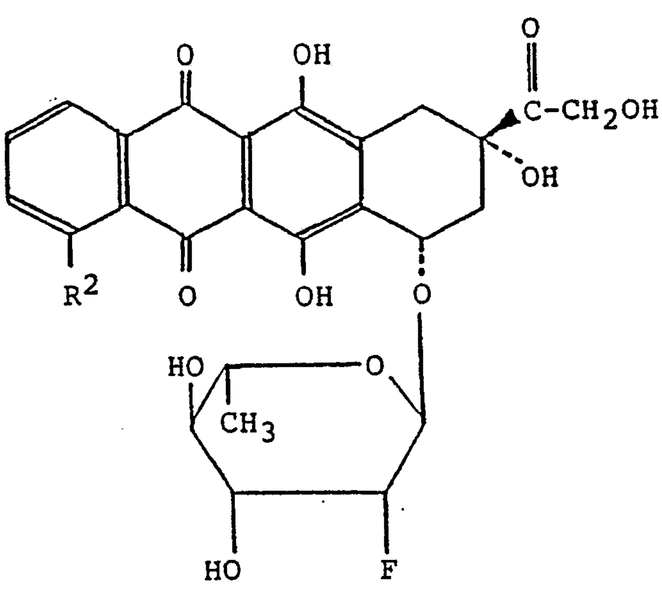

(Ib)

wherein $R^2$ is a methoxy group or a hydrogen atom, which comprises hydrolyzing a halomethyl group $-CH_2-W$ of a 7-O-(2,6-dideoxy-2-fluoro-α-L-mannopyranosyl)-14-halodaunomycinone or -14-halo-4-demethoxydaunomycinone represented by the formula

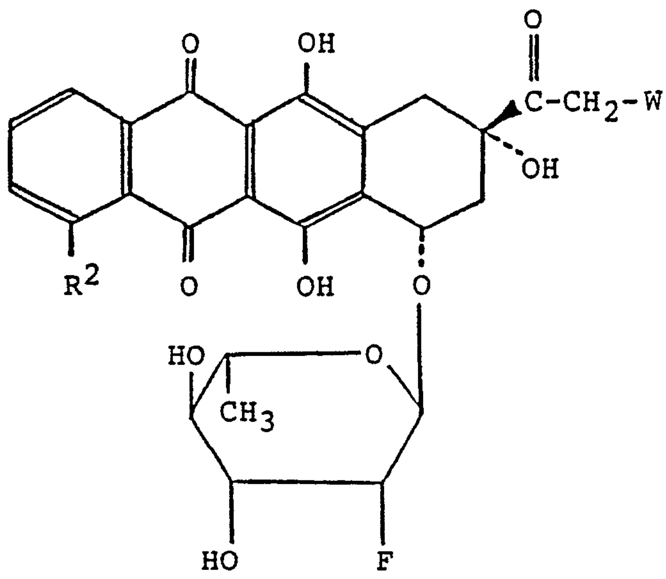

(Id)

wherein $R^2$ has the same meaning as defined above and W is a bromine, chlorine or iodine atom.

In the process according to the fourth aspect of this invention, the hydrolysis of the 14-halomethyl group ($-CH_2-W$) of the compound of the formula (Id) can be practiced in the following manner. Thus, the hydrolysis can be achieved by reacting the compound of the formula (Id) with sodium formate or lithium formate, and when formyloxy groups have been introduced into the 14-position by a concurrent side-reaction, then treating further the reaction solution with aqueous ammonia or an aqueous solution of sodium bicarbonate (a modified process of the Alkamone's process described in Example 1 of Japanese Patent Publication No. 36919/82 or U.S. Patent No. 4,125,607). As a solvent useful in the reaction between the compound of the formula (Id) and sodium formate or lithium formate, may be mentioned water, dimethylsulfoxide, dimethylformamide, an ether such as dioxane, tetrahydrofuran or dimethoxyethane an alkyl acetate, or a ketone such as acetone. The reaction temperatures therefor may desirably be at 0-50 °C, while the reaction time may preferably be 1-48 hours. The reaction between the compound of the formula (Id) and sodium formate or lithium formate provides formation of the compound of the formula (Id).

When the compound of the formula (Id) is reacted with sodium formate or lithium formate as above, however, a 14-O-formyl compound of the following formula:

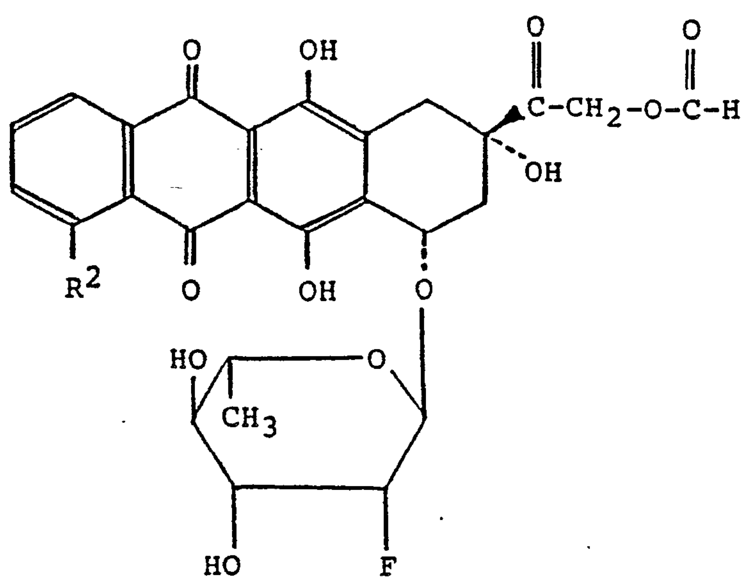

(V)

where $R^2$ is as defined above may be formed as by-product from a portion of the compound of the formula (Id) in some instances. When hydrolyzing this 14-O-formyl compound (V) with an aqueous solution of

sodium bicarbonate or aqueous ammonia at 0° C-40° C, for example, in an ether such as tetrahydrofuran or dioxane, a ketone such as acetone, an alcohol such as methanol, a halogenated hydrocarbon such as chloroform, dimethylformamide, dimethylsulfoxide, or mixed solvents of two or more of such solvents, the compound of the formula (Ib) can be formed.

Among the compounds of the formula (Id) useful as starting compounds in the process according to the fourth aspect of this invention, such compound in which W is bromine atom can be prepared by the following method. Thus, it can be prepared by brominating the 14-methyl group of the compound of the formula (Ia) which was obtained as the final compounds in the process according to the third aspect of this invention. Prior to this bromination, it is however preferred to react the compound of the formula (Ia) with methyl orthoformate at a temperature of 0° C-50° C in methanol or dioxane, or mixed solvents thereof so that the 13-carbonyl group of the compound of the formula (Ia) undergoes the formation of a dimethylketal. By this dimethylketal formation, a compound of the following formula:

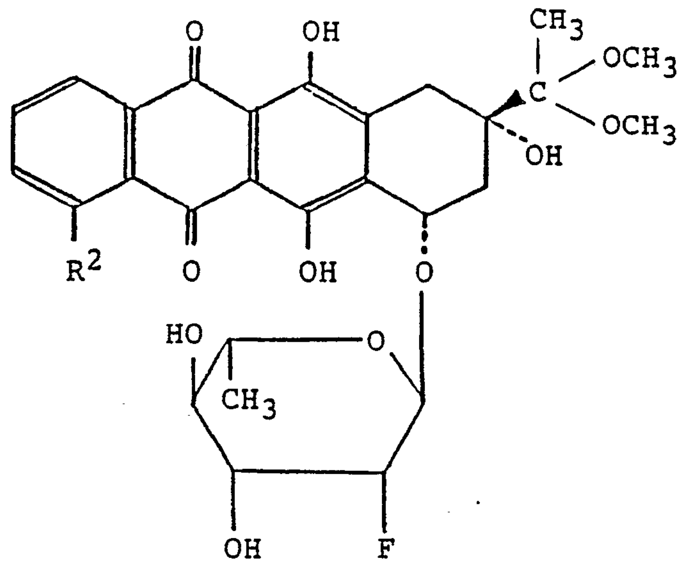

(VI)

can be prepared. When the compound of the formula (VI) is reacted with bromine at a temperature of 0° C-50° C in a halogenated hydrocarbon, e.g., dichloromethane, a lower alkanol, e.g., methanol or ethanol, dioxane or tetrahydrofuran, a compound of the following formula:

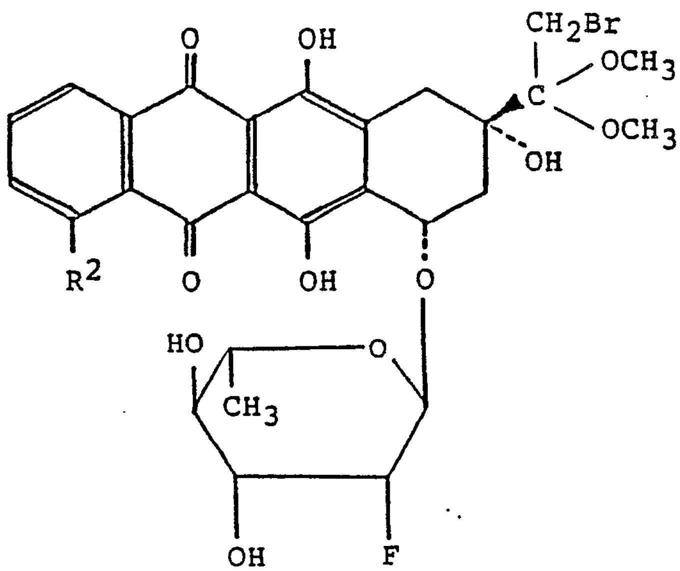

(VII)

can be prepared. Upon removing the dimethylketal group from the compound of the formula (VII) by

hydrolysis with hydrobromic acid, there is produced a compound of the following formula

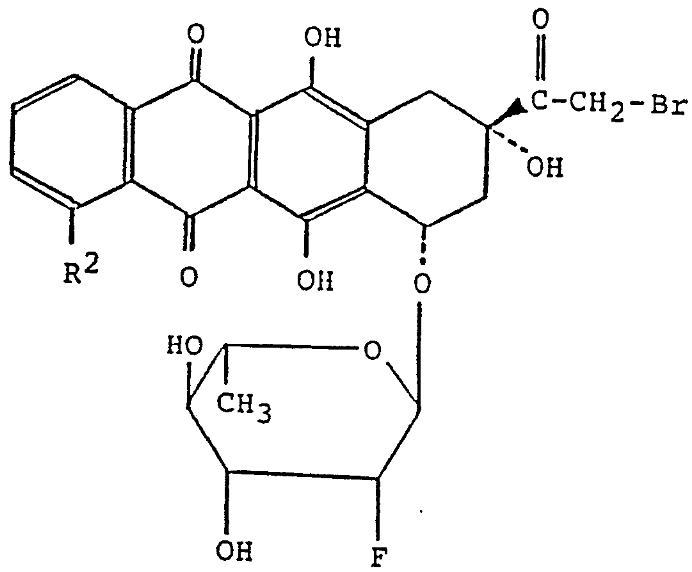

(Id')

The dimethylketal group of the compound of the formula (VII) can be removed also when the compound (VII) is treated with acetone.

Of the compounds of the formula (Id), such compound in which W is a chlorine or iodine atom can be prepared in the following way. Thus, chlorination or iodination of the compound of the formula (VI) is conducted by reacting the compound (VI) with chlorine or iodine in the same manner as in the bromination of the compounds of the formula (VI) except that chlorine or iodine is used in place of bromine. The resulting chlorinated or iodinated product from the compound (VI) may then be after-treated in the same manner as above.

Further, among the compounds of the formula (I) according to this invention, the half-ester type compounds of the formula (Ic) where the group R[1] is a group $-O-CO-(CH_2)_n-COOH$ can be prepared by a process in which the compound of the above formula (Id) or a compound of formula (Ie) as given hereinafter is reacted with a mono-alkali metal salt of an aliphatic dicarboxylic acid having formula (IV) as given hereinafter, for example, the mono-sodium salt or mono-potassium salt of the aliphatic dicarboxylic acid.

In the fifth aspect of this invention, there is thus provided a process for the preparation of an adriamycin half-ester derivative represented by the general formula

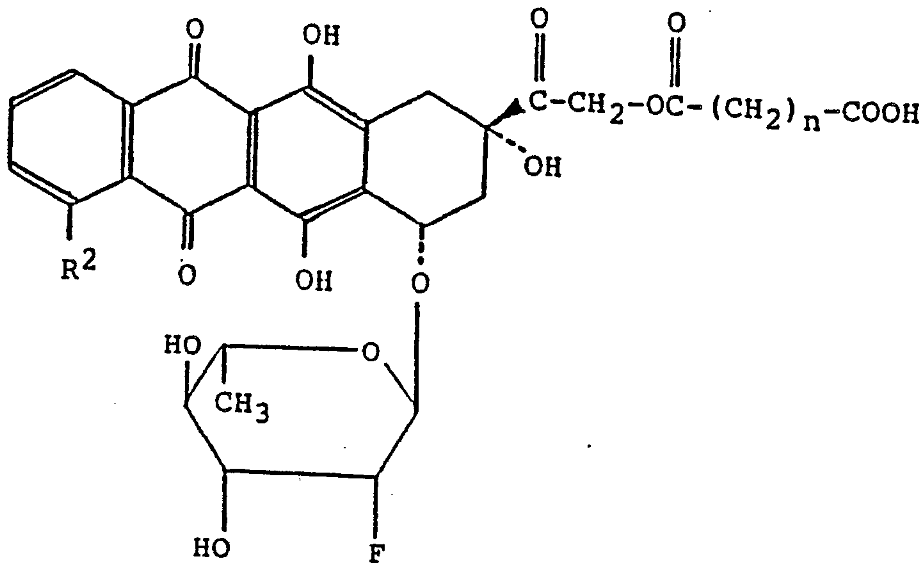

(Ic)

wherein $R^2$ is a methoxy group or a hydrogen atom and n denotes an integer of 1 to 10, which comprises reacting a 7-O-(2,6-dideoxy-2-fluoro-α-L-mannopyranosyl)-14-halodaunomycinone or -14-halo-4-demethoxydaunomycinone derivative represented by the formula

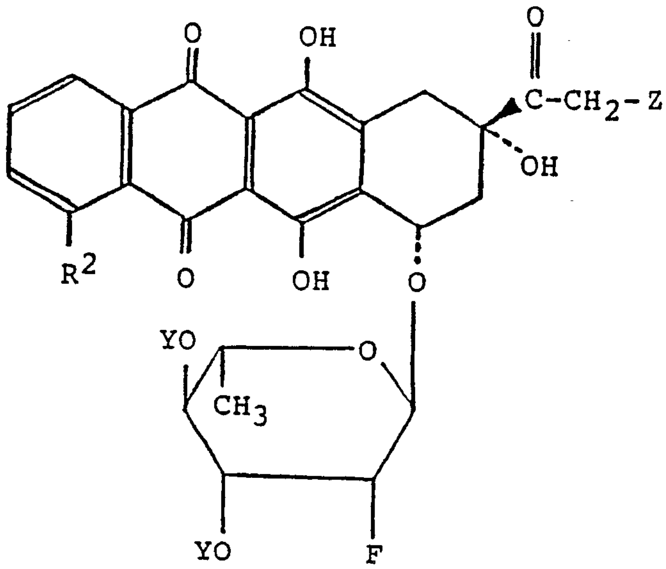

(Ie)

wherein $R^2$ has the same meaning as defined above, Z is a bromine, chlorine or iodine atom and Y is a hydrogen atom or a hydroxyl-protecting group, with a mono-alkali metal salt of an aliphatic dicarboxylic acid, represented by the formula

$A\text{-}OOC\text{-}(CH_2)_n\text{-}COOH$ (IV)

wherein A is an alkali metal atom and n has the same meaning as defined above, to form a compound of the formula

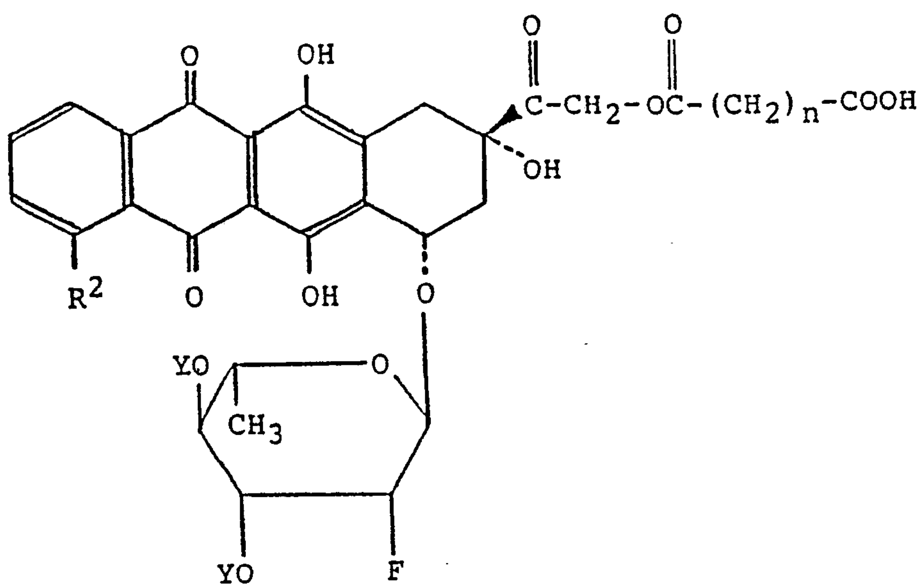

(Ic-1)

wherein $R^2$, Y and n have the same meanings as defined above, and where the compound of the formula (Ic-1) contains the remaining hydroxyl-protecting groups (Y), then removing the hydroxyl-protecting groups (Y) from the compound of the formula (Ic-1) in a known manner.

As examples of the aliphatic dicarboxylic acid according to the formula (IV), may be mentioned succinic acid, gultaric acid, adipic acid, pimelic acid and suberic acid. The mono-alkali metal salts of these aliphatic acids are preferably the mono-sodium or mono-potassium salts.

To prepare the compound of the formula (Ic-1) from the compound of the formula (Ie) and the

compound of the formula (IV) by their condensation reaction in the process according to the fifth aspect of this invention, the condensation reaction is carried out at 0-100 °C, usually, at a temperature in the vicinity of room temperature, for a time of 5-30 hours, usually, for about 15 hours or so. In this reaction, acetone, tetrahydrofuran, methanol, ethanol, DMF, DMSO, mixed solvents of such solvent(s) with water, or the like can be used as the solvent.

It is then important to use such a mono-alkali metal salt of a dicarboxylic acid according to the formula (IV), in which exactly one carboxyl group of the two carboxyl groups in the molecule has formed the salt (carboxylate) with the alkali metal cation, as the dicarboxylic acid mono-alkali metal salt of the formula (IV), and to react said mono-alkali metal salt with the compound of the formula (Ie) in controlled molar proportions.

Where the compound of the formula (Ic-1) thus obtained still contains the residual hydroxyl-protecting groups (Y), the hydroxyl-protecting groups (Y) are removed by a known deprotecting method as that the target compound of the formula (Ic) is afforded.

The reaction product of the formula (Ic) can be recovered from the reaction mixture by a method known per se in the art. The reaction product of the formula (Ic) thus recovered can be purified by chromatography on a silica gel, using a chloroform-methanol mixture or another solvent system as a developing solvent. The compound of the formula (Ic) can be converted into the form of its metal salt by reacting the free carboxyl group of the compound of the formula (Ic) with a cation of an alkali metal such as sodium or potassium, an alkaline earth metal such as calcium or the other metals in a usual manner.

The half-ester compounds of the formula (Ic) according to this invention have a higher solubility in water, as compared to the compounds of the formula (Ia) or (Ib) according to this invention, whereby the compounds of the formula (Ic) can be easily formulated into injectionable solutions.

Incidentally, the solubilities (as measured at room temperature) of some examples of the half-ester compound of the formula (Ic) amongst the compounds (I) according to this invention, which are each dissolved in a phosphate buffer solution of pH 7.4 [comprising a mixture of an aqueous potassium dihydrogen phosphate and aqueous sodium hydroxide which contained 0.05 M of potassium dihydrogen phosphate ($KH_2PO_4$)] are shown in Table 2.

Table 2

| Compound (Ic) of this invention | Solubility (mg/ml) |
|---|---|
| Compound of Example 6 | 25 |
| Compound of Example 7 | 28 |
| Compound of Example 8 | 32 |
| Compound of Example 9 | 32 |
| Compound of Example 10 | 30 |
| Compound of Example 11 | <1 |

In turn, we have further made a development of our investigation in an attempt to provide new, another anthracycline derivatives which are able to exhibit and retain an improved antitumor activities even when they are administered to tumor-bearing patients at a considerably lowered dosage as compared to the presently available chemotherapeutic antitumor agent, adriamycin, which is practically given at a dosage of 0.4 mg/kg to 2 mg/kg per day. As a result, we have now further succeeded in synthesizing new some derivatives from the anthracyclines of the formula (b) shown hereinbefore and the anthracyclines of the formula (c) shown hereinbefore. Thus, we have now succeeded in producing such new, another anthracycline derivatives having the general formula (I′) shown below, and we have found that the these new anthracycline derivatives having the general formula (I′) can meet our objects as desired.

In a sixth aspect of this invention,there is thus provided an anthracycline dirivative having the general formula

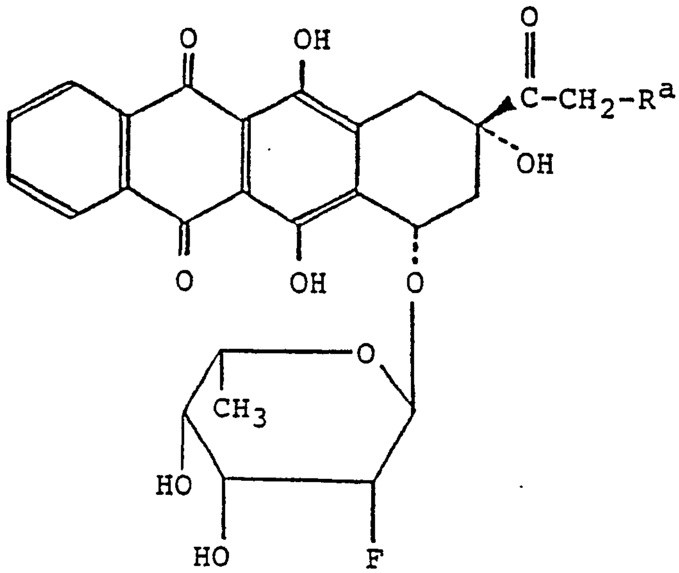

(I')

wherein $R^a$ is a hydrogen atom or a hydroxyl group or is a group of the formula

$$-O\overset{O}{\overset{\|}{C}}-(CH_2)_p-H$$

where p denotes an integer of 1 to 6, or is a group of the formula

$$-O\overset{O}{\overset{\|}{C}}-(CH_2)_q-COOH$$

where q denotes an integer of 1 to 6, or a salt thereof.

The compounds of the general formula (I′) according to the sixth aspect of this invention may largely be classified into following four classes (D), (E), (F) and (G) :-

(D) 4-Demethoxy-7-O-(2,6-dideoxy-2-fluoro-α-L-talopyranosyl) daunomycinone having the formula

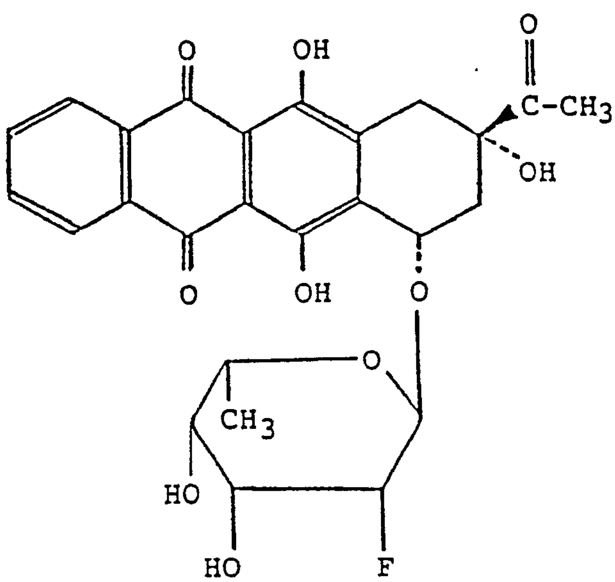

(I'-a)

(E) 4-Demethoxy-7-O-(2,6-dideoxy-2-fluoro-α-L-talopyranosyl) adriamycinone having the formula

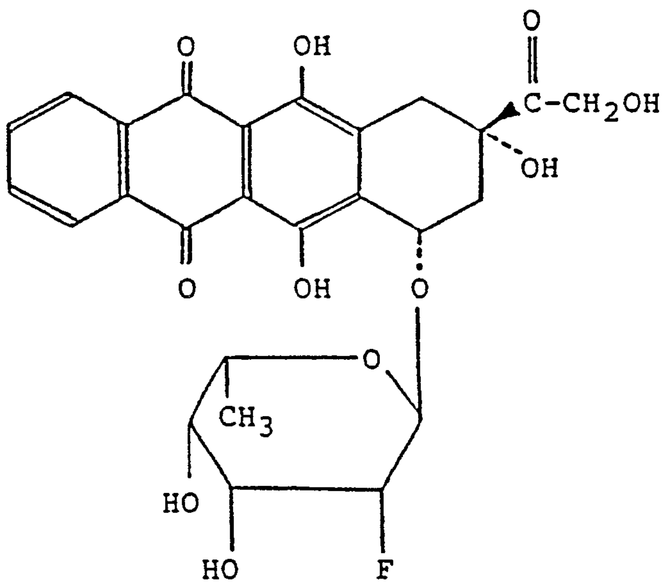

(I'-b)

(F) An ester derivative of 4-demethoxyadriamycin analogue having the general formula

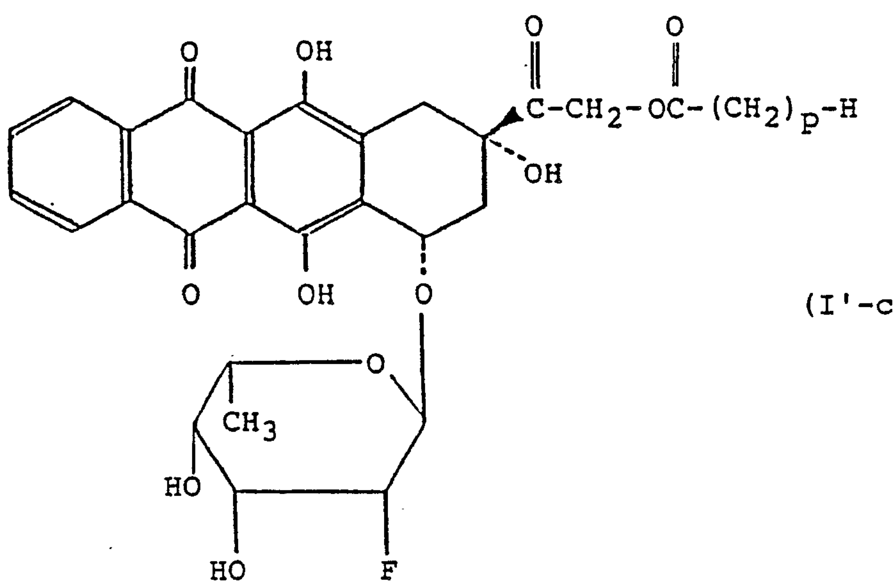

(I'-c)

where p denotes an integer of 1 to 6.

(G) A half-ester derivative of 4-demethoxyadriamycin analogue having the general formula

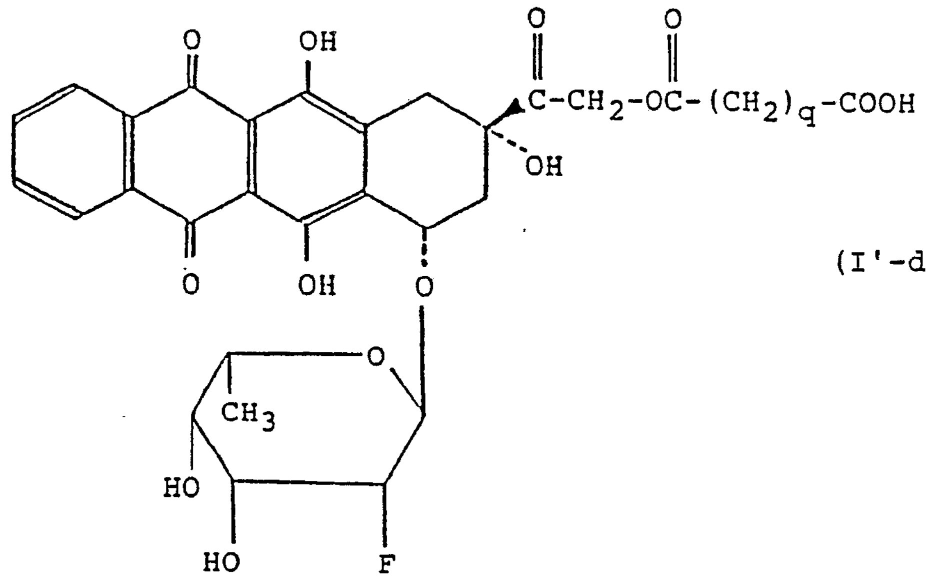

(I'-d)

where q denotes an integer of 1 to 6, or a salt thereof.

The names of specific examples of the compounds of the general formula (I') according to the sixth aspect of this invention and their physical properties will be shown in Examples 12 to 19 given hereinafter.

It has been confirmed from some tests that the compounds of the general formula (I') according to the sixth aspect of this invention also have markedly high antitumor activities against some experimental tumors in animals and their antitumor activities are remarkably higher than those of daunomycin and adriamycin. It has also been discovered that the compounds of the general formula (I') can exhibit usefully high antitumor activities even at their low dosages. In addition, the compounds of the general formula (I') exhibit high antibacterial activities, too and are also useful as antibacterial agent.

Some tests on the antitumor activities of some illustrative compounds of the general formula (I') will be described below.

Test 3 :

Antitumor activities against leukemia in $CDF_1$ mice as induced by mouse leukemia, Leukemia L-1210 cells

To evaluate the antitumor effects of the compounds of the formula (I') according to the sixth aspect of this invention against experimental tumors in animals, the procedure of Test 1 given hereinbefore is repeated. Thus, $CDF_1$ mice were intraperitoneally transplanted with cells of Leukemia L-1210 (at a dose of 1 x $10^5$ cells/mouse). After an elapsed time of 24 hours from the tumor transplantation, a test compound of the formula (I') of the sixth aspect invention to be evaluated was administered intraperitoneally for 9 consecutive days once per day. Observation was made for 60 day, where survival days of each mouse tested were recorded. In comparison with the control test wherein mice were treated with the administration of physiological saline alone, the increase (%) in the life-span of the treated mice was calculated as T/C %, where T is the mean survival days of the treated animals and C is the mean survival days of the untreated control animals. For the comparison, daunomycin and adriamycin were also tested in the same manner. The results are summarized in Table 3.

Table 3

| Compound tested | % Increase in life-span (T/C,%) | | | | | | | |
|---|---|---|---|---|---|---|---|---|
| | Dose,(mg/kg/day) | | | | | | | |
| | 5 | 2.5 | 1.25 | 0.6 | 0.3 | 0.15 | 0.08 | 0.04 |
| 4-Demethoxy-7-0-(2,6-dideoxy-2-fluoro-α-L-talopyranosyl)-daunomycinone (Compound of Example 12) | 80* | 101* | >436* | 202 | 138 | 106 | | |
| 4-Demethoxy-7-0-(2,6-dideoxy-2-fluoro-α-L-talopyranosyl)-adriamycinone (Compound of Example 13) | 77* | 80* | 96* | >427 | >348 | >534 | | |
| 4-Demethoxy-7-0-(2,6-dideoxy-2-fluoro-α-L-talopyranosyl)adriamycinone 14-0-hemiadipate (Compound of Example 17) | | | >296* | 242* | 166 | 130 | 99 | 99 |
| 4-Demethoxy-7-0-(2,6-dideoxy-2-fluoro-α-L-talopyranosyl)-adriamycinone 14-0-hemipimelate (Compound of Example 19) | | | 158* | 245 | 175 | 138 | 96 | 93 |
| Daunomycin (comparative drug) | 117* | 151* | 193 | 166 | 133 | 130 | | |
| Adriamycin (comparative drug) | 191* | 228 | 222 | 142 | 136 | 123 | | |
| 7-0-(2,6-Dideoxy-2-fluoro-α-L-talopyranosyl)-daunomycinone (Reference) | 184 | 217 | 171 | 118 | 105 | 105 | | |
| 7-0-(2,6-Dideoxy-2-fluoro-α-L-talopyranosyl)-adriamycinone (Reference) | >750 | >350 | 275 | 185 | 182 | 127 | | |

In Table 3, asterisks (*) indicate that development of toxicity such as toxicity-related death or a weight loss was observed on the corresponding mice tested.

Adriamycin employed as a comparative drug in Test 3 above is a carcinostatic agent which is actually used in clinical treatments. Adriamycin is usually administered to men at doses in a range of from 0.4 mg/kg to 2 mg/kg, depending on the types of cancers to be treated. When adriamycin is administered at a dose of from 2.5 mg/kg/day to 5 mg/kg/day to the mice which has been inoculated with L-1210 cells, adriamycin exhibits the antitumor effects such that the rate (%) of increase in life-span (T/C, %) amounts to about 228-191 %, and adriamycin can then be accompanied by development of toxicity (see the results of Table 3 above). In contrast, it should be worthy to note that the compound of the formula (I′) according to the sixth aspect of this invention as administered at a suitable low dose in a range of from 0.6 mg/kg/day to 0.15 mg/kg/day is able to bring about remarkably high rate (%) of increase in life-span (T/C, %) with no or little development of the toxicity, and also that especially, the particular compound of Example 13 of the sixth aspect invention {namely, 4-demethoxy-7-O-(2,6-dideoxy-2-fluoro-α-L-talopyranosyl)adriamycinone} as given intraperitoneally at an extremely low dosage of 0.15 mg/kg/day is able to exhibit very much excellent antitumor effects such that the resultant increase rate (%) in the life-span (T/C, %) reaches a value of greater than 534% with the complete cure of the leukemia in the mice (three mice were completely cured among the treated four mice). Therefore, the compounds of the formula (I′) are advantageous in that their antitumor effects can be expected in clinical treatments also when they are administered not at a large dose to cancer-bearing patients.

In view of the above test results, the new anthracycline compound of the general formula (I′) according to the sixth aspect of this invention is considered to have excellent antitumor activities and can be expected to be useful as an antitumor agent in actual clinical applications and also to be useful for the therapeutic treatment of various tumors, equally to adriamycin.

As will be evident from the results of Test 3 above, the compounds of the general formula (I′) according to the sixth aspect invention show excellent antitumor activities against experimental tumors such as leukemia L-1210 cells and other leukemia cells, so that they are useful as therapeutic agents for malignant tumors and for the therapeutic treatment of solid cancers and ascitic cancers.

In a seventh aspect of this invention, therefore, then is provided a pharmaceutical antitumor composition which comprises as an active ingredient an anthracycline derivative of the general formula (I′), in association with a pharmaceutically acceptable liquid or solid carrier for the active ingredient.

The compound of the formula (I′) of the sixth aspect of this invention can generally be administered parenterally or orally in the same manner as the compound of the general formula (I) according to the first aspect of this invention. The compound of the formula (I′) can again be formulated into usual, dosable preparation forms with suitable excipents in the same manner as the compound of the formula (I) according to the first aspect of this invention.

Suitable dosage of the compound of the formula (I′) according to the sixth aspect of this invention may be determined in the same way as that for the compound of the formula (I) according to the first aspect of this invention. As a guideline, the compound of the formula (I′) may be administered at a dosage as same as or lower than that for adriamycin when given as an antitumor agent and generally may be administered at a dose in a range of from 0.2 mg/kg to 0.8 mg/kg once a day.

The new compound of the general formula (I′) according to the sixth aspect of this invention also exhibits antibacterial activities against Gram-positive bacteria and may be useful as a therapeutic agent for treatment of infections caused by Gram-positive bacteria in a usual, dosable preparation form, similarly to the compound of the general formula (I) according to the first aspect of this invention.

Suitable processes for the preparation of the compounds of the general formula (I′) according to this invention are now described in the following.

Among the compounds of the general formula (I′) according to the sixth aspect of this invention, such a compound of the formula (I′-a), which is a compound of the formula (I′) where $R^a$ is a hydrogen atom, namely 4-demethoxy-7-O-(2,6-dideoxy-2-fluoro-α-L-talopyranosyl)daunomycinone, can be synthesized by reacting the 7-hydroxyl group of 4-demethoxydaunomycinone having the formula (II′) given below with a glycosyl compound having the formula (III″) given below, namely, 2,6-dideoxy-2-fluoro-α-L-talopyranosyl halide or its hydroxyl-protected derivative, and where the resulting reaction product contains any remaining hydroxyl-protecting groups, then removing the hydroxyl-protecting groups from the reaction product.

In an eighth aspect of this invention, there is thus provided a process for the preparation of a 4-demethoxydaunomycin analogue having the formula

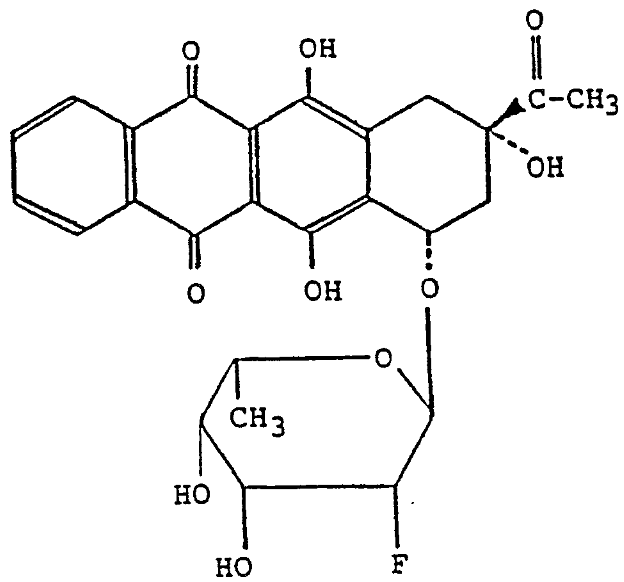

(I'-a)

which comprises condensing 4-demethoxydaunomycinone of the formula

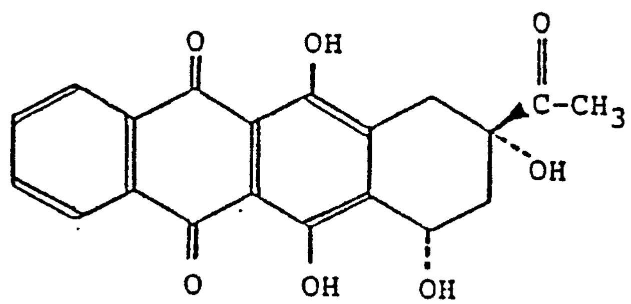

(II')

with a 2,6-dideoxy-2-fluoro-α-L-talopyranosyl halide or a hydroxyl-protected derivative thereof having the formula

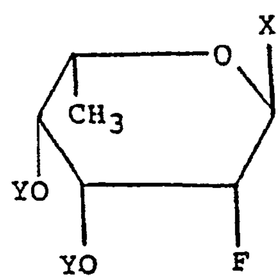

(III")

wherein X is a bromine, chlorine or iodine atom and Y is a hydrogen atom or a hydroxyl-protecting group, to produce a compound of the formula

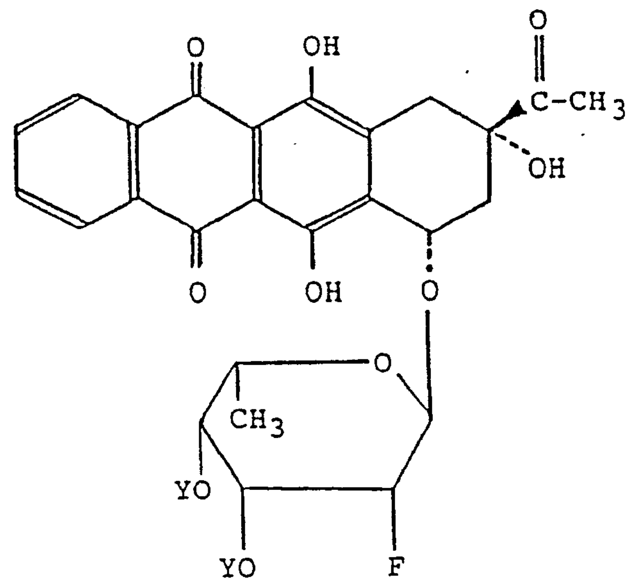

(I'-a-1)

wherein Y is a defined above, and where the compound of the formula (I′-a-1) contains the remaining hydroxyl-protecting group (Y), then removing the hydroxyl-protecting groups (Y) from the compound of the formula (I′-a-1) in a known manner.

In the process according to the eighth aspect of this invention, the reaction between 4-dimethoxydaunomycinone of the formula (II′) and 2,6-dideoxy-2-fluoro-α-L-talopyranosyl derivative of the formula (III″) can be effected by a conventional condensation method in the same manner as the reaction between the daunomycinone compound of the formula (II) and the 2,6-dideoxy-2-fluoro-α-L-mannopyranosyl derivative of the formula (III) which is carried out in the process according to the third aspect of this invention.

When the product of the formula (I′-a-1) so obtained contains the remaining hydroxyl-protecting groups (Y) therein, these hydroxyl-protecting groups may be removed therefrom in a known manner, for example, by acidic or alkaline hydrolysis or any other appropriate reaction according to a conventional deprotection technique, so that the desired compound of the formula (I′-a) is afforded.

Among the talopyranosyl compounds of the formula (III″) employed in the process of the eighth aspect invention, 3,4-di-O-acetyl-2,6-dideoxy-2-fluoro-α-L-talopyranosyl bromide or chloride which is preferably used may be prepared by the method as described in the above-mentioned Japanese patent application first publication "Kokai" Nos. 145097/87 and 145096/87 or European patent application publication No. 230, 013 Al. The compound of the formula (I′-a) so produced is normally in the form of an orange solid and can be purified by reprecipitation or recrystallisation from chloroform-hexane or other suitable mixed organic solvents, similarly to the compound of the formula (Ia) according to the first aspect of this invention.

Among the compounds of the formula (I′) according to the sixth aspect of this invention, such a compound of the formula (I′-b) which is a compound of the formula (I′) where $R^a$ is a hydroxyl group, namely 4-demethoxy-7-O-(2,6-dideoxy-2-fluoro-α-L-talopyranosyl)adriamycinone, can be synthesized when the 14-methyl group of the compound of the formula (I′-a-1) which is obtained as an intermediate or the 14-methyl group of the compound of the formula (I′-a) which is obtained as the final product in the process of the eight aspect of this invention is converted into a halomethyl group, followed by converting the halomethyl group into a hydroxymethyl group ($-CH_2OH$) by hydrolysis, and where the resulting conversion product from the hydrolysis still contains any hydroxyl-protecting groups (Y), then removing the hydroxyl-protecting groups therefrom in a known manner. It is, however, not essential to protect the hydroxyl groups of the glycoside moiety upon the aforesaid hydrolysis of the halomethyl group.

In the ninth aspect of this invention, therefore, there is provided a process for the preparation of a 4-demethoxyadriamycin analogue having the formula

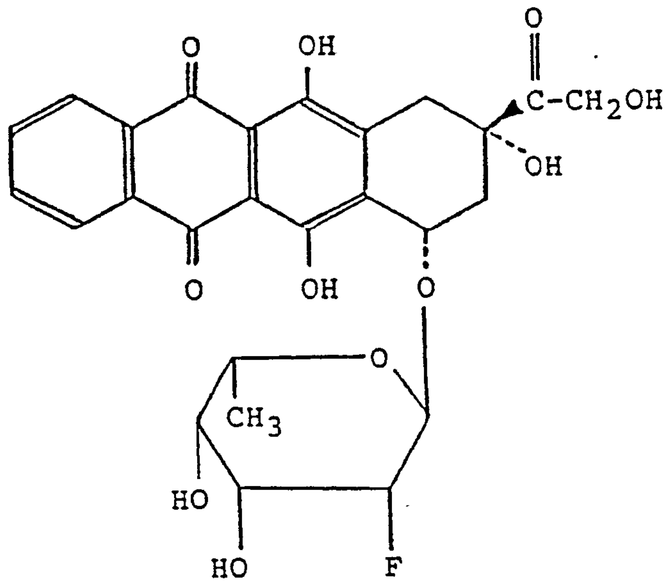

(I'-b)

which comprises hydrolyzing a halomethyl group (-$CH_2$-W) of a 4-demethoxy-7-O-(2,6-dideoxy-2-fluoro-α-L-talopyranosyl)-14-halo-daunomycinone derivative having the formula

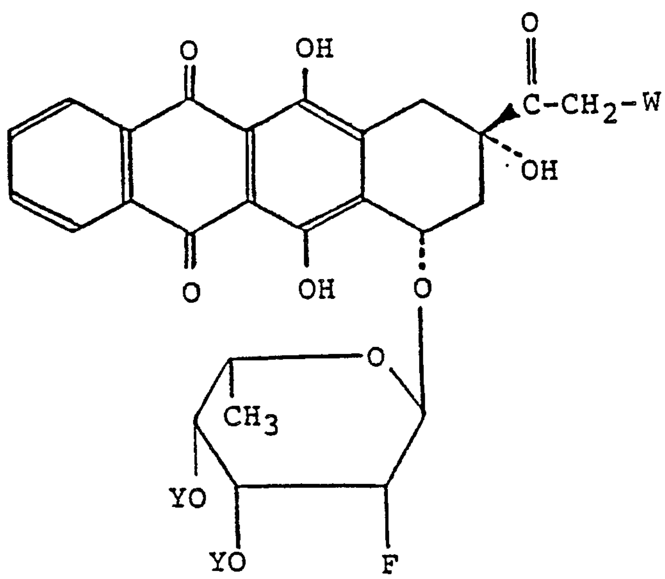

(I'-e)

wherein W is a bromine, chlorine or iodine atom and Y is a hydrogen atom or a hydroxyl-protecting group, to produce a compound of the formula

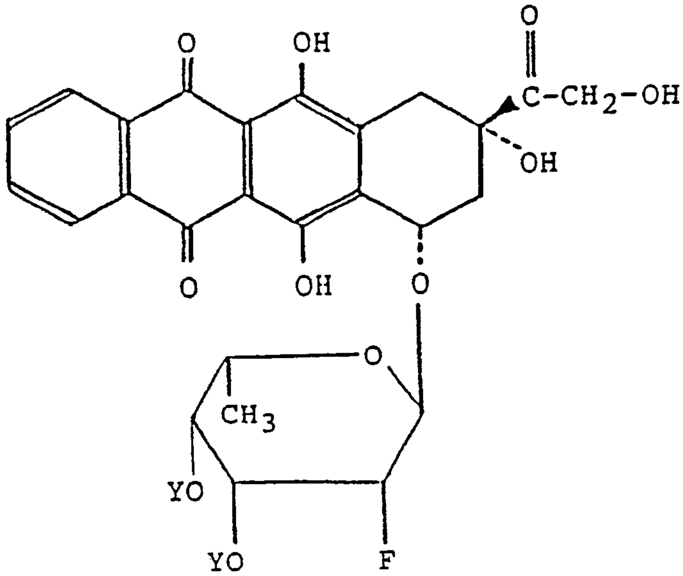

(I'-b-1)

wherein Y is as defined above, and where the compound of the formula (I′-b-1) contains the remaining hydroxyl-protecting groups (Y), then removing the hydroxyl-protecting groups (Y) from the compound of the formula (I′-b-1) in a known manner.

In the process according to the ninth aspect of this invention, the hydrolysis of the 14-halomethyl group ($-CH_2-W$) of the compound of the formula (I′-e) can be carried out in the same manner as when the 14-halomethyl group ($-CH_2-W$) of the compound of the formula (Id) is hydrolyzed in the process of the fourth aspect of this invention. Thus, the hydrolysis of the 14-halomethyl group of the compound of the formula (I′-e) can be achieved by reacting the compound of the formula (I′-e) with sodium formate or lithium formate, and when a formyloxy group has occasionally been introduced into the 14-position by a concurrent side-reaction, then treating the by-formed formyloxy derivative in suitable solvent with aqueous ammonia or aqueous sodium bicarbonate. The reaction of the compound of the formula (I′-e) with sodium or lithium formate gives the formation of the compound of the formula (I′-b-1).

When the compound of the formula (I′-e) is reacted with sodium formate or lithium formate as above, however, a 14-O-formyl compound of the formula:

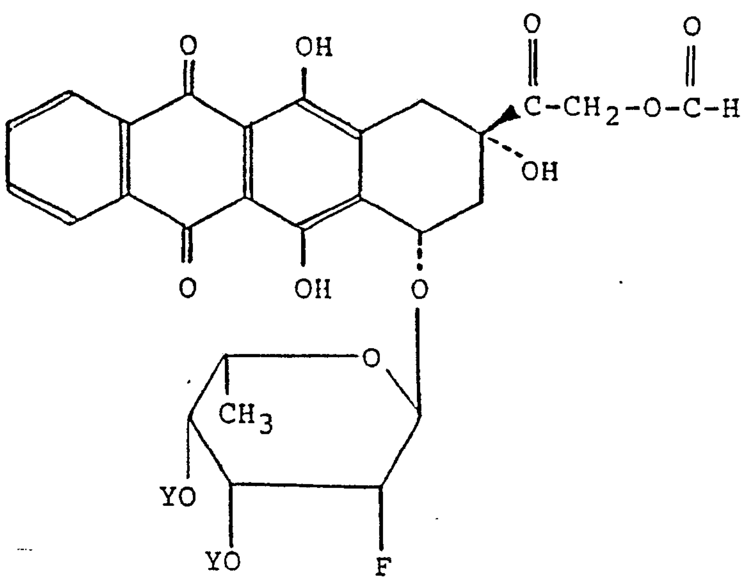

(V')

where Y is a defined above may be formed as a by-product from a portion of the compound of the formula (I′-e) in some instances. When this 14-O-formyl compound (V′) is after-treated by hydrolyzing with an aqueous solution of sodium bicarbonate or aqueous ammonia at 0 ° C-40 ° C, for example, in an ether such

as tetrahydrofuran or dioxane, a ketone such as acetone, an alcohol such as methanol, a halogenated hydrocarbon such as chloroform, dimethylformamide, dimethylsulfoxide, or mixed solvents thereof, the compound of the formula (I'-b-1) can be afforded. Where the compound of the formula (I'-b-1) so formed contains the remaining hydroxyl-protecting groups (Y) therein, these hydroxyl-protecting groups can be removed in a known manner, by for example, acidic or alkaline hydrolysis or any other suitable reaction, to afford the compound of the formula (I'-b).

Among the compounds of the formula (I'-e) useful as starting compounds in the process of the ninth aspect of this invention, such compound in which W is a bromine atom and Y is a hydrogen atom can be prepared by the following method. Thus, it can be prepared by brominating the 14-methyl group of the compound of the formula (I'-a) which was obtained as the final compounds in the process of the eighth aspect of this invention. Prior to this bromination, however, it is preferred to react the compound of the formula (I'-a) with methyl orthoformate at a temperature of 0°C-50°C in methanol or dioxane, or suitable mixed solvents thereof so that the 13-carbonyl group of the compound of the formula (I'-a) undergoes formation of a dimethylketal. By this dimethylketal formation, a compound of the formula

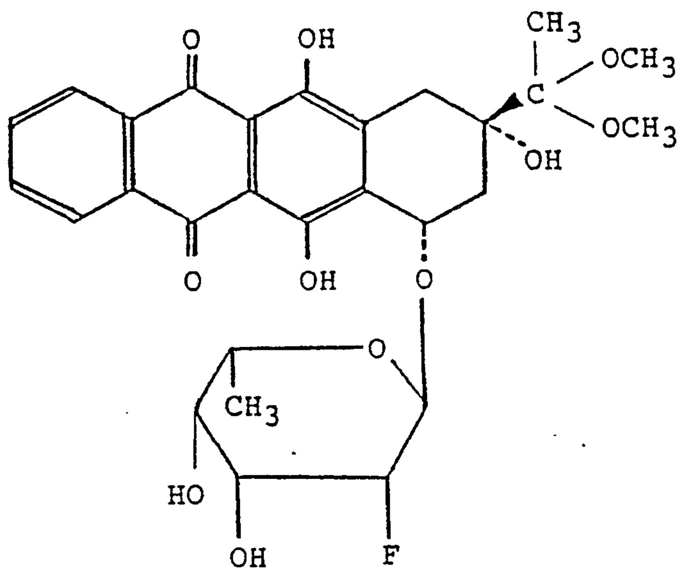

(VI')

can be prepared. When the compound of formula (VI') is reacted with bromine at a temperature of 0°C-50°C in a halogenated hydrocarbon, such as dichloromethane, a lower alkanol such as methanol or ethanol, dioxane or tetrahydrofuran, a compound of the formula

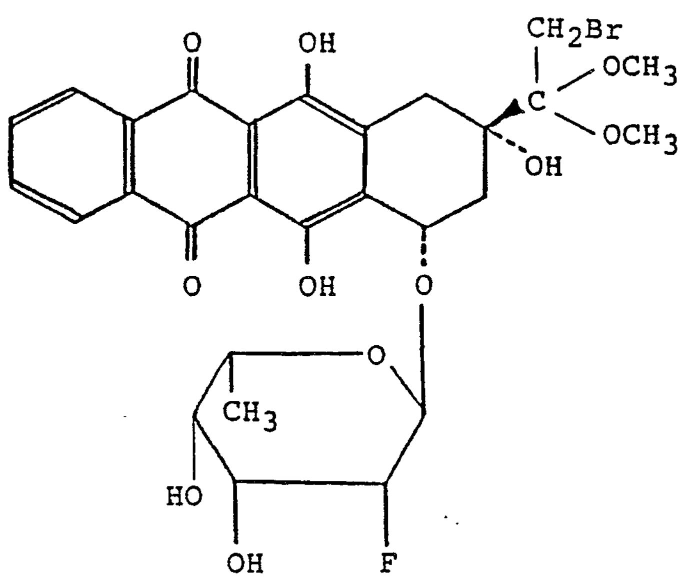

(VII')

can be prepared. Upon removing the dimethylketal group {$>(OCH_3)_2$} from the compound of the formula (VII') by hydrolysis with hydrobromic acid, there is produced a compound of the formula

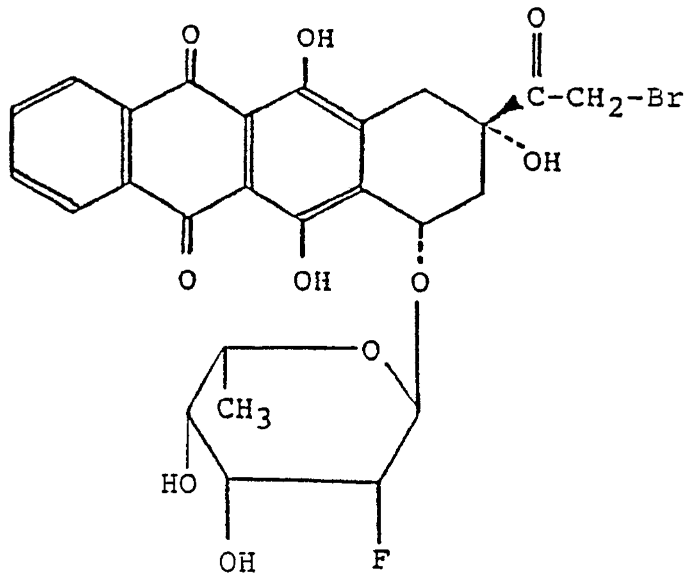

(I'-e-1)

The dimethylketal group of the compound of the formula (VII') can be removed also when the compound (VII') is treated with acetone. When the compound of the formula (VII') is treated with acetone as above for the removal of the dimethylketal group therefrom, the acetone can concurrently react with the hydroxyl groups at the 3- and 4-position of the talose moiety of the compound (VII') to introduce an O-isopropylidene group into these hydroxyl groups, and there is thus produced such a hydroxyl-protected derivative of the compound (I'-e-1) having the following formula

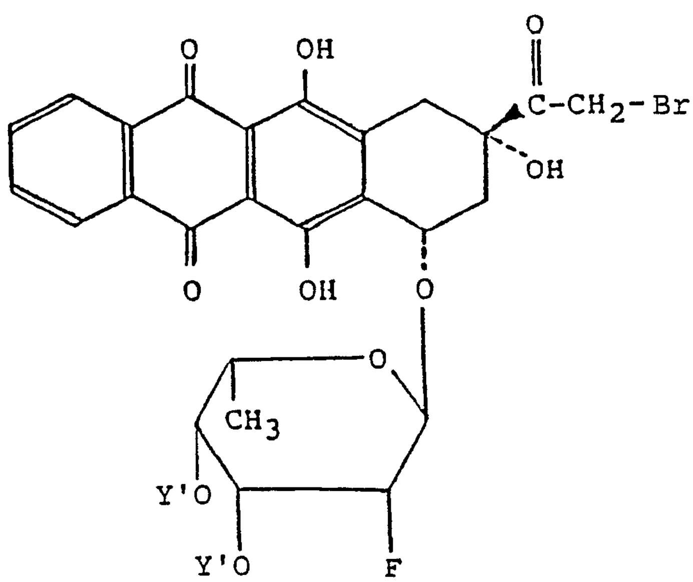

(I'-e-2)

wherein two Y' groups as taken together form an isopropylidene group.

Among the starting compounds of the formula (I'-e), such compound in which W is a chlorine or iodine atom and Y is a hydrogen atom can be prepared in the following way. Thus, the chlorination or iodination of the compound of the formula (VI') is carried out by reacting the compound (VI') with chlorine or iodine in the same manner as in the bromination of the compound of the formula (VI') expect that chlorine or iodine is used in place of the bromine. The resulting chlorinated or iodinated product from the compound (VI') may then be hydrolysed in the after-treatment in the same manner as above for the purpose of the removal of the dimethylketal group therefrom, so that the compound of the formula (I'-e) where W is a chlorine or

iodine atom and Y is a hydrogen atom is produced.

Further, amongst the compounds of the formula (I′) according to the sixth aspect invention, the compound of the formula (I′) where $R^a$ is the group $-O-CO-(CH_2)_p-H$, namely the ester-type compound of the formula (I′-c) can be prepared by a process in which the compound of the above formula (I′-e) is reacted with an alkali metal salt of an aliphatic carboxylic acid having the formula (IV′) shown hereinafter, for example, the sodium salt or potassium salt of said carboxylic acid.

According to the tenth aspect of this invention, therefore, there is provided a process for the preparation of an ester derivative of a 4-demethoxyadriamycin analogue having the formula

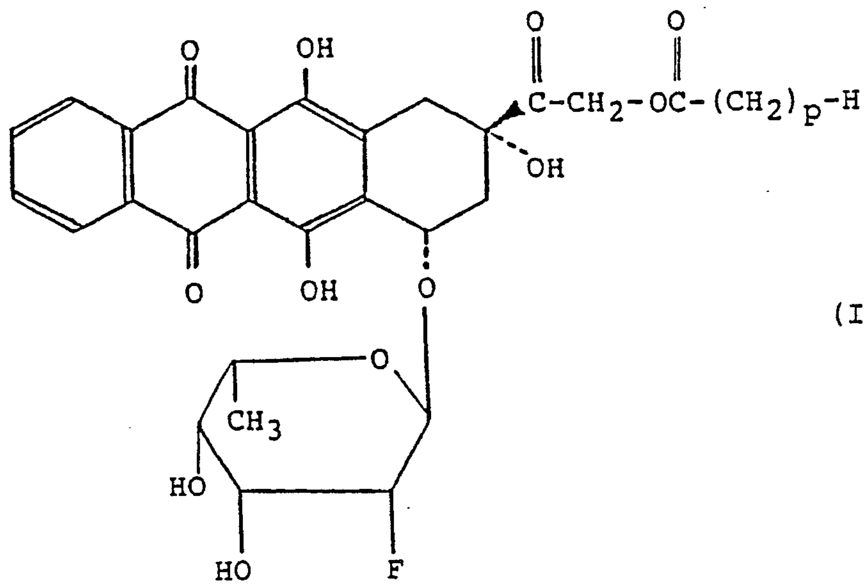

(I′-c)

wherein p denotes an integer of 1 to 6, which comprises reacting a 4-demethoxy-7-O-(2,6-dideoxy-2-fluoro-α-L-talopyranosyl)-14-halodaunomycinone derivative having the formula

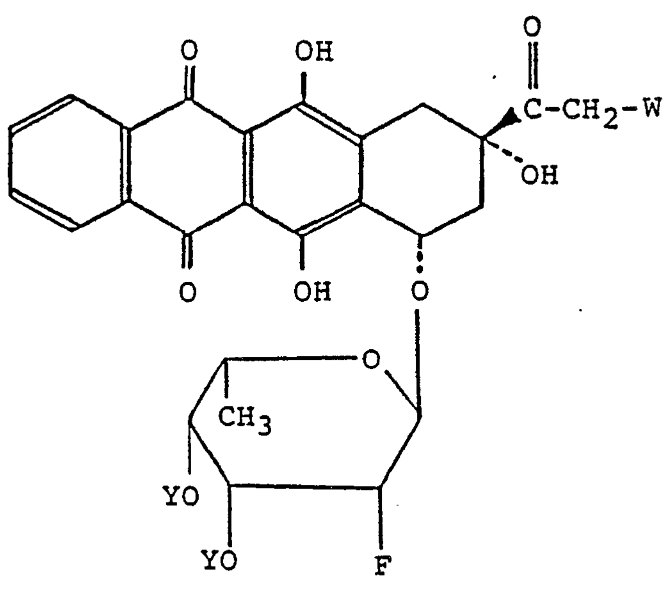

(I′-e)

wherein W is a bromine, chlorine or iodine atom and Y is a hydrogen atom or a hydroxyl-protecting group, with an alkali metal salt of an aliphatic carboxylic acid having the formula

$A-OOC-(CH_2)_p-H$ (IV′)

wherein A is an alkali metal atom and p denotes an integer of 1 to 6, to produce a compound of the formula

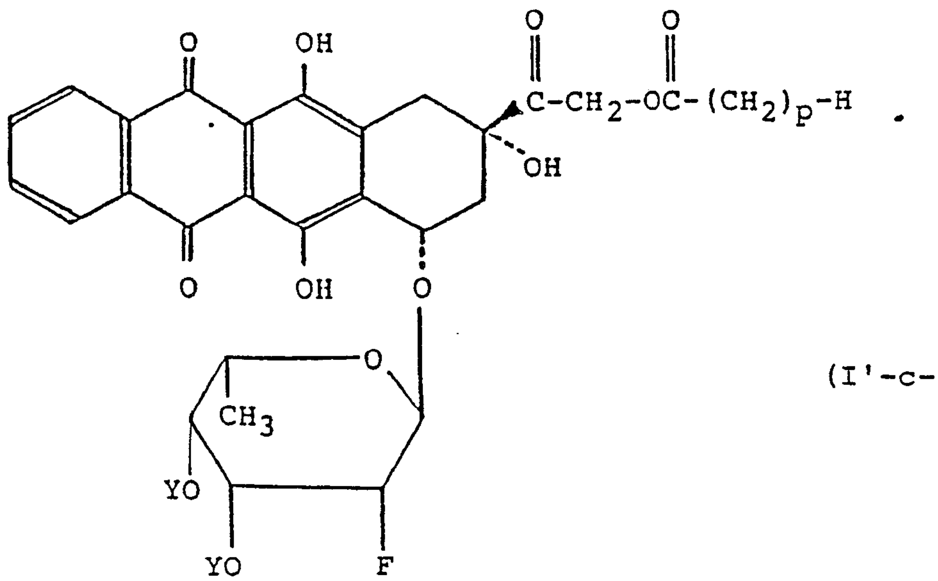

(I'-c-1)

wherein Y and p are as defined above, and where the compound of the formula (I'-c-1) contains the remaining hydroxyl-protecting groups (Y), then removing the hydroxyl-protecting groups (Y) from the compound of the formula (I'-c-1) in a known manner.

The particular compound of the formula (I'-e-1) given hereinbefore, namely 4-demethoxy-7-O-(2,6-dideoxy-2-fluoro-α-L-talopyranoxyl)-14-bromodaunomycinone or its corresponding 14-chloro or 14-iodo derivative may be converted into their hydroxyl-protected derivative by reacting the compound (I'-e-1) or its halo analogous with a known hydroxyl-protecting reagent which is usually available for the introduction of an alkylidene group, aralkylidene group, cycloalkylidene group or tetrahydropyranylidene group known as a di-valent hydroxyl-protecting group, for example, 2,2-dimethoxypropane, benzaldehyde, cyclohexanone dimethylketal or 4,4-dimethoxytetrahydropyran according to the conventional technique for the protection of hydroxyl group, whereby the hydroxyl groups at the 3- and 4-positions of the talose moiety of the compound (I'-e-1) or its halo analogue can be blocked with said di-valent hydroxyl-protecting group to afford the hydroxyl-protected derivative having the general formula

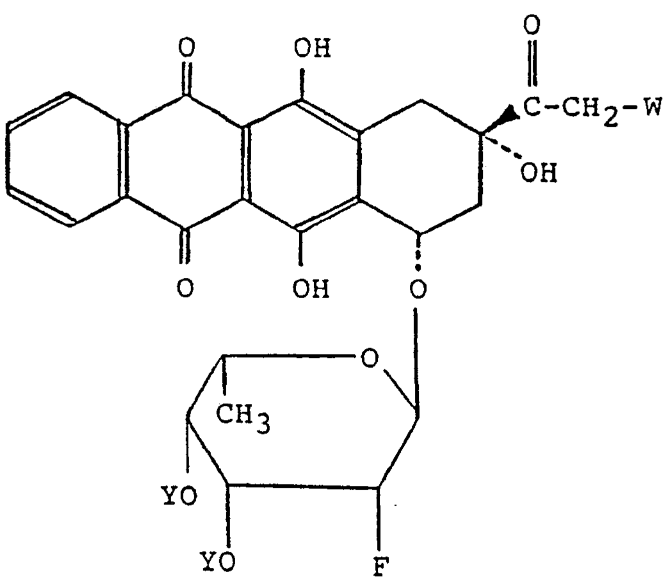

(I'-e-3)

wherein W is as defined above and the two Y groups as taken together form a di-valent hydroxyl-protecting group such as isopropylidene, benzylidene, cyclohexylidene or tetrahydropyranylidene group. However, once when the aforesaid 4-demethoxy-7-O-(2,6-dideoxy-2-fluoro-α-L-talopyranosyl)-14-bromo-( or chloro- or iodo-) daunomycinone has been obtained, this compound can be directly reacted with the carboxylic acid

alkali metal salt of the formula (IV′) without the hydroxyl groups of the talose moiety of the daunomycin analogue being protected beforehand.

In the process of the tenth aspect invention, the aliphatic carboxylic acid alkali metal salt of the formula (IV′) may preferably be sodium salts, potassium salts or lithium salts of acetic acid, propionic acid, butyric acid and valeric acid, for example.

In the process of the tenth aspect invention, the production of the compound of the formula (I′-c) can be achieved by condensing the compound of the formula (I′-e) with the carboxylic acid salt of the formula (IV′) at a temperature of 0 to 100° C, usually at room temperature or thereabout for a time of 5 to 30 hours, normally for about 15 hours. The condensation reaction may be effected in a reaction medium composed of acetone, tetrahydrofuran, methanol, ethanol, DMF, DMSO or mixed solvents thereof with water.

When the compound of the formula (I′-c-1) contains the remaining hydroxyl-protecting groups (Y) therein, the hydroxyl-protecting groups are removed therefrom by a conventional deprotection method to give the desired compound of the formula (I′-c).

Furthermore, amongst the compounds of the general formula (I′), such compound of the formula (I′) where $R^a$ is the group $-O-CO-(CH_2)_q-COOH$, namely the half-ester type compound of the formula (I′-d) can be produced by a process in which the compound of the formula (I′-e) is reacted with a nono-alkali metal salt of an aliphatic dicarboxylic acid having the formula (IV″) given hereinafter, for example, the mono-sodium salt or mono-potassium salt of the aliphatic dicarboxylic acid.

In the eleventh aspect of this invention, therefore, there is provided a process for the preparation of a half-ester derivative of 4-demethoxyadriamycin analogue having the formula

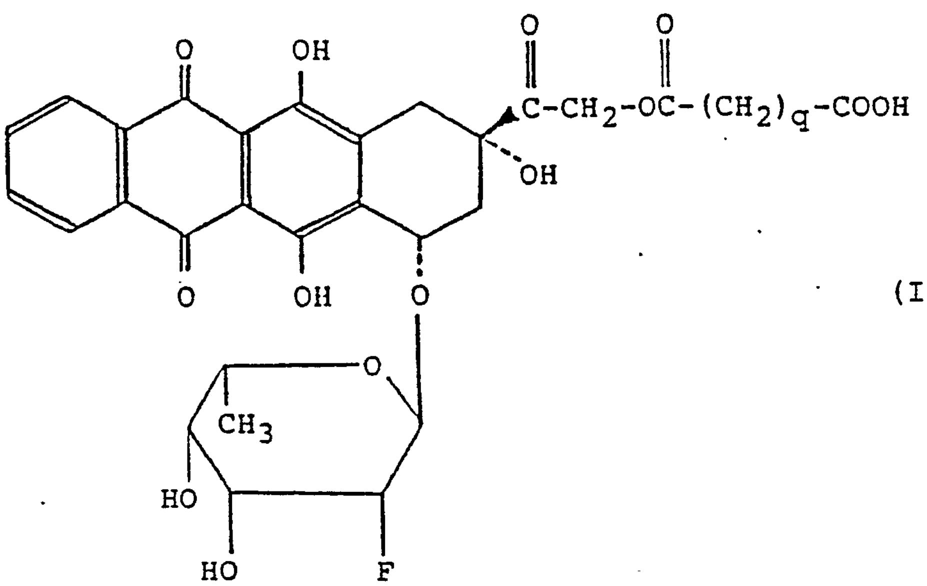

(I′-d)

wherein q denotes an integer of 1 to 6, which comprises reacting a 4-demethoxy-7-O-(2,6-dideoxy-2-fluoro-α-L-talopyranoxyl)-14-halodaunomycinone derivative having the formula

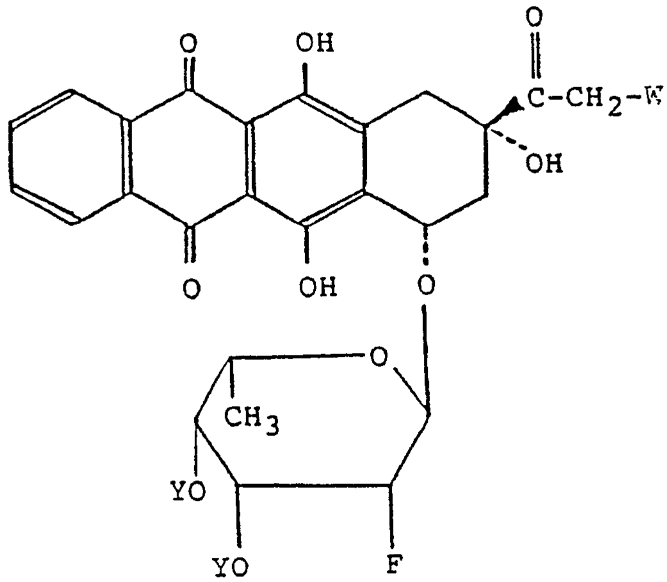

(I'-e)

wherein W is a bromine, chlorine or iodine atom and Y is a hydrogen atom or a hydroxyl-protecting group, with a nono-alkali metal salt of an aliphatic dicarboxylic acid having the formula

$A\text{-}OOC\text{-}(CH_2)_q\text{-}COOH$ (IV″)

wherein A is an alkali metal atom and q denotes an integer of 1 to 6, to produce a compound of the formula

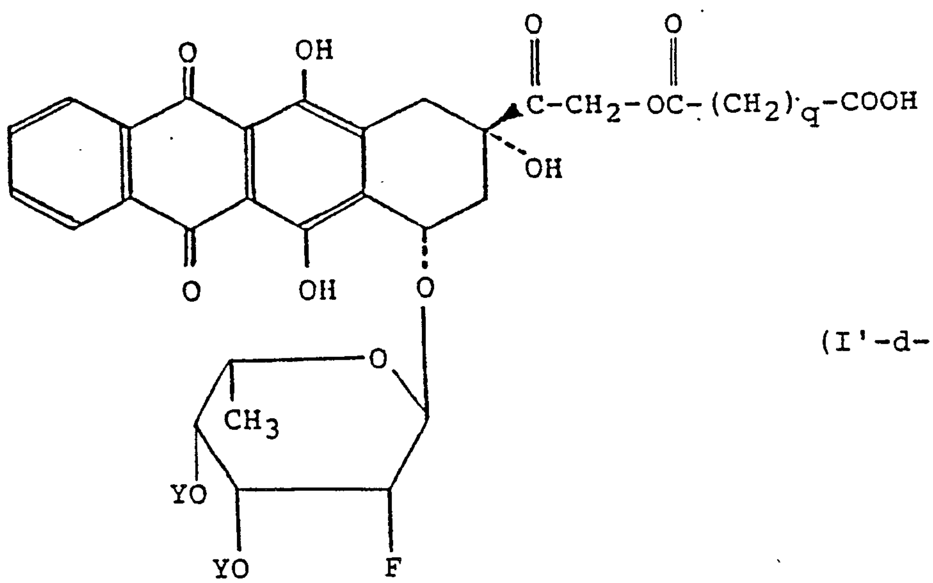

(I'-d-1)

wherein Y and q are as defined above, and where the compound of the formula (I′-d-1) contains the remaining hydroxyl-protecting groups (Y), then removing the hydroxyl-protecting groups (Y) from the compound of the formula (I′-d-1) in a known manner.

As examples of the aliphatic dicarboxylic acid according to the formula (IV″), may be mentioned succinic acid, gultaric acid, adipic acid, pimelic acid and suberic acid. The mono-alkali metal salts of these aliphatic acids are preferably the mono-sodium or mono-potassium salts.

To prepare the compound of the formula (I′-d) from the compound of the formula (I′-e) and the compound of the formula (IV″) by their condensation reaction in the process according to the eleventh aspect of this invention, the condensation reaction is carried out at a temperature of 0-100 °C, usually, at a temperature in the vicinity of room temperature, for a time of 5-30 hours, usually, for about 15 hours or so. In this reaction, acetone, tetrahydrofuran, methanol, ethanol, DMF, DMSO, mixed solvents of such solvent(s) with water, or the like may be used as the solvent.

It is then important to use such a mono-alkali metal salt of dicarbosylic acid according to the formula

(IV″), in which exactly one carboxyl group of the two carboxyl groups in the molecule has formed the salt (carboxylate) with the alkali metal cation, as the dicarboxylic acid mono-alkali metal salt of the formula (IV″), and to react said mono-alkali metal salt with the compound of the formula (I′-e) in controlled molar proportions.

Where the compound of the formula (I′-d-1) thus obtained still contains the residual hydroxyl-protecting groups (Y), the hydroxyl-protecting groups (Y) may be removed by a known deprotecting method so that the target compound of the formula (I′-d) is afforded.

The reaction product of the formula (I′-d) can be recovered from the reaction mixture by a method known per se in the art. The reaction product of the formula (I′-d) thus recovered can be purified by chromatography on a silica gel, using a chloroform-methanol mixture or another solvent system as a development solvent. The compound of the formula (I′-d) can be converted into the form of its metal salt by reacting the free carboxyl group of the compound of the formula (I′-d) with a cation of an alkali metal such as sodium or potassium, an alkaline earth metal such as calcium, magnesium or the other metals in a usual manner.

The half-ester compounds of the formula (I′-d) according to this invention have a higher solubility in water, as compared to the compounds of the formula (I′-a) or (I′-b) or (I′-c) according to this invention, whereby the compounds of the formula (I′-d) can be easily formulated into injectionable solutions.

Incidentally, the solubilities (as measured at room temperature) of some examples of the ester compounds of the formula (I′-c) or (I′-d) amongst the compounds of the formula (I′) according to the sixth aspect of this invention, which are each dissolved in a phosphate buffer solution of pH 7.4 [comprising a mixture of an aqueous potassium dihydrogen phosphate and aqueous sodium hydroxide which contained 0.05M of potassium dihydrogen phosphate ($KH_2PO_4$)] are shown in Table 4.

Table 4

| Compound (I′-c) or (I′-d) of this invention | Solubility (mg/ml) |
|---|---|
| Compound of Example 14 | <1 |
| Compound of Example 16 | 10 |
| Compound of Example 17 | 6 |
| Compound of Example 18 | <1 |
| Compound of Example 19 | <1 |

In a further aspect of this invention, there is provided a method of treating a tumor cell in a mammal, including human, which comprises administering an amount of a compound of the formula (I) or a compound of the formula (I′) as defined hereinbefore to a mammal bearing the tumor cell, with the amount of the compound administered being effective to suppress the growth of the tumor cell.

This invention will be now illustrated with reference to the following Examples 1 to 11 which show the production of the compounds of the formula (I) according to the first aspect of this invention, along with Referential Examples 1 to 3 which show the preparation of several examples of the necessary starting compounds therefor, and also with reference to the following Examples 12 to 19 which show the production of the compounds of the formula (I′) according to the sixth aspect of this invention, along with Referential Example 4 which shows the preparation of one example of the necessary starting compounds. This invention is not limited to these Examples.

Referential Example 1

(1) Preparation of 3,4-di-O-acetyl-2,6-dideoxy-2-fluoro-α- and β-L-mannopyranosyl fluorides [Compound (g)] and 3,4-di-O-acetyl-2,6-dideoxy-2-fluoro-α- and -β-L-mannopyranoses [Compound (h)]

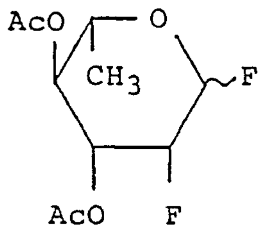

Compound (g)

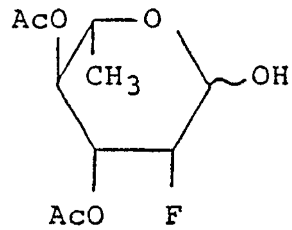

Compound (h)

Di-O-acetyl-6-deoxy-L-glucal of the formula (f) above-described [W. Roth and W. Pigman, "Methods in Carbohydr. Chem.", 2, p.p. 407-408, Academic Press Inc., New York and London (1963)] (2.03 g) was dissolved in a mixed solvent of acetonitrile (40 ml) and water (4 ml), followed by bubbling of argon gas containing 5% of fluorine (about 6 ℓ over 6 hours) into the solution at room temperature to conduct the fluorination.

The reaction solution was concentrated and the resultant syrup was dissolved in chloroform (about 100 ml). The chloroform solution thus-prepared was washed successively with an aqueous solution of sodium bicarbonate and with water and then dried over anhydrous sodium sulfate. The aqueous layer (the washings) was extracted with chloroform (about 50 ml x 3). The extracts were combined with the above-mentioned chloroform solution, followed by drying. The chloroform solution was concentrated and then dried under reduced pressure, so that there was obtained a mixture (2.62 g) of a pale yellow syrup and colorless crystals, which contained the titled compounds. The mixture was used as a raw material for the next reaction stage without any further purification. This mixture contained the target compounds of the formulae (g) and (h) as well as their 2-F epimers.

(2) Preparation of 1,3,4-tri-O-acetyl-2,6-dideoxy-2-fluoro-α- and β-L-mannopyranoses

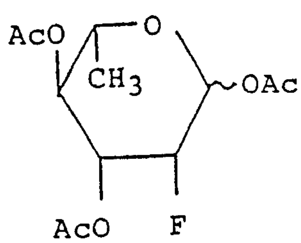

Compound (i)

The mixture (2.62 g) obtained in Referential Example 1-(1), which contained the compound (h), was dissolved in a mixed solvent of acetic acid (25 ml) and acetic anhydride (25 ml). After ice-cooling, concentrated sulfuric acid (0.5 ml) was added to conduct the acetolysis of the compounds (g) and (h) overnight at room temperature.

The resulting reaction solution was taken up into chloroform (about 500 ml). The resultant solution was washed successively with an aqueous solution of sodium bicarbonate and with water, dried over anhydrous sodium sulfate and then concentrated under reduced pressure to obtain a pale yellow syrup (2.80 g). The syrup was purified on a silica gel column ["Wako Gel·C-200", 250 g; developing system: a (10:1) mixture of toluene and ethyl acetate]. A mixture of the titled compounds of the formula (i), where the ratio of the α-L-isomer to the β-L-isomer was about 8:1, was obtained as colorless crystals in a yield of 792 mg (yield: 29% based on di-O-acetyl-6-deoxy-L-glucal).

α-L-Isomer:

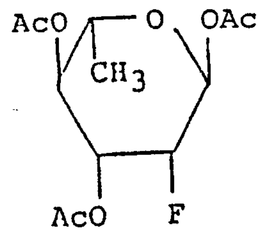

$^1$H-NMR spectrum ($CDCl_3$, TMS as internal standard, 250 $MH_z$):
δ 6.21 (dd, H-1)
4.73 (dt, H-2)
$J_{H-1,H-2}$ = $J_{H-2,H-3}$ = 2.5 Hz
$^{19}$F-NMR spectrum ($CDCl_3$, $CFCl_3$ as internal standard, 235 MHz):
δ-204.2 (ddd, F-2, $J_{F-2,H-2}$48, $J_{F-2,H-3}$ 29, $J_{F-2,H-1}$ 7 Hz)

β-L-Isomer:

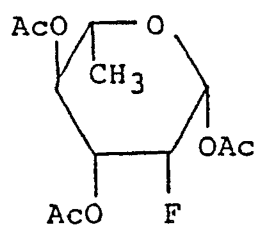

$^1$H-NMR spectrum ($CDCl_3$, TMS as internal standard, 250 MHz):
δ 5.77 (d, H-1),
$^{19}$F-NMR spectrum ($CDCl_3$, $CFCl_3$ as internal standard, 235 MHz):
δ-220.2 (dddd, F-2, $J_{F-2,H-2}$ 52,
$J_{F-2,H-3}$ 27, $J_{F-2,H-1}$ 19, $J_{F-2,H-4}$ 2 Hz)

(3) Preparation of 3,4-di-O-acetyl-2,6-dideoxy-2-fluoro-α-L-mannopyranosyl bromide

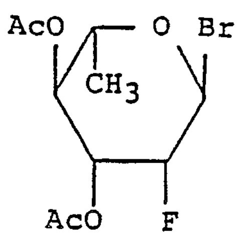

Compound (j)

Crystals (300 mg) of 1,3,4-tri-O-acetyl-2,6-dideoxy-2-fluor-α- and β-L-mannopyranose obtained in the preceding procedure (2) were dissolved in a (1:1) mixed solvent (3 ml) of acetic acid and acetic anhydride. An acetic acid solution (6 ml) containing 30% of hydrogen bromide was added, followed by a reaction for 1 hour to conduct the bromination of the compound (i).

After dissolving the resulting reaction solution in chloroform (100 ml), the resultant solution was washed successively with an aqueous solution of sodium bicarbonate and with water, dried over anhydrous sodium sulfate and then concentrated under reduced pressure. The titled compound was obtained as a colorless

syrup in a yield of 310 mg (97%). $^1$H-NMR spectrum ($CDCl_3$, TMS as internal standard, 250 MHz):
δ 6.40 (dd, H-1)
4.97 (ddd, H-2)
5.60 (ddd, H-3)
$^{19}$F-NMR spectrum ($CDCl_3$, $CFCl_3$ as internal standard 235 MHz):
δ-181.6 (dddt, F-2, $J_{F-2,H-2}$ 50,
$J_{F-2,H-3}$ 27, $J_{F-2,H-1}$ 10,
$J_{F-2,H-4}$ = $J_{F-2,H-5}$ 1 Hz)

Example 1

(a) Preparation of 7-O-(3,4-di-O-acetyl-2,6-dideoxy-2-fluoro-α-L-mannopyranosyl)daunomycinone

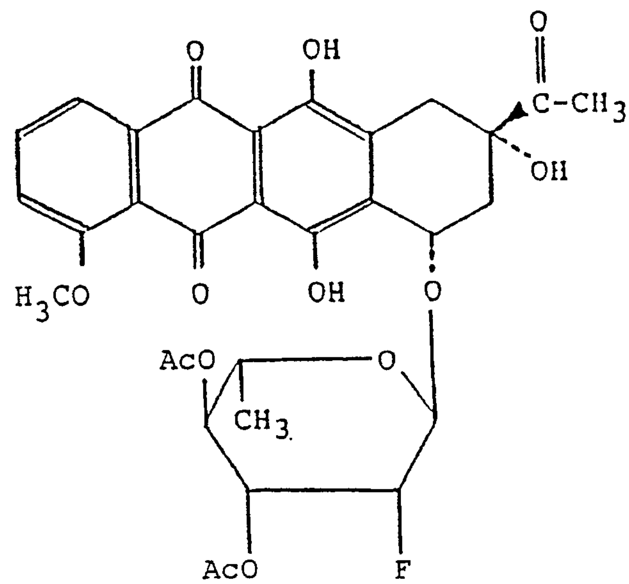

Daunoymcinone (32.0 mg) was dissolved in a mixture of 3,4-di-O-acetyl-2,6-dideoxy-2-fluoro-α-L-mannopyranosyl bromide (37.0 mg, 1.5 molar equivalents) and anhydrous dichloromethane (8 ml), and the resulting liquid solution was stirred overnight at room temperature in the presence of mercuric oxide (81.0 mg, 4.7 molar equivalents), mercuric bromide (23.0 mg, 0.8 molar equivalent) and molecular sieve 3A (150 mg) to conduct the condensation reaction.

The reaction mixture was filtered to remove the solids which were then washed with chloroform. The filtrate was combined with the chloroform washing. The chloroform solution thus obtained was washed successively with an aqueous solution of potassium iodide, an aqueous solution of sodium bicarbonate and water, dried over anhydrous sodium sulfate, and then concentrated under reduced pressure. A solid (70.3 mg) thus obtained was purified on a silica gel column ["Wako Gel C-300", 5 g; developing system: a (20:1) mixture of chloroform and acetone]. The titled compound was obtained as a red solid in a yield of 35.6 mg (yield: 71% based on daunomycinone). In addition, daunomycinone (3.2 mg, 10%) was recovered.
$[\alpha]_D^{24}$ +232° (c 0.1, chloroform) m.p. 137-141 °C
$^1$H = NMR spectrum ($CDCl_3$, TMS as internal standard (hereinafter abbreviated as i.s.), 250 MHz):
δ 5.53 (dd, H-1′)
4.78 (dt, H-2′)
4.06 (s, $OCH_3$ at the 4-position)
2.43 (s, 3H at the 14-position)
$^{19}$F-NMR spectrum ($CDCl_3$, $CFCl_3$ as i.s., 235 MHz):
δ-204.9 (F-2′, $J_{F-2′, H-1}$ 8 Hz)

(b) Preparation of 7-O-(2,6-dideoxy-2-fluoro-α-L-mannopyranosyl)daunomycinone

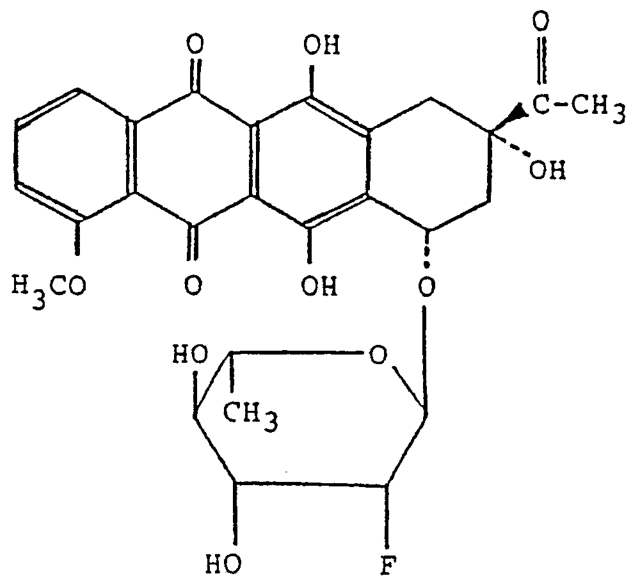

7-O-(3,4-Di-O-acetyl-2,6-dideoxy-2-fluoro-α-L- mannopyranosyl)daunomycinone (96.0 mg) obtained in the above procedure (a) was suspended in an ice-cooled 0.2 M aqueous solution of sodium hydroxide (9.6 ml), followed by stirring at the same temperature (namely, under ice-cooling) to conduct the deacetylation reaction (for the deprotection).

After adding 1 M hydrochloric acid (1.5 ml) to the resultant homogeneous reaction solution of a purple color to adjust it to pH 3-4, sodium chloride (2.1 g) was added, followed by extraction with chloroform (20 ml x 4). The combined chloroform solution (the extract)was washed with saturated saline, drided over sodium sulfate and then concentrated under reduced pressure. The titled compound was obtained as a red solid in a yield of 73.6 mg (86%).

$[\alpha]_D^{24}$ +164° (c 0.1, chloroform)

$^{1}$H-NMR spectrum ($CDCl_3$, TNS as i.s., 250 MHz):

δ 5.53 (dd, H-1′)

4.66 (dt, H-2′)

4.06 (s, $OCH_3$ at the 4-position)

2.39 (s, 3H at the 14-position)

$^{19}$F-NMR spectrum ($CDCl_3$, $CFCl_3$ as i.s., 235 MHz):

δ-205.2(F-2′ $J_{F\text{-}2'}$, H-1′ 8Hz)

$^{13}$C-NMR spectrum (deutero-pyridine, namely deuterated pyridine; TMS as i.s., 62.9 MHz):

δ 102.0 (C-1′ $J_{c\text{-}1'}$, F-2′ 30.4 Hz)

91.9 (C-2′, $J_{c\text{-}2'}$, F-2′ 174.2 Hz)

24.5 (C-14)

Example 2

(a) Preparation of 7-O-(3,4-di-O-acetyl-2,6-dideoxy-2-fluoro-α-L-mannopyranosyl)-4-demethoxydaunomycinone

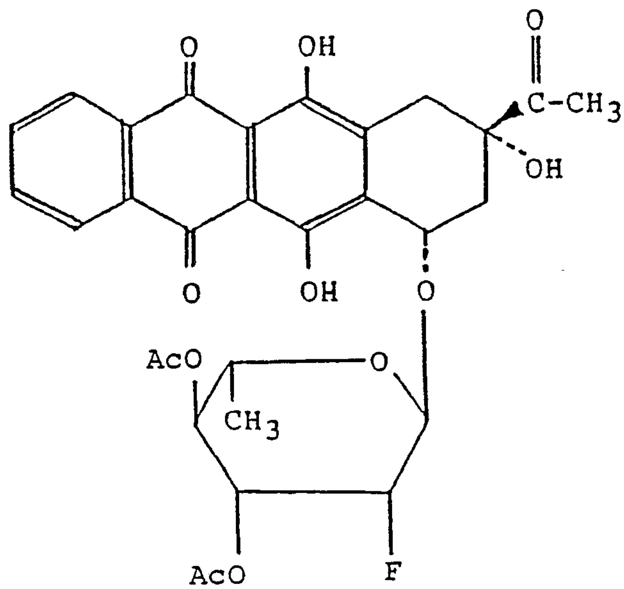

The condensation between the starting 4-demethoxydaunomycinone (53.3 mg) and 3,4-di-O-acetyl-2,6-dideoxy-2-fluoro-α-L-mannopyranosyl bromide (66.6 mg) as employed in Example 1, was conducted and the purification of the reaction product was conducted in the same manner as in Example 1-(a). The titled compound was obtained as an orange solid in a yield of 63.6 mg (73% based on 4-demethoxydaunomycinone).
$[\alpha]_D^{24}$ +158° (c 0.1, chloroform)

(b) Preparation of 4-demethoxy-7-O-(2,6-dideoxy-2-fluoro-α-L-mannopyranosyl)daunomycinone

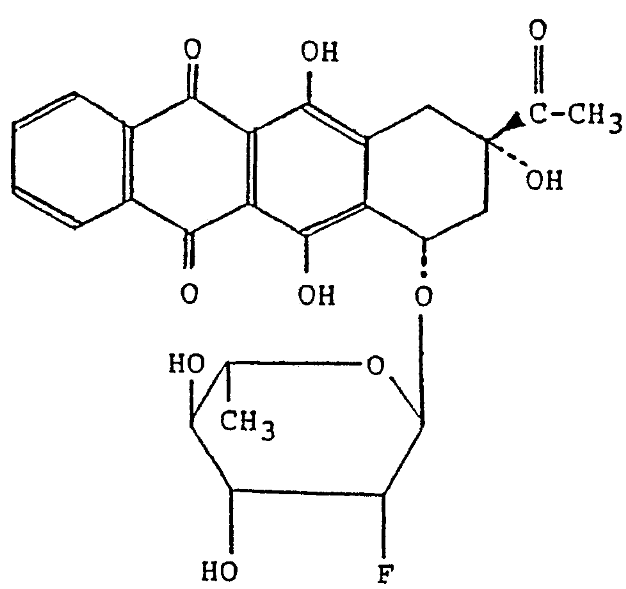

7-O-(3,4-Di-O-acetyl-2,6-dideoxy-2-fluoro-α-L-mannopyranosyl)-4-demethoxydaunomycinone (103 mg) obtained as above was deacetylated in the same manner as in Example 1-(b). The titled compound was obtained as an orange solid in a yield of 75.6 mg (83%).
$[\alpha]_D^{24}$ +107° (c 0.1, chloroform)

Referential Example 2

(a) Preparation of 7-O-(2,6-dideoxy-2-fluoro-α-L-mannopyranosyl)-14-bromodaunomycinone

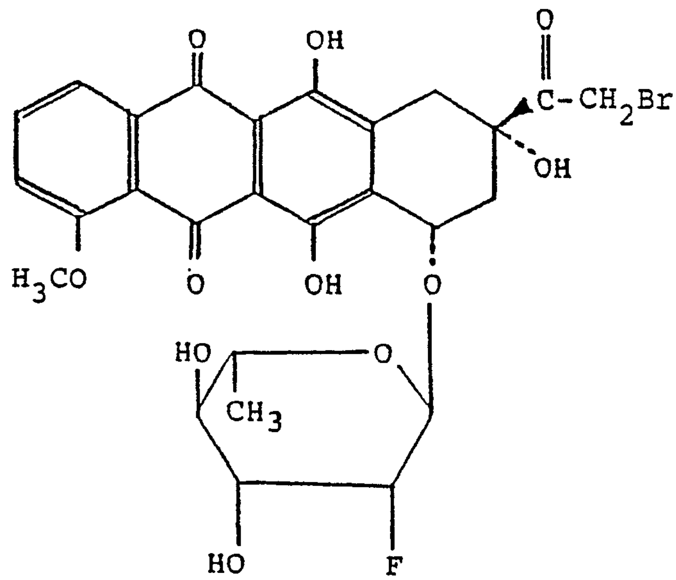

7-O-(2,6-Dideoxy-2-fluoro-α-L-mannopyranosyl)daunomycinone (50.0 mg) was dissolved in a mixed solvent of anhydrous methanol (1 ml) and anhydrous dioxane (2 ml), followed by addition of methyl orthoformate (66.0 mg, 7 molar equivalents). While stirring the resulting reaction mixture under ice-cooling, a solution of bromine (28.0 mg) in anhydrous dichloromethane (0.28 ml) was added. The resulting mixture was stirred for 30 minutes at the same temperature (namely, under ice-cooling), followed by continuing the reaction overnight at room temperature. As a result, the dimethylketal formation at the 13-carbonyl group and the bromination of the 14-methyl group as described hereinbefore had been effected. The resultant reaction solution was concentrated and the resultant solid was dissolved in acetone (3 ml). The solution thus obtained was left over for 30 minutes at room temperature, whereby the removal of the 13-ketal group was effected.

The reaction solution so obtained was concentrated to give a solid. The solid was washed with isopropyl ether and then dried under reduced pressure, thereby affording 57.0 mg of a red solid comprising principally the titled compound. The solid was used in the reaction of Example 3 below, without any further purification.

Example 3

Preparation of 7-O-(2,6-dideoxy-2-fluoro-α-L-mannopyranosyl)adriamycinone

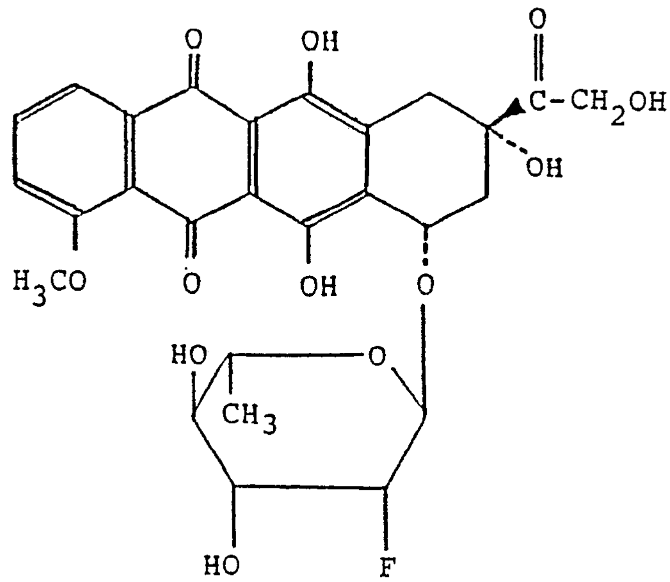

7-O-(2,6-Dideoxy-2-fluoro-α-L-mannopyranosyl)-14-bromodaunomycinone (57.0 mg) as obtained in Referential Example 2 and sodium formate (85.0 mg) were taken up in a (4:1) mixture (5 ml) of acetone and water, followed by vigorously stirring the resulting mixture overnight at room temperature in mixed solvent (5ml) of acetone and water, so that the hydrolysis was effected.

The reaction solution obtained was concentrated to afford a red solid, which was then washed with water and dried under reduced pressure. Since the red solid thus obtained contained a small amount of the 14-O-formyl derivative in addition to the titled compound, the red solid was dissolved in a (1:1) mixed solvent (5 ml) of chloroform and methanol. 1 M Aqueous ammonia (0.2 ml) was added under ice-cooling, followed by effecting the hydrolytic reaction for 10 minutes at the same temperature under ice-cooling (for the removal of the 14-O-formyl group). The resulting reaction solution was added with a mixture of chloroform (5 ml) and water (5 ml). After thoroughly shaking the resultant mixture, the chloroform layer was recovered and the aqueous layer was extracted with chloroform (5 ml x 5). The chloroform layer and the chloroform extracts were combined together, washed with 20% aqueous sodium chloride, dried over sodium sulfate, and then concentrated under reduced pressure to give a solid. The solid was thereafter purified by preparative thin layer chromatography [developer: a (10:1) mixed solvent of chloroform and methanol]. The titled compound was obtained as a red solid in a yield of 26.4 mg (51%).

$[\alpha]_D^{24}$ +110° (c 0.1, chloroform)

m.p. 120-123° C

$^1$H-NMR spectrum (deutero-pyridine, TMS as i.s., 250 MHz):

δ 5.94 (dd, H-1′)
5.34 (dt, H-2′)
3.96 (s,4-$OCH_3$)
5.32 (14-$CH_2$)

$^{19}$F-NMR spectrum (deutero-pyridine, $CFCl_3$, as i.s., 235 MHz);

δ-201.2 (ddd, F-2′, $J_{F-2'}$, H-1′ 8.5 Hz)

$^{13}$C-NMR spectrum (deutero-pyridine, TMS as i.s., 62.9 MHz):

δ 102.0 (c-1′, $J_{C-1'}$, F-2′ 30.5 Hz)
91.9 (C-2′, $J_{C-2'}$, F-2′ 174.3 Hz)
65.5 (C-14)

Referential Example 3

(a) Preparation of 7-O-(2,6-dideoxy-2-fluoro-α-L-mannopyranosyl)-14-bromo-4-demethoxydaunomycinone

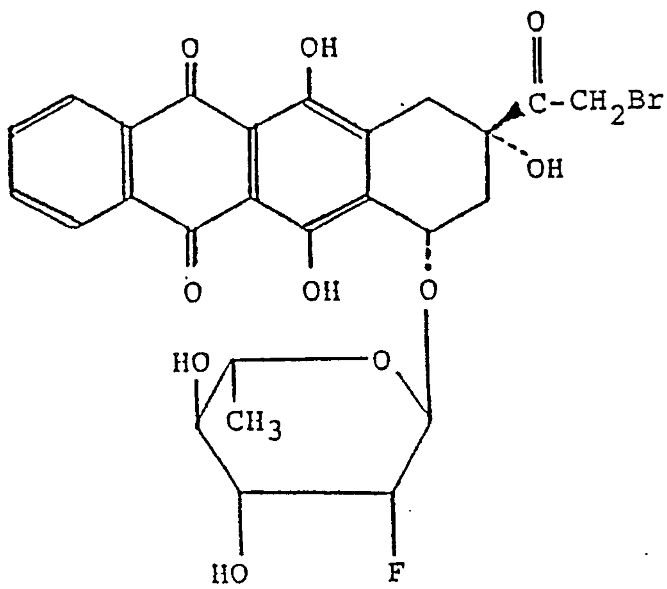

7-O-(2,6-Dideoxy-2-fluoro-α-L-mannopyranosyl)-4-demethoxydaunomycinone (53.4 mg) was brominated at the 14-position by the same reaction and procedure as in Referential Example 2, to afford 58.9 mg of an orange solid comprising principally the titled compound.

Example 4

Preparation of 7-O-(2,6-dideoxy-2-fluoro-α-L-mannopyranosyl)-4-demethoxyadriamycinone

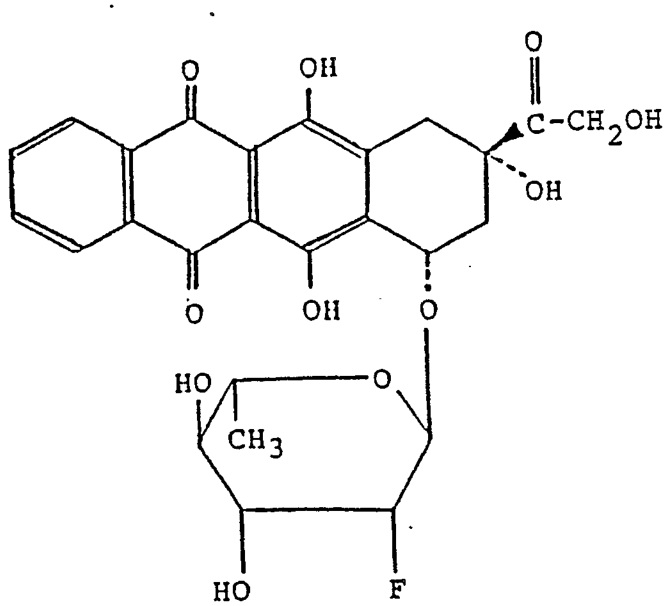

7-O-(2,6-Dideoxy-2-fluoro-α-L-mannopyranosyl)-14-bromo-4-demethoxydaunomycinone (66.8 mg) which was obtained in Referential Example 3, was used as the starting compound and hydrolysed and after-treated in the same manner as in Example 3 with the intermediate formation of the 14-O-formyl derivative, to obtain 32.6 mg (54%) of the titled compound as an orange solid.
$[\alpha]_D^{24}$ +65° (c 0.1, chloroform)

Example 5

Preparation of 7-O-(2,6-dideoxy-2-fluoro-α-L-mannopyranosyl)adriamycinone 14-O-valerate

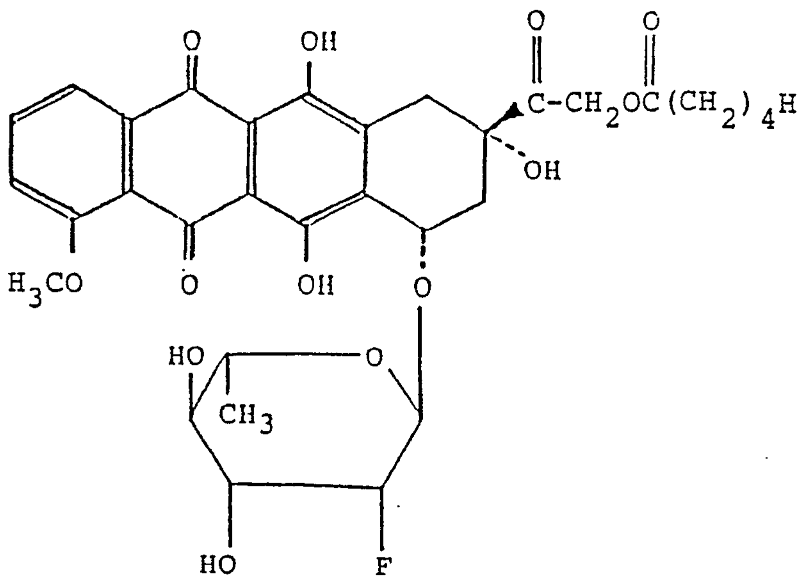

7-O-(2,6-Dideoxy-2-fluoro-α-L-mannopyranosyl)-14-bromodaunomycinone (68.4 mg) as used in Example 3 was dissolved in a mixture of sodium valerate (186 mg) and a (4:1) mixed solvent (7.5 ml) of acetone and water, followed by stirring the resultant solution overnight at room temperature. The condensation reaction between the starting 14-bromodaunomycinone derivative and sodium valerate was effected.

The resulting reaction mixture was concentrated under reduced pressure to obtain a solid, which was then dissolved in a (5:1) mixed solven (12 ml) of chloroform and methanol. The resultant solution was washed with water, dried over sodium sulfate and then concentrated under reduced pressure. A solid (77.8 mg) thus obtained was purified by preparative thin layer chromatography [ developer: a (10:1) mixed solvent of chloroform and methanol ]. The titled compound was obtained as a red solid in a yield of 52.9 mg (75%). $[\alpha]_D^{24}$ +207° [c 0.1, in a (1:1 v/v) mixture of $CHCl_3$ and MeOH]

Example 6

Preparation of 7-O-(2,6-dideoxy-2-fluoro-α-L-mannopyranosyl)adriamycinone 14-O-hemisuccinate

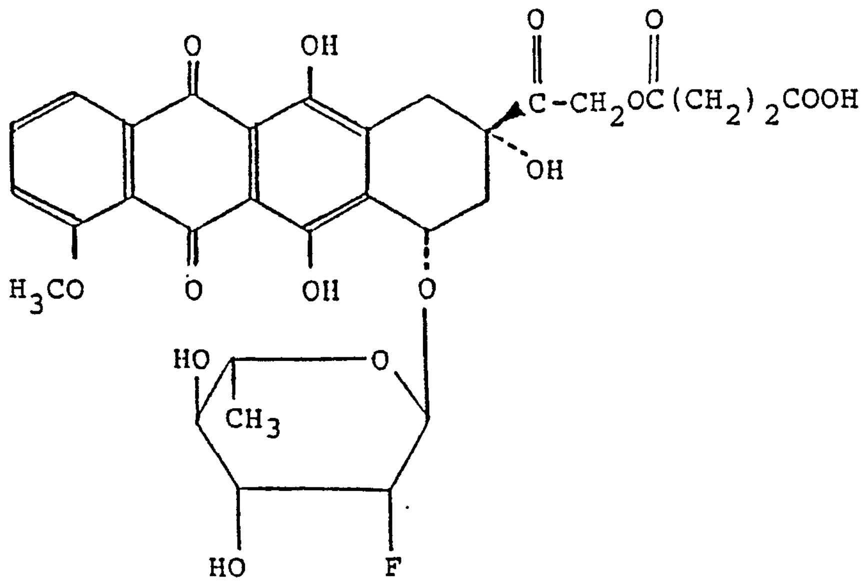

7-O-(2,6-Dideoxy-2-fluoro-α-L-mannopyranosyl)-14-bromodaunomycinone (78.7 mg) as used in Example 3 was reacted with mono-sodium succinate (243 mg) and the reaction mixture was treated in the same manner as in Example 5. The titled compound was obtained as a red solid in a yield of 56.6 mg (68%).
$[\alpha]_D^{24}$ +64° (c 0.05, chloroform)

Example 7

Preparation of 7-O-(2,6-dideoxy-2-fluoro-α-L-. mannopyranosyl)adriamycinone 14-O-hemiglutarate

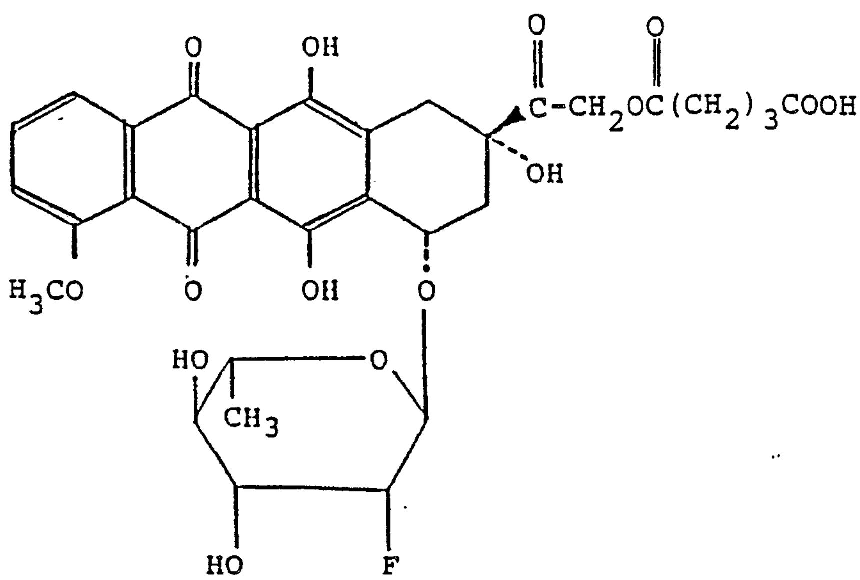

7-O-(2,6-Dideoxy-2-fluoro-α-L-mannopyranosyl)-14-bromodaunomycinone (54.0 mg) as used in Example 3 was reacted with mono-sodium glutarate (183 mg) and the reaction mixture was treated in the same manner as in Example 5. The titled compound was obtained as a red solid in a yield of 46.6 mg (80%).
$[\alpha]_D^{24}$ +167° [c 0.05, in a (1:1 v/v) mixture of $CHCl_3$ and MeOH]

Example 8

Preparation of 7-O-(2,6-dideoxy-2-fluoro-α-L-mannopyranosyl)adriamycinone 14-O-hemiadipate

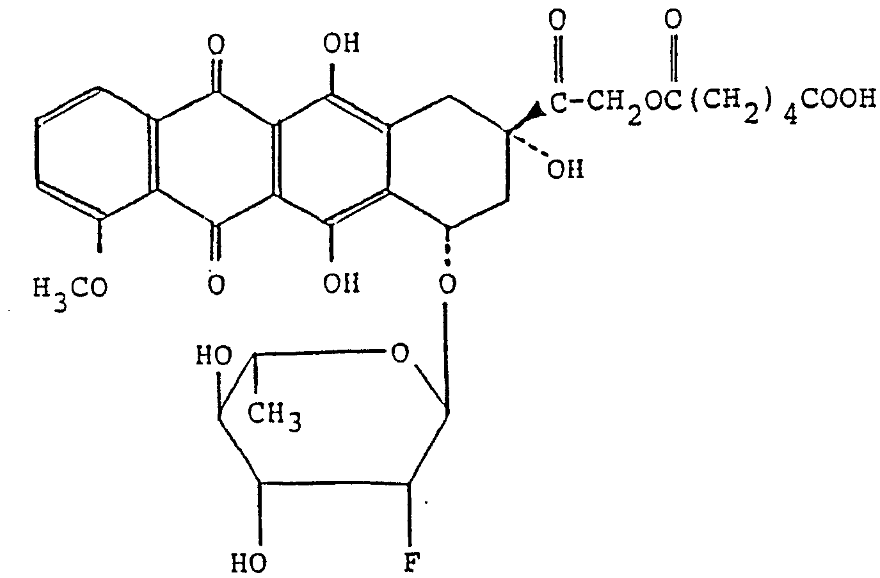

7-O-(2,6-Dideoxy-2-fluoro-α-L-mannopyranosyl)-14-bromodaunomycinone (64.0 mg) as used in Example 3 was reacted with mono-sodium adipate (237 mg) and the reaction mixture was treated in the same manner as in Example 5. The titled compound was obtained as a red solid in a yield of 54.2 mg (77%). $[\alpha]_D^{24}$ +157° [c 0.08, in a (1:1 v/v) mixture of $CHCl_3$ and MeOH]

Example 9

Preparation of 7-O-(2,6-dideoxy-1-fluoro-α-L-mannopyranosyl)adriamycinone 14-O-hemipimelate

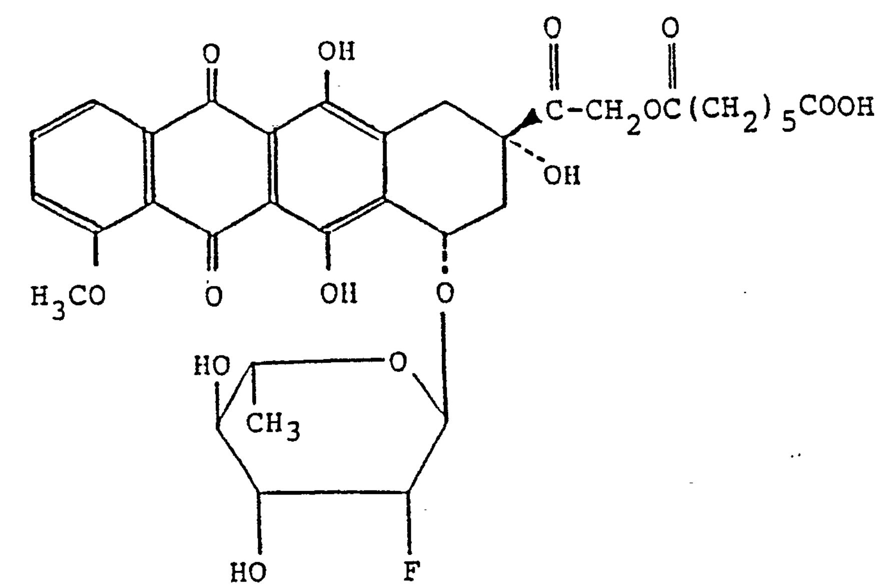

7-O-(2,6-Dideoxy-2-fluoro-α-L-mannopyranosyl)-14-bromodaunomycinone (57.0 mg) as used in Example 3 was reacted with mono-sodium pimelate (227 mg) and the reaction mixture was treated in the same manner as in Example 5. The titled compound was obtained as a red solid in a yield of 46.5 mg (73%).
$[\alpha]_D^{23}$ +190° [c 0.1, in a (1:1 v/v) mixture of $CHCl_3$ and MeOH].

Example 10

Preparation of 7-O-(2,6-dideoxy-2-fluoro-α-L-mannopyranosyl)adriamycinone 14-O-hemisuberate

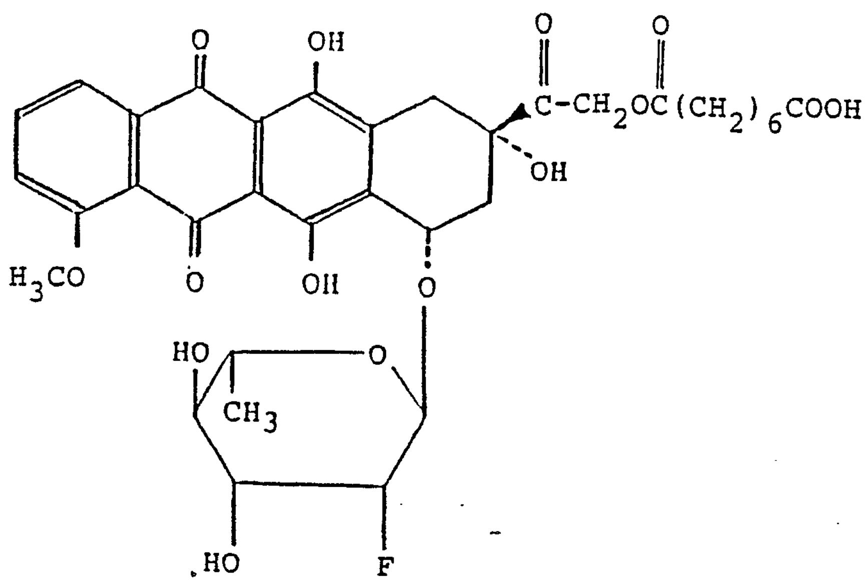

7-O-(2,6-Dideoxy-2-fluoro-α-L-mannopyranosyl)-14-bromodaunomycinone (77.5 mg) as used in Example 3 was reacted with mono-sodium suberate (334 mg) and the reaction mixture was treated in the same manner as in Example 5. The titled compound was obtained as a red solid in a yield of 65.6 mg (74%).
$[\alpha]_D^{23}$ +142° [c 0.1, in a (1:1 v/v) mixture of $CHCl_3$ and MeOH]

EXAMPLE 11

Preparation of 7-O-(2,6-dideoxy-2-fluoro-α-L-mannopyranosyl)-4-demethoxyadriamycinone 14-O-hemipimelate

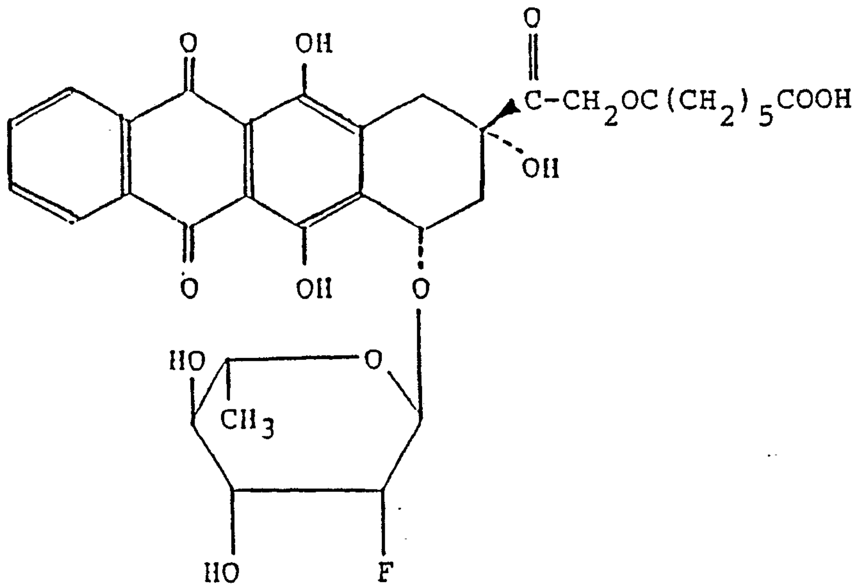

7-O-(2,6-Dideoxy-2-fluoro-α-L-mannopyranosyl)-14-bromo-4-demethoxydaunomycinone (69.0 mg) as used in Example 4 was reacted with mono-sodium pimelate (288 mg) and the reaction mixture was treated in the same manner as in Example 5. The titled compound was obtained as an orange solid in a yield of 58.9 mg (76%).

$[\alpha]_D^{23}$ +162° [c 0.1, in a (1:1 v/v) mixture of $CHCl_3$ and MeOH ]

Example 12

(a) Preparation of 4-demethoxy-7-O-(3,4-di-O-acetyl-2,6-dideoxy-2-fluoro-α-L-talopyranosyl)daunomycinone

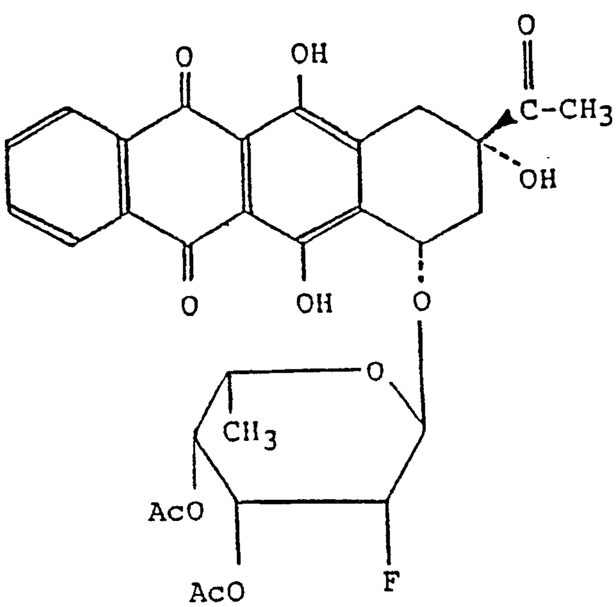

(I'-a-2)

4-Demethoxydaunomicinone (217 mg), mercuric chloride (yellow) (939 mg), mercuric bromide (367 mg) and powdery molecular sieve 3A (2.1 g) were suspended in dry dichloromethane (43 ml). To the resulting suspension was added a solution (5 ml) of 3,4-di-O-acetyl-2,6-dideoxy-2-fluoro-α-L-talopyranosyl bromide (256 mg) [a synthetic intermediate compound described in Japanese Patent application First publication

"Kokai" No. 145097/87 and T. Tsuchiya, Y. Takagi, K-D. OK, T. Takeuchi, N. Wako and H. Umezawa, "The Journal of Antibiotics "Vol. 39, pp. 731-733 (1986) ] in dry dichloromethane, and the mixture obtained was stirred at ambient temperature in the dark for 18 hours, to effect the condensation reaction between the 3,4-di-O-acetyl-2,6-dideoxy-2-fluoro-α-L-talopyranosyl bromide and 4-demethoxydaunomycinone

The reaction solution was filtered, and the filtrate obtained was diluted with chloroform, washed successively with 30% aqueous potassium iodide, saturated aqueous sodium hydrogen carbonate and water. The washed solution was dried over anhydrous sodium sulfate, and concentrated under reduced pressure. The residue obtained was then isolated and purified by silica gel column chromatopraphy [developer: a (8:1) mixture of chloroform and ethyl acetate], to afford the titled compound as a reddish orange solid (yield: 245 mg, 69%). This compound was reprecipitated from a mixture of chloroform and hexane for further purification.

$[\alpha]_D^{23}$ + 144° (c 0.11, chloroform)
¹H-NMR spectrum (CDCl₃):
δ 5.64(1H, dd, H-1′)
4.61(1H, dm H-2′)
2.42(3H, s, Ac)
2.18 and 2.04 (each 3H, s, OAc)

(b) Preparation of 4-demethoxy-7-O-(2,6-dideoxy-2-fluoro-α-L-talopyranosyl)daunomycinone

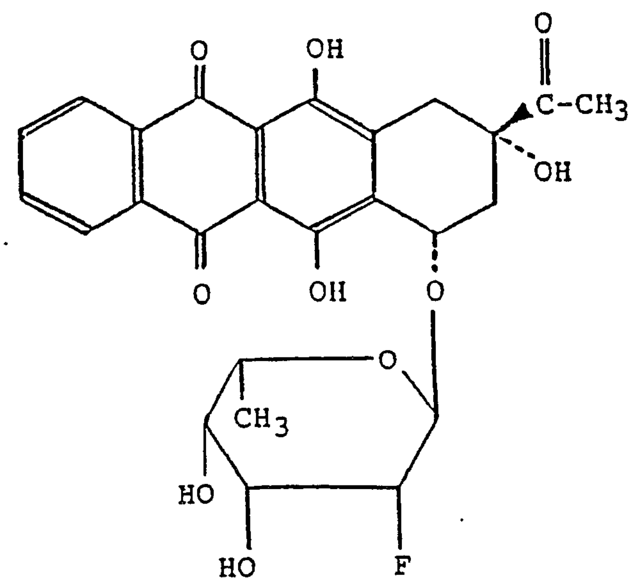

(I'-a)

7-O-(3,4-di-O-acetyl-2,6-dideoxy-2-fluoro-α-L-talopyranosyl)-4-demethoxydaunomycinone (153 mg) as obtained in the above procedure (a) was dissolved in 0.2 N aqueous sodium hydroxide (12 ml). The solution was stirred at 0°C for one hour to effect the hydrolysis for the removal of the acetyl groups from the compound (I′-a-2).

The resulting reaction solution was mixed with 1N aqueous hydrochloric acid (2.7 ml), to which was added sodium chloride (2.2 g). The mixture obtained was extracted with chloroform. The extract in chloroform was washed with 20% aqueous sodium chloride, dried over anhydrous sodium sulfate and concentrated under reduced pressure. The residue was reprecipitated from a mixture of chloroform and hexane to afford the titled compound as an orange solid (yield; 109 mg, 83%)

$[\alpha]_D^{24}$ + 128° [c 0.1, chloroform-methanol (1:1)]
¹H-NMR spectrum (CDCl₃):
δ 5.60 (1H, broad d, H-1′)
4.64 (1H, broad d, H-2′)
2.42 (3H, s, Ac)
¹⁹F-NMR spectrum
(CDCl₃, CFCl₃ as internal standard)

δ-201.1 (dddd)
$J_{F,H-1'}$ 9.5, $J_{F,H-2'}$ 49,
$J_{F,H-3'}$ 33, $J_{F,OH-4'}$ 8.5Hz

Referential Example 4

(a) Preparation of 4-demothoxy-7-O-(2,6-dideoxy-2-fluoro-3,4-O-isopropylidene-α-L-talopyranosyl)-14-bromodaunomycinone

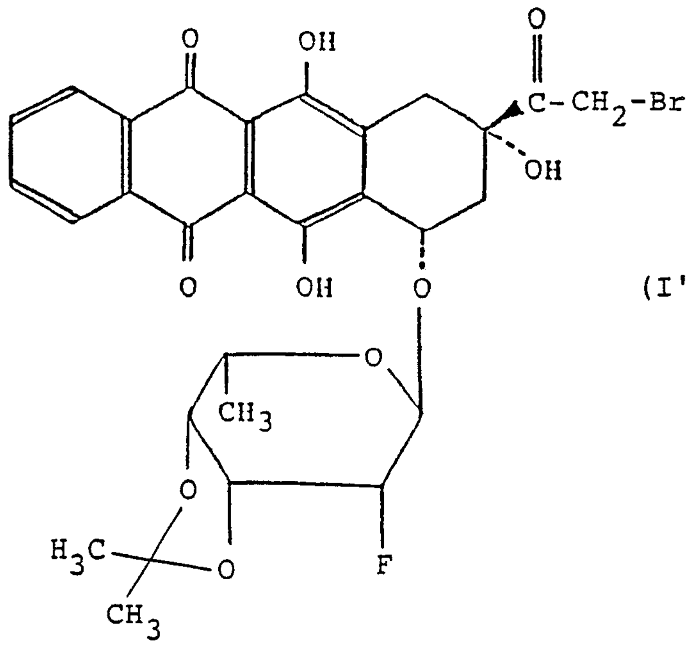

(I'-e-2)

4-Demethoxy-7-O-(2,6-dideoxy-2-fluoro-α-L-talopyranosyl) daunomycinone (50 mg) as obtained in Example 12 above and methyl orthoformate (0.1 ml) were taken up into a mixture of dry methanol (1.2 ml) and dry dioxane (1.8 ml), and the resulting solution was cooled to 0° C. To the cooled solution was added a solution of bromine (24 mg) in dry dichloromethane (0.23 ml). The mixture obtained was stirred at 0° C for 30 minutes and then at ambient temperature for one hour, whereby the 13-carbonyl group of the daunomycinone compound was protected with dimethylketal group, with the 14-methyl group being brominated.

The resulting reaction solution was admixed with isopropyl ether (3 ml) and hexane (38 ml) to deposit a precipitate comprising 4-demethoxy-(2,6-dideoxy-2-fluoro-α-L-talopyranosyl)-14-bromodaunomycinone 13-dimethylketal. The precipitated solid was collected by centrifugation and washed twice with isopropyl ether. The solid product compound was dissolved in acetone (8 ml) and the solution was kept at ambient temperature for 1.5 hours, so that the 13-dimethylketal group was removed with the free 13-carbonyl group being regenerated, and concurrently an isopropylidene group was introduced into the 3′- and 4′- hydroxyl groups of the compound due to the participation of the acetone in the reaction with these hydroxyl groups. The resulting reaction solution was concentrated and dried under reduced pressure to afford the titled compound as an orange solid (yield; 56 mg, 91%).

Example 13

Preparation of 4-demethoxy-7-O-(2,6-dideoxy-2-fluoro-α-L-talopyranosyl)adriamycinone

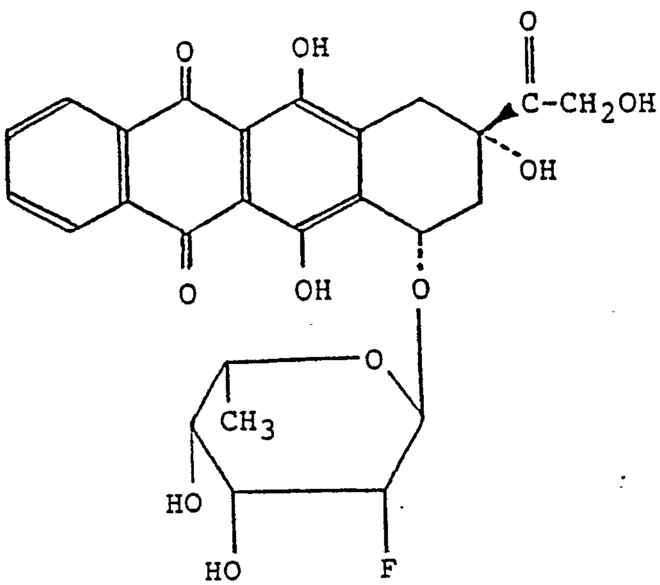

(I'-b)

4-Demethoxy-7-O-(2,6-dideoxy-2-fluoro-3,4-O-isopropylidene-α-L-talopyranosyl)-14-bromodaunomycinone (41 mg) as obtained in the Referential Example 4 was dissolved in acetone (6 ml). To the solution was added an aqueous solution (1.5 ml) of sodium formate (99 mg), and the resulting mixture was stirred vigorously at ambient temperature for 19 hours to effect the reaction. The reaction solution was concentrated to a small volume, and then was added with water, and ice-cooled. Thus, a precipitate comprising 4-demethoxy-7-O-(2,6-dideoxy-3,4-O-isopropylidene-2-fluoro-α-L-talopyranosyl)-adriamycinone was deposited. The precipitated solid was collected by centrifugation, washed with water and dried.

The solid product so obtained was dissolved in a mixture (3.8 ml) of chloroform and methanol (1:1), to which was added 1N aqueous ammonia (0.5 ml) at 0° C. The mixture was stirred at 0° C for 30 minutes to effect the hydrolytic reaction for the removal of the 14-O-formyl group which had been introduced into a portion of the intermediate compound as formed during the treatment with sodium formate. The reaction solution was then admixed with water and extracted with chloroform. The organic extract in chloroform was washed with 20% aqueous sodium chloride, dried over anhydrous sodium sulfate and concentrated under reduced pressure, to afford an orange solid (37 mg) comprising 4-demethoxy-7-O-(2,6-dideoxy-3,4-O-isopropylidene-2-fluoro-α-L-talopyranosyl)adriamycinone.

This solid product was dissolved in 80% aqueous acetic acid (4 ml), and the resulting solution was stirred at 80° C for 15 minutes to effect the hydrolysis reaction for the removal of the 3′,4′-O-isopropylidene group. The reaction solution was mixed with water (15 ml) and sodium chloride (300 mg), and the mixture was extracted with chloroform. The organic extract in chloroform was washed successively with saturated aqueous sodium hydrogen carbonate and 20% aqueous sodium chloride. The washed solution was dried over anhydrous sodium sulfate and concentrated under reduced pressure. For the purification, the residue obtained was subjected to a column chromatography on silica gel as developed with chloroform-methanol (15:1) as eluent. In this way, the titled compound was obtained as an orange solid (yield; 23.4 mg, 68%). This solid was reprecipitated from a mixture of chloroform-methanol-hexane.

$[\alpha]_D^{22}$ + 115° (c 0.1, chloroform-methanol (1:1)

$^1$H-NMR spectrum ($CDCl_3$):

δ 5.62 (1H, broad d, H-1′)

4.62 (1H, dm, H-2′)

4.76 (2H, d,-$CH_2OH$)

$^{19}$F-NMR spectrum ($CDCl_3$, $CFCl_3$ as internal standard):

δ-200.9 (dddd)

$J_{F,H\text{-}1'}$ 10, $J_{F,H\text{-}2'}$ 49,

$J_{F,H\text{-}3'}$ 33, $J_{F,OH\text{-}4'}$ 8Hz

Example 14

Preparation of 4-demothoxy-7-O-(2,6-dideoxy-2-fluoro-α-L-talopyranosyl)adriamycinone 14-O-valerate

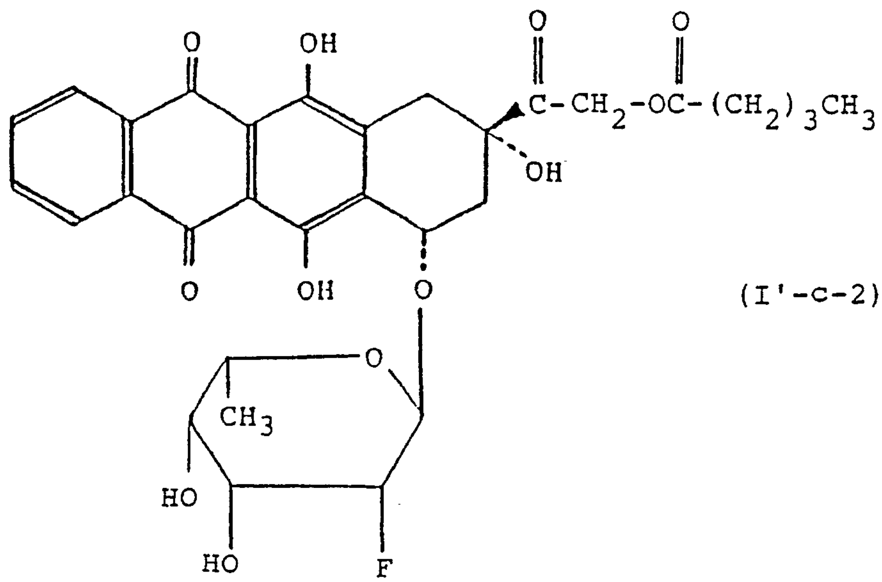

(I'-c-2)

4-Demethoxy-7-O-(2,6-dideoxy-2-fluoro-3,4-O-isopropylidene-α-L-talopyranosyl)-14-bromodaunomycinone (82 mg) of the formula (I′-e-2) as obtained in the Referential Example 4 was dissolved in acetone (10 ml). The solution was mixed with an aqueous solution (3.2 ml) of sodium valerate (290 mg), and the resultant mixture was stirred vigorously at ambient temperature for 18 hours to effect the condensation reaction between these two starting compounds. The reaction solution was concentrated under reduced pressure to remove acetone therefrom, and the residue obtained was extracted with chloroform. The extract in chloroform was washed with water, dried over anhydrous sodium sulfate and concentrated under reduced pressure. The residue obtained was purified by subjecting to a column chromatography on silica gel (20 ml) as developed with chloroform-methanol (20:1). In this way, 4-demethoxy-7-O-(2,6-dideoxy-2-fluoro-3,4-O-isopropylidene-α-L-talopyranosyl(adriamycinone 14-O-valerate (51 mg) was obtained as an orange solid.

This solid product obtained was dissolved in 80% aqueous acetic acid (3 ml ), and the solution was stirred atd 80°C for 15 minutes to effect the hydrolysis reaction for the removal of the 3′,4′-O-isopropylidene group. The reaction solution containing the above titled compound as formed was diluted with water, and the diluted solution was extracted with chloroform. The organic extract in chloroform was washed once with water and concentrated to a small volume under reduced pressure, to which was added hexane to deposit a precipitate comprising the titled compound. For the purification, the precipitate as separated was subjected to a column chromatography on silica gel (10 ml) as developed with chloroform-methanol (10:1) as eluent. In this way, the titled compound was obtained as an orange solid (yield; 41 mg, 51%).

$[\alpha]_D^{24}$ + 141° [c 0.1, chloroform-methanol (1:1)]

## Example 15

Preparation of 4-demethoxy-7-O-(2,6-dideoxy-2-fluoro-α-L-talopyranosyl)adriamycinone 14-O-hemisuccinate

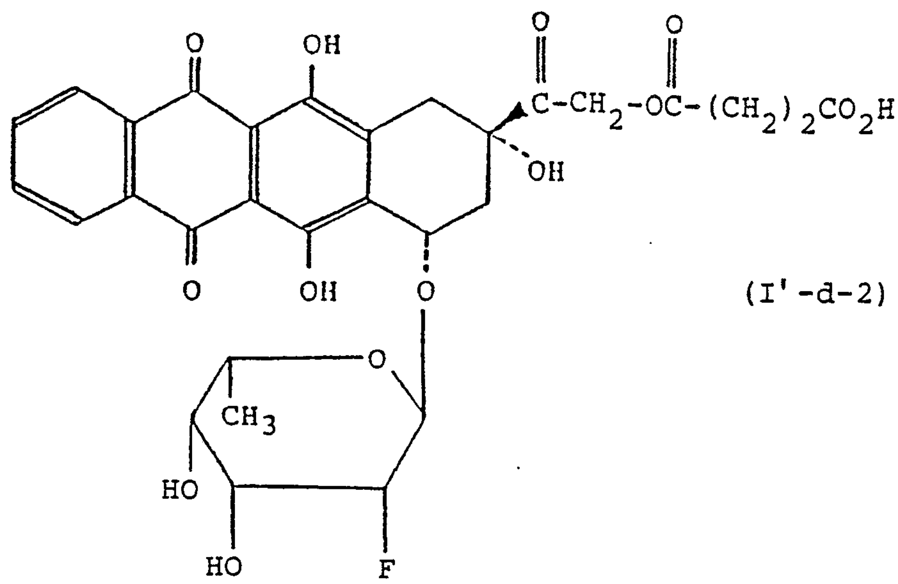

(I'-d-2)

The compound of the formula (I′-e-2) (100 mg) as obtained in the Referential Example 4 and mono-sodium succinate (390 mg) were used as the starting materials and reacted with each other and the resulting reaction solution containing the condensation product as formed was after-treated according to the same procedure as in the Example 14, so that the corresponding hemiester compound, namely 4-demethoxy-7-O-(2,6-dideoxy-2-fluoro-3,4-O-isopropylidene-α-L- talopyranosyl)adriamycinone (14-O-hemi-succinate (79 mg) was obtained. This hemiester compound was then treated with 80% aqueous acetic acid in the same manner as in the Example 14, to effect the hydrolysis reaction for the removal of the 3′,4′-O-isopropylidene group from said hemiester compound. In this way, the titled compound was obtained as an orange solid (yield; 59 mg,59%).
$[\alpha]_D^{24}$ + 104° [c 0.05, chloroform-methanol (1:1)]

Example 16

Preparation of 4-demethoxy-7-O-(2,6-dideoxy-2-fluoro-α-L-talopyranosyl)adriamycinone 14-O-hemiglutarate

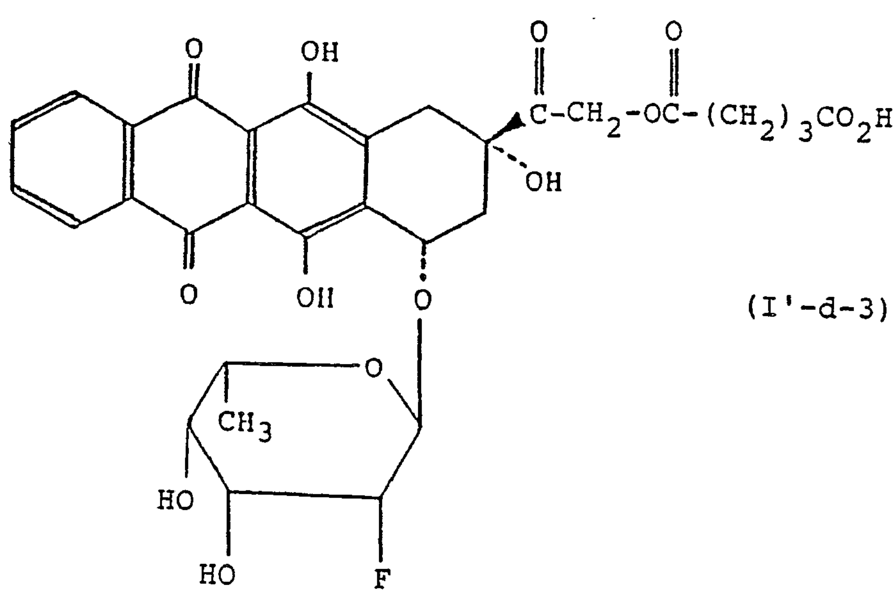

(I'-d-3)

The compound of the formula (I′-e-2) (113 mg) as obtained in the Referential Example 4 and mono-sodium glutarate (490 mg) were used as the starting materials and reacted with each other and the resulting reaction solution containing the condensation product was after-treated according to the same procedure as in the Exmaple 14, so that the corresponding hemi-ester compound, namely 4-demethoxy-7-O-(2,6-dideoxy-2-fluoro-3,4-O-isopropylidene-α-L-talopyranosyl)adriamycinone 14-O-hemiglutarate (82 mg) was obtained. This hemi-ester compound was then treated with 80% aqueous acetic acid in the same manner as in the Example 14, to effect the hydrolysis for the removal of the 3′,4′-O-isopropylidene group from said hemi-ester compound. In this way, the titled compound was obtained as an orange solid (yield; 64 mg, 56%).

]α]$_D^{24}$ + 118 [c 0.05, chloroform-methanol (1:1)]

This 14-O-hemiglutarate is soluble in the phosphate buffer solution (comprising 0.05 M $KH_2PO_4$-NaOH) at pH 7.4.

Example 17

Preparation of 4-demethoxy-7-O-(2,6-dideoxy-2-fluoro-α-L-talopyranosyl)adriamycinone 14-O-hemiadipate

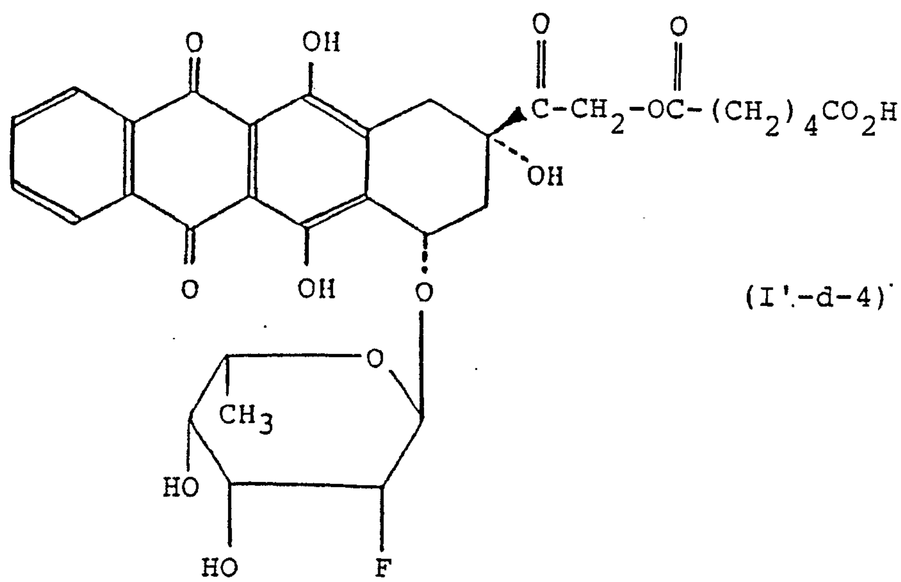

(I′-d-4)

The compound of the formula (I′-e-2) (53 mg) as obtained in the Referential Example 4 and mono-sodium adipate (250 mg) were used as the starting materials and reacted with each other and the resulting reaction solution containing the condensation product as produced was after-treated according to the same procedure as in the Example 14, so that the corresponding hemi-ester compound, namely 4-demethoxy-7-O-(2,6-dideoxy-2-fluoro-3,4-O-isopropylidene-α-L-talopyranosyl)adriamycinone 14-O-hemiadipate (45 mg) was obtained. This hemiester compound was then treated with 80% aqueous acetic acid in the same manner as in the Example 14, to effect the hydrolysis reaction for the removal of the 3′,4′-O-isopropylidene group from the hemi-ester compound. In this way, the titled compound was obtained as an orange solid (yield; 34 mg, 62%).

[α]$_D^{24}$ + 115° [c 0.1, chloroform-methanol (1:1)]

$^1$H-NMR spectrum (CDCl$_3$-CD$_3$OD, 1:1)

δ 5.53(1H, dd, H-1′)

4.58(1H, dm, H-2′)

5.33 and 5.16 (each 1H, d, H-14a,b)

2.51 and 2.35 (each 2H, t, α-and δ-$CH_2$ of hemiadipate group)

1.78-1.69 (4H, m, β-and γ-$CH_2$ of hemiadipate)

$^{19}$F-NMR spectrum (CDCl$_3$-CD$_3$OD (1:1), CFCl$_3$ as internal standard)

δ-201.4 (ddd)

$J_{F,H\text{-}1'}$ 10, $J_{F,H\text{-}2'}$ 49.5,

$J_{F,H-3'}$ 33.5Hz

Example 18

Preparation of 4-demethoxy-7-O-(2,6-dideoxy-2-fluoro-α-L-talopyranosyl)adriamycinone 14-O-hemipimelate

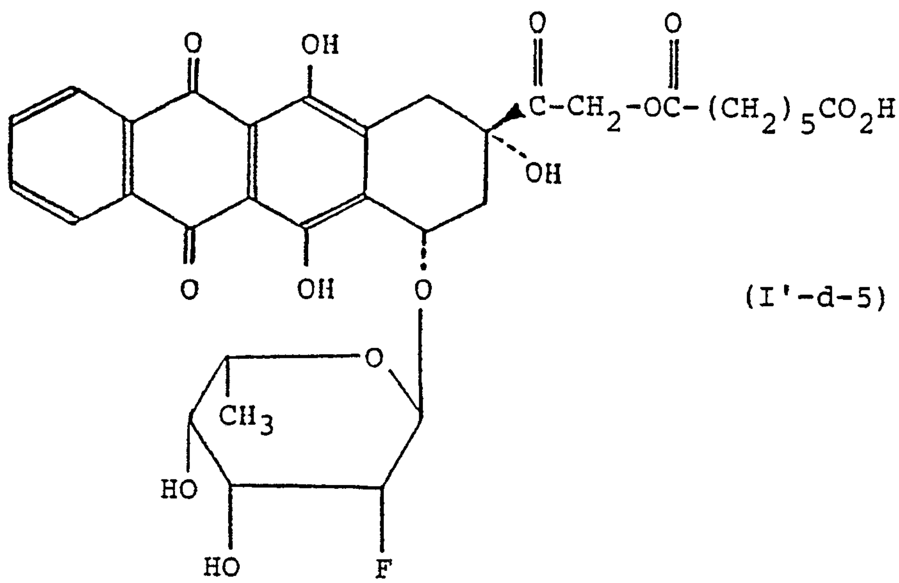

(I'-d-5)

The compound of the formula (I'-e-2) (62 mg) as obtained in the Referential Example 4 and mono-sodium pimelate (250 mg) were used as the starting materials and reacted with each other and the resulting reaction solution containing the condensation product was after-treated according ot the same procedure as in the Example 14, so that the corresponding hemi-ester compound, namely 4-demethoxy-7-O-(2,6-dideoxy-2-fluoro-3,4-O-isopropylidene-α-L-talopyranosyl) adriamycinone 14-O-hemipimelate (49 mg) was obtained. This hemi-ester compound was treated with 80% aqueous acetic acid in the same manner as in the Example 14 to effect the hydrolysis reaction for the removal of the 3',4'-O-isopropylidene group from the hemiester compound. The captioned compound was obtained as an orange solid (yield; 31 mg, 47%).

$[\alpha]_D^{23}$ + 119° (c 0.1, chloroform-methanol (1:1)

$^1$H-NMR spectrum ($CDCl_3$-$CD_3OD$, 1:1)

δ 5.54(1H, broad, d, H-1')

4.58(1H, dm, H-2')

5.32 and 5.16 (each 1H, d, H-14a,b)

2.49 and 2.33 (each 2H, t, α-and ε-$CH_2$ of hemipimelate group)

1.79-1.61 (4H, m, β-and δ-$CH_2$ of hemipimelate group)

1.45 (2H, m, γ-$CH_2$ of hemipimelate group)

$^{19}$F-NMR spectrum

($CDCl_3$-$CD_3OD$ (1:1), $CFCl_3$ as internal standard)

δ-201.3 (ddd)

$J_{F,H-1'}$ 10, $J_{F,H-2'}$ 49.5,

$J_{F,H-3'}$ 34,

The titled compound, 14-O-hemipimelate is dissimilar to the 14-O-hemiadipate of the Example 17 in that the 14-O-hemipimelate is scarcely soluble in te phosphate buffer solution [comprising 0.05 M $KH_2PO_4$-NaOH] at pH 7.4.

Example 19

Preparation of 4-demethoxy-7-O-(2,6-dideoxy-2-fluoro-α-L-talopyranosyl)adriamycinone 14-O-hemisuberate

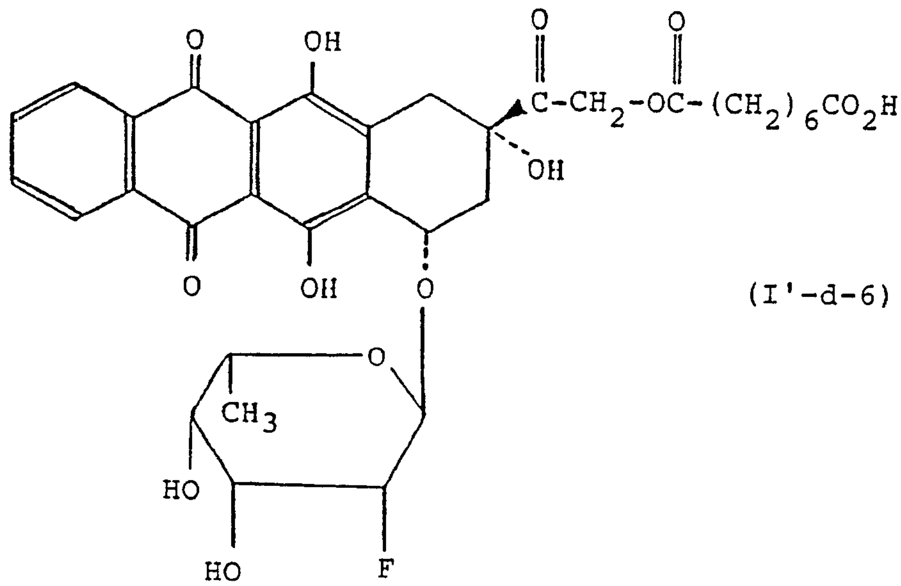

(I'-d-6)

The compound of the formula (I′-e-2) (83 mg) as obtained in the Referential Example 4 and mono-sodium suberate (460 mg) were used as the starting materials and reacted with each other and the resulting reaction solution containing the condensation product was after-treated by the same procedure as in the Example 14, so that the corresponding hemi-ester compound, namely 4-demethoxy-7-O-(2,6-dideoxy-2-fluoro-3,4-O-isopropylidene-α-L-talopyranosyl)adriamycinone 14- O-hemisuberate (yield; 61 mg, 64%) was obtained. This hemiester compound was treated with 80% aqueous acetic acid in the same manner as in the Example 14 to effect the hydrolysis reaction for the removal of the 3′,4′-O-isopropylidene group from the hemiester compound. The titled compound was obtained as an orange solid (yield; 47 mg, 52%).

$[\alpha]_D^{24}$ + 107° [c 0.1, chloroform-methanol (1:1)].

The captioned 14-O-hemisuberate is scarcely soluble in the phosphate buffer solution at pH 7.4 which is described in the Example 18.

## Claims

1. An anthracycline derivative represented by the formula

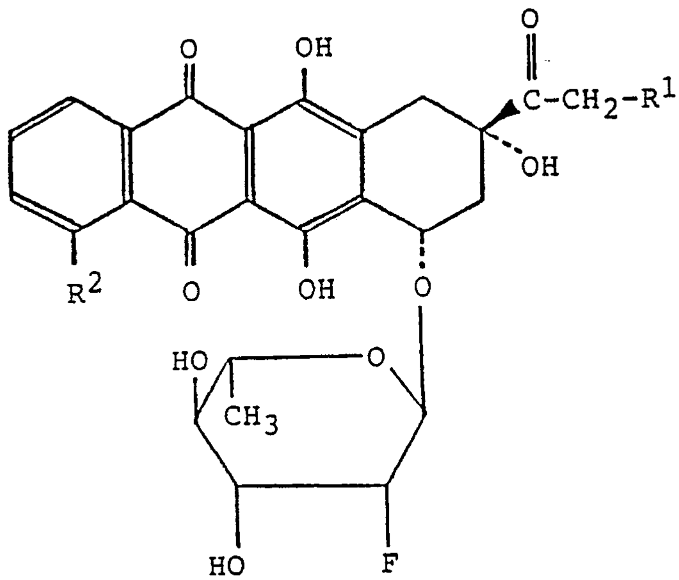

(I)

wherein $R^1$ is a hydrogen atom or a hydroxyl group or is a group having the formula

$-O\overset{\overset{O}{\|}}{C}-(CH_2)_n-COOH$

where n denotes an integer of 1 to 10, and $R^2$ is a methoxy group or a hydrogen atom, or a salt thereof.

2. The compound as claimed in Claim 1, which is a daunomycin derivative represented by the formula

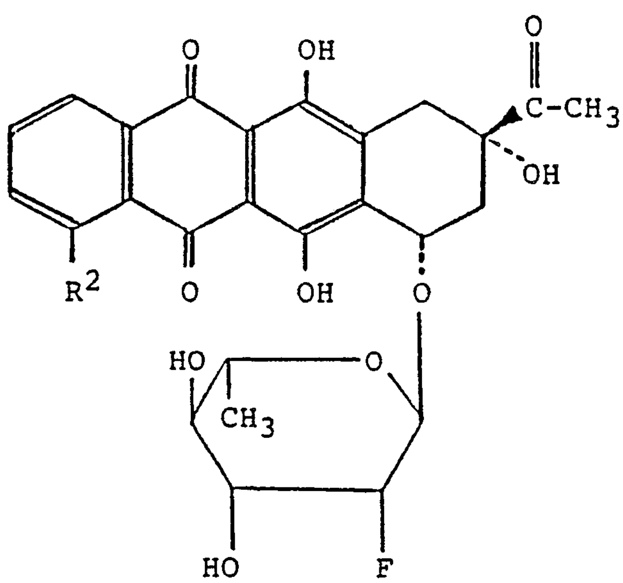

(Ia)

wherein $R^2$ is a methoxy group or a hydrogen atom.

3. The compound as claimed in Claim 1, which is an adriamycin derivative represented by the formula

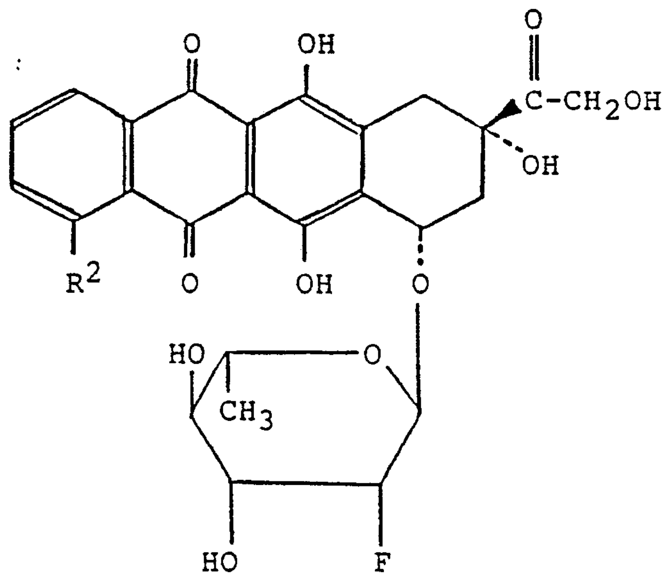

(Ib)

wherein $R^2$ is a methoxy group or a hydrogen atom.

4. The compound as claimed in Claim 1, which is an adriamycin half-ester derivative represented by the formula

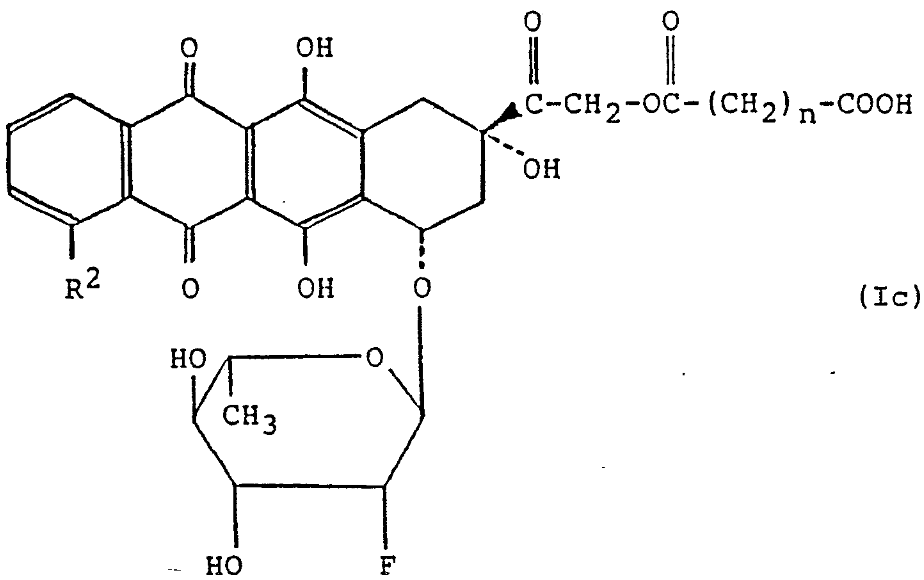

(Ic)

wherein $R^2$ is a methoxy group or a hydrogen atom and n is an integer of 1 to 10, or a salt thereof.

5. A compound as claimed in Claim 1 or 2 which is selected from 7-O-(2,6-dideoxy-2-fluoro-α-L-mannopyranosyl)daunomycinone and 7-O-(2,6-dideoxy-2-fluoro-α-L-mannopyranosyl)-4-demethoxydaunomycinone.

6. A compound as claimed in Claim 1 or 3 which is selected from 7-O-(2,6-dideoxy-2-fluoro-α-L-mannopyranosyl)adriamycinone and 7-O-(2,6-dideoxy-2-fluoro-α-L-mannopyranosyl)-4-demethoxyadriamycinone.

7. A compound as claimed in Claim 1 or 4 which is 7-O-(2,6-dideoxy-2-fluoro-α-L-mannopyranosyl)-adriamycinone 14-O-hemipimelate.

8. A compound as claimed in Claim 1 or 4 which is 7-O-(2,6-dideoxy-2-fluoro-α-L-mannopyranosyl)-4-demethoxyadriamycinone 14-O-hemipimelate.

9. A process for the preparation of a daunomycin derivative represented by the formula

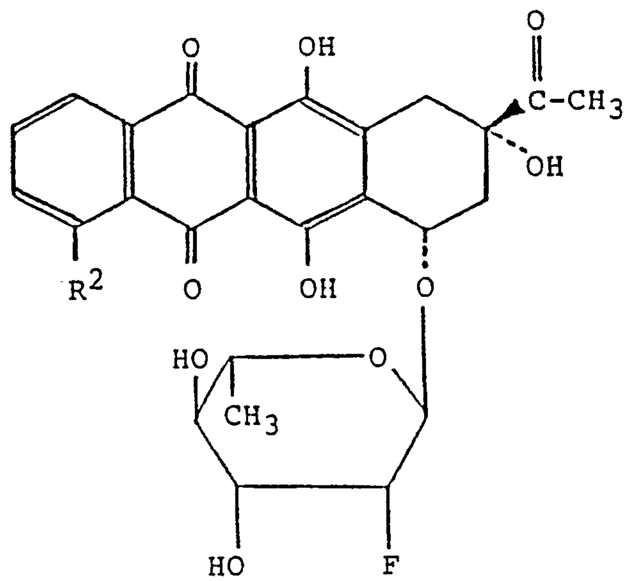

(Ia)

wherein $R^2$ is a methoxy group or a hydrogen atom, which comprises condensing daunomycinone or 4-demethoxydaunomycinone represented by the formula

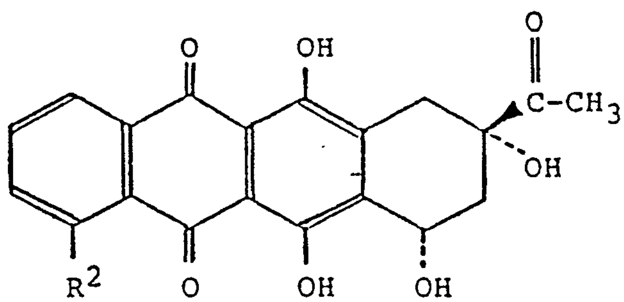

(II)

wherein $R^2$ has the same meaning as defined above, with a 2,6-dideoxy-2-fluoro-α-L-mannopyranosyl halide or a hydroxyl-protected derivative thereof represented by the formula

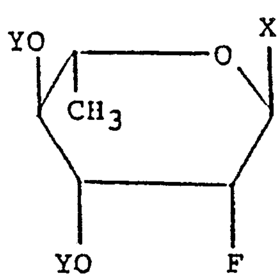

wherein X is a bromine, chlorine or iodine atom and Y is a hydrogen atom or a hydroxyl-protecting group, to form a compound represented by the formula

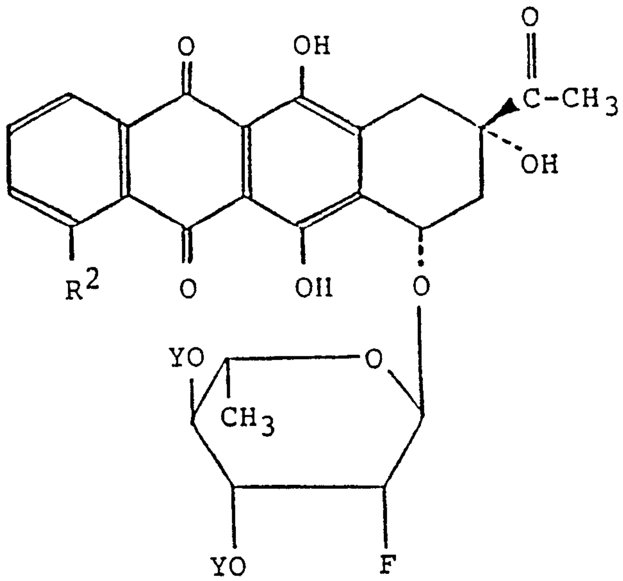

(Ia-1)

wherein $R^2$ and Y have the same meanings as defined above, and where the compound of the formula (Ia-1) contains the remaining hydroxyl-protecting groups (Y), then removing the hydroxyl-protecting groups (Y) from the compound of the formula (Ia-1) in a known manner.

10. A process for the preparation of an adriamycin derivative represented by the formula

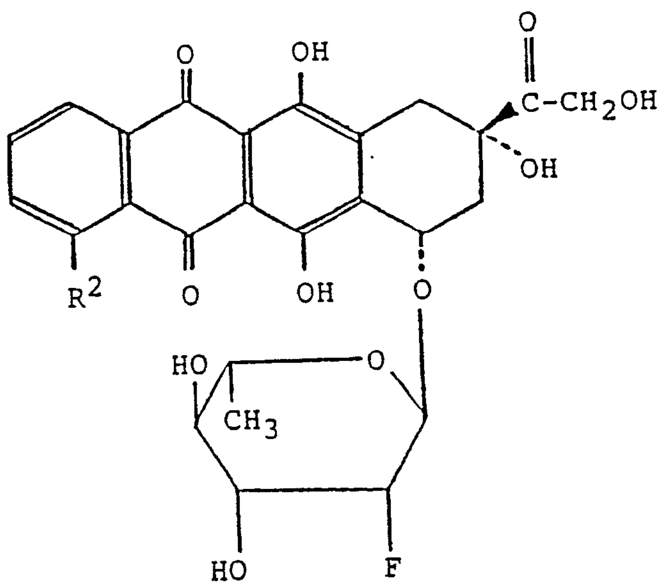

(Ib)

wherein $R^2$ is a methoxy group or a hydrogen atom, which comprises hydrolyzing a halomethyl group ($-CH_2-W$) of a 7-O-(2,6-dideoxy-2-fluoro-α-L-mannopyranosyl)-14-halodaunomycinone or -14-halo-4-demethoxydaunomycinone represented by the formula

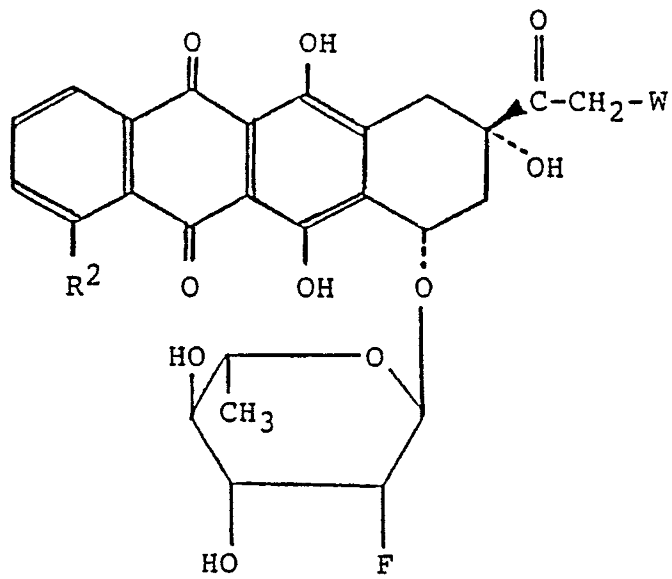

(Id)

wherein $R^2$ has the same meaning as defined above and W is a bromine, chlorine or iodine atom.

11. A process for the preparation of an adriamycin half-ester derivative represented by the formula

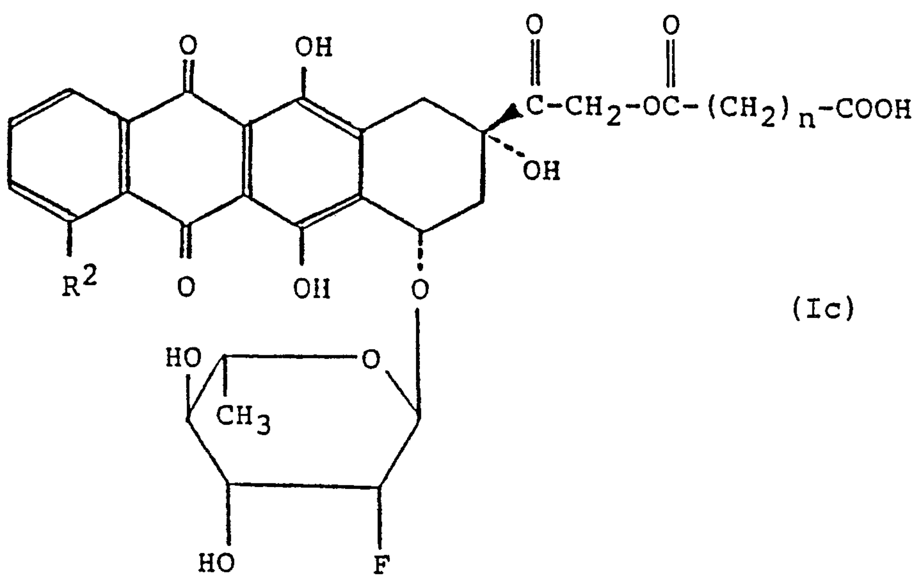

(Ic)

wherein $R^2$ is a methoxy group or a hydrogen atom and n denotes an integer of 1 to 10, which comprises reacting a 7-O-(2,6-dideoxy-2-fluoro-α-L-mannopyranosyl)-14-halodaunomycinone or -14-halo-4-demethoxydaunomycinone derivative represented by the formula

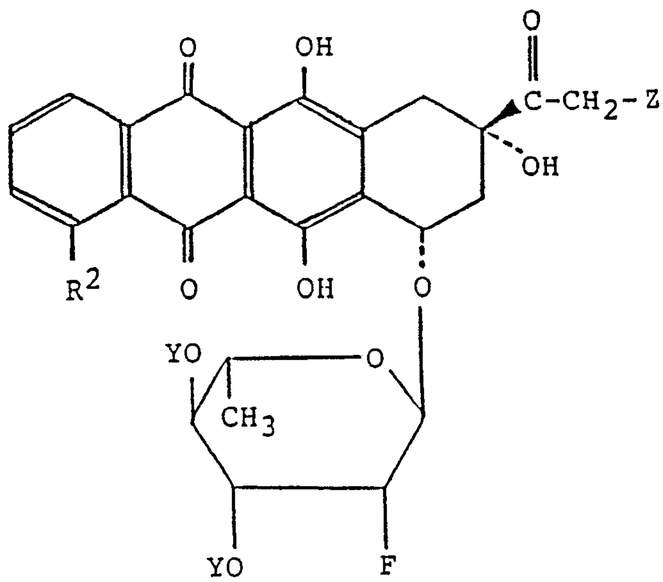

(Ie)

wherein $R^2$ has the same meaning as defined above, Z is a bromine, chlorine or iodine atom and Y is a hydrogen atom or a hydroxyl-protecting group, with a mono-alkali metal salt of an aliphatic dicarboxylic acid, represented by the formula

$A\text{-}OOC\text{-}(CH_2)_n\text{-}COOH$ (IV)

wherein A is an alkali metal atom and n has the same meaning as defined above, to form a compound of the formula

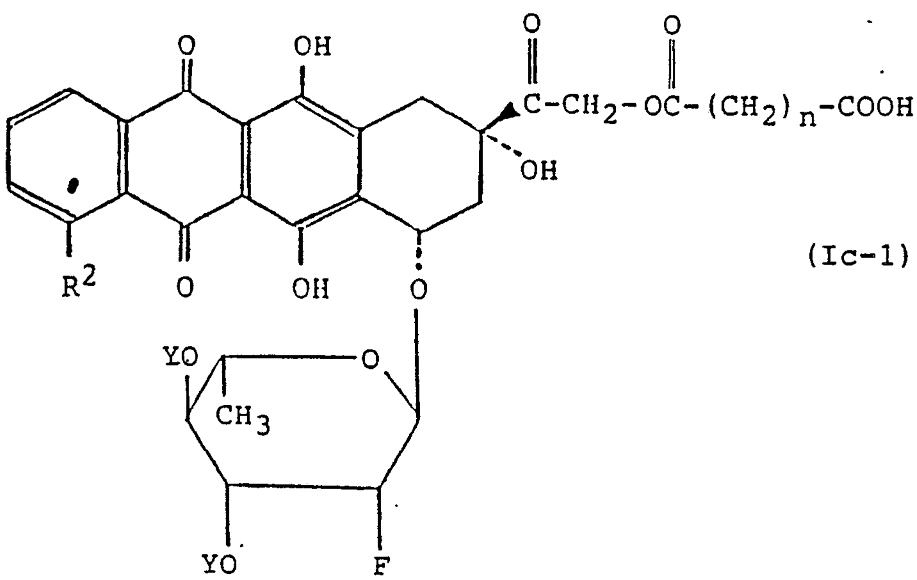

(Ic-1)

wherein $R^2$, Y and n have the same meanings as defined above, and where the compound of the formula (Ic-1) contains the remaining hydroxyl-protecting groups (Y), then removing the hydroxyl-protecting groups (Y) form the compound of the formula (Ic-1) in a known manner.

12. A pharmaceutical composition comprising as an active ingredient an anthracycline derivative as defined in Claim 1 and represented by the formula

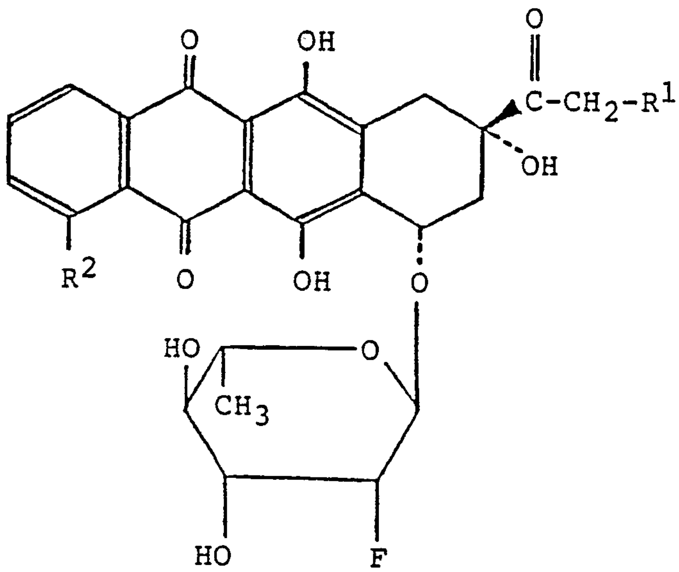

(I)

wherein $R^1$ is a hydrogen atom or a hydroxyl group or is a group having the formula

$-O\overset{\overset{O}{\|}}{C}-(CH_2)_n-COOH$

where n denotes an integer of 1 to 10, and $R^2$ is a methoxy group or a hydrogen atom, or a salt thereof.

13. An anthracycline derivative having the formula

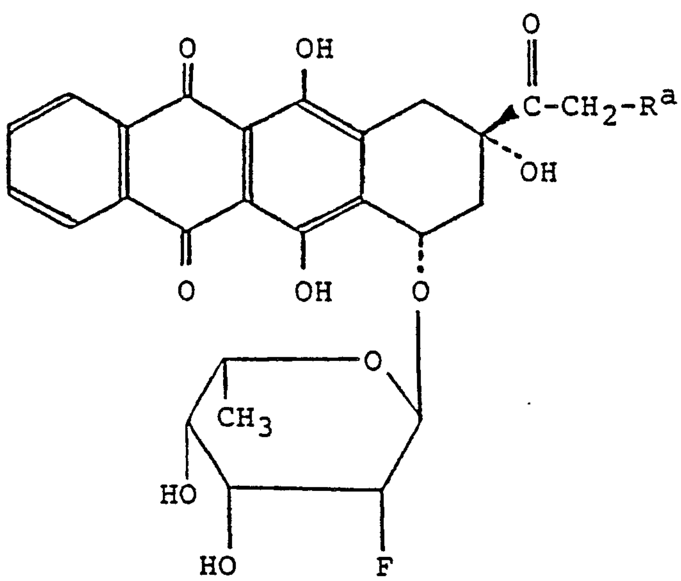

(I')

wherein $R^a$ is a hydrogen atom or a hydroxyl group or is a group of the formula

$-O\overset{\overset{O}{\|}}{C}-(CH_2)_p-H$

where p denotes an integer of 1 to 6, or is a group of the formula

$-O\overset{\overset{O}{\|}}{C}-(CH_2)_q-COOH$

where q denotes an integer of 1 to 6, or a salt thereof.

14. The compound as claimed in Claim 13, which is a 4-demethoxydaunomycin analogue having the formula

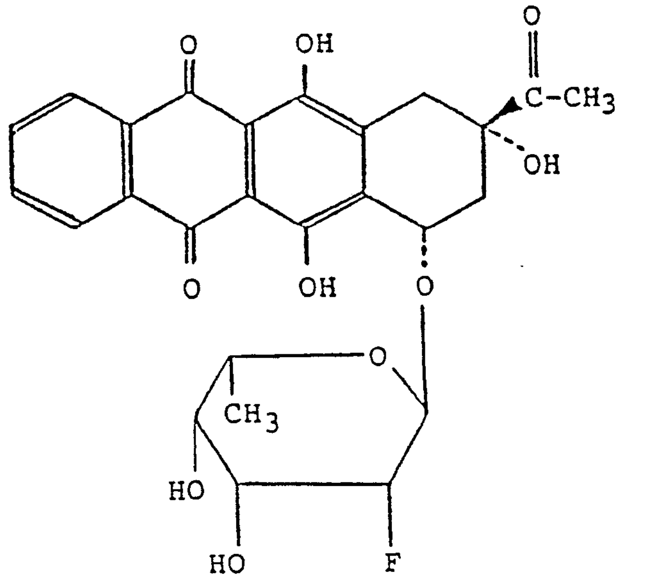

(I'-a)

namely, 4-demethoxy-7-O-(2,6-dideoxy-2-fluoro-α-L-talopyranosyl)daunomycinone.

15. The compound as claimed in Claim 13, which is a 4-demethoxyadriamycin analogue having the formula

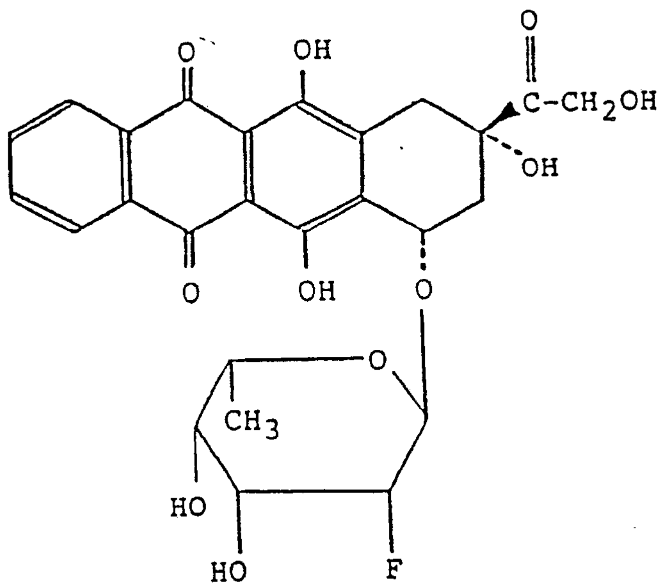

(I'-b)

namely, 4-demethoxy-7-O-(2,6-dideoxy-2-fluoro-α-L-talopyranosyl)adriamycinone.

16. The compound as claimed in Claim 13, which is an ester derivative of 4-demethoxyadriamycin analogue having the formula

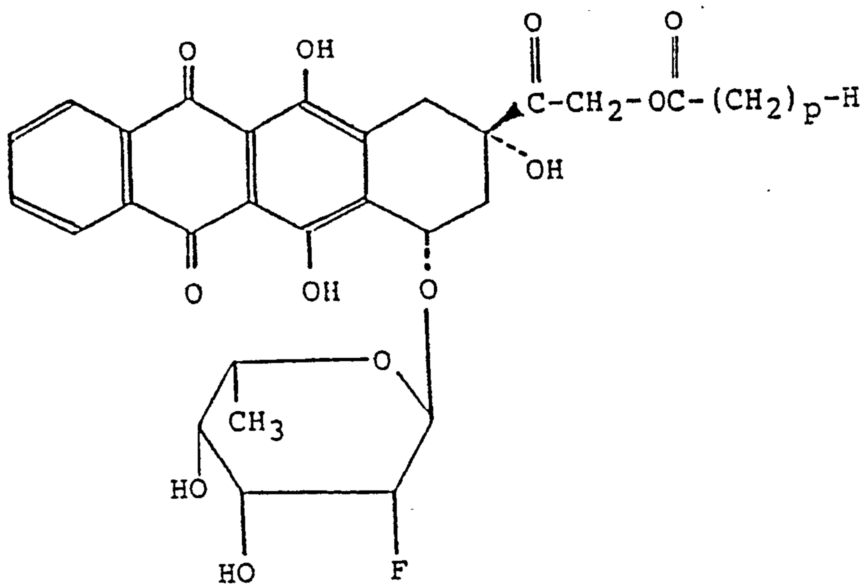

(I'-c)

where p denotes an integer of 1 to 6.

17. The compound as claimed in Claim 13, which is a half-ester derivative of 4-demethoxyadriamycin analogue having the formula

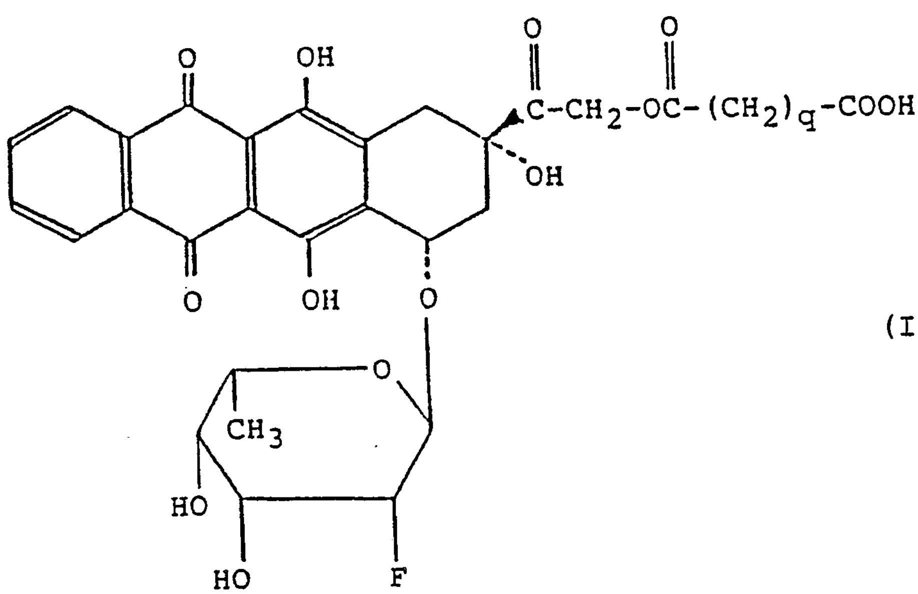

(I'-d)

where q denotes an integer of 1 to 6, or a salt thereof.

18. A compound as claimed in Claim 13 or 17 which is 4-demethoxy-7-O-(2,6-dideoxy-2-fluoro-α-L-talopyranosyl) adriamycinone 14-O-hemiadipate.

19. A compound as claimed in Claim 13 or 17 which is 4-demethoxy-7-O-(2,6-dideoxy-2-fluoro-α-L-talopyranosyl) adriamycinone 14-O-hemipimelate.

20. A compound as claimed in Claim 13 or 16 which is 4-demethoxy-7-O-(2,6-dideoxy-2-fluoro-α-L-talopyranosyl) adriamycinone 14-O-valerate.

21. A process for the preparation of a 4-demethoxydaunomycin analogue having the formula

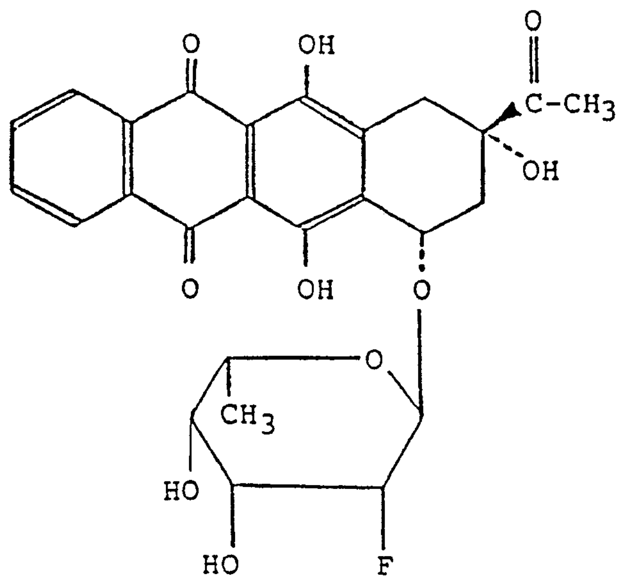

(I'-a)

which comprises condensing 4-demethoxydaunomycinone of the formula

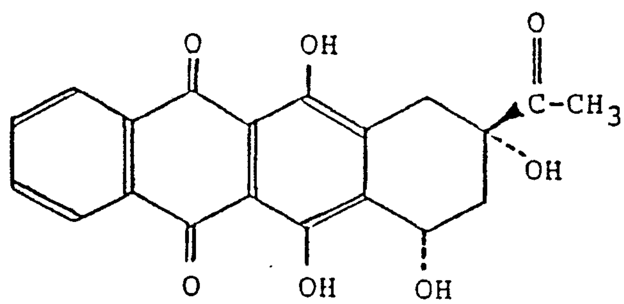

(II')

with a 2,6-dideoxy-2-fluoro-α-L-talopyranosyl halide or a hydroxyl-protected derivative thereof having the formula

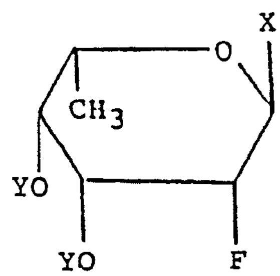

(III")

wherein X is a bromine, chlorine or iodine atom and Y is a hydrogen atom or a hydroxyl-protecting group, to produce a compound of the formula

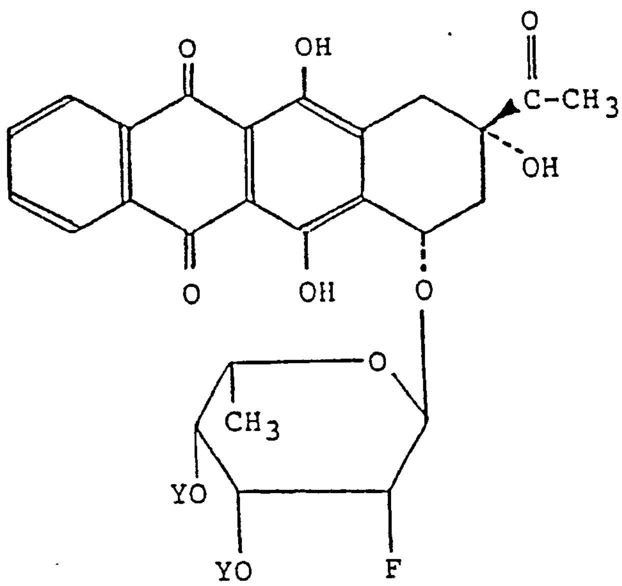

(I'-a-1)

wherein Y is as defined above, and where the compound of the formula (I'-a-1) contains the remaining hydroxyl-protecting groups (Y), then removing the hydroxyl-protecting groups (Y) from the compound of the formula (I'-a-1) in a known manner.

22. A process for the preparation of a 4-demethoxyadriamycin analogue having the formula

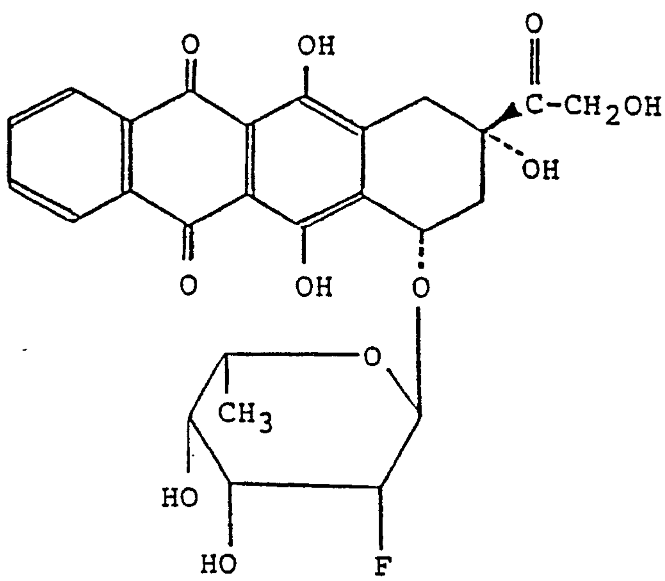

(I'-b)

which comprises hydrolyzing a halomethyl group ($-CH_2-W$) of a 4-demethoxy-7-O-(2,6-dideoxy-2-fluoro-α-L-talopyranosyl)-14-halo-daunomycinone derivative having the formula

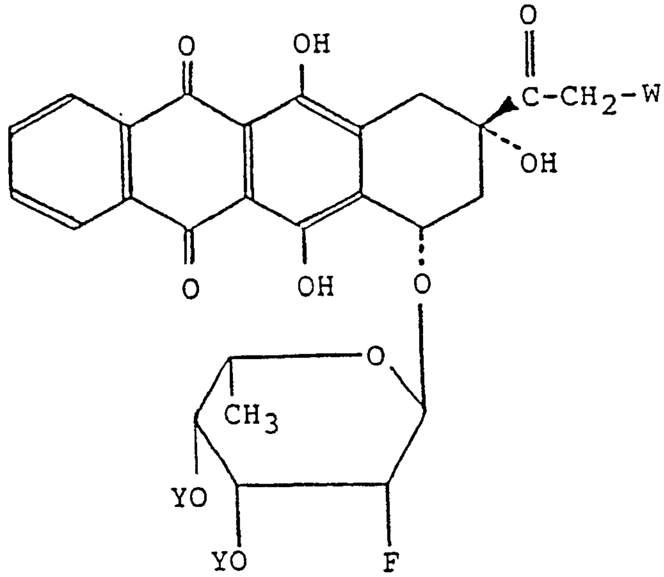

(I'-e)

wherein W is a bromine, chlorine or iodine atom and Y is a hydrogen atom or a hydroxyl-protecting group, to produce a compound of the formula

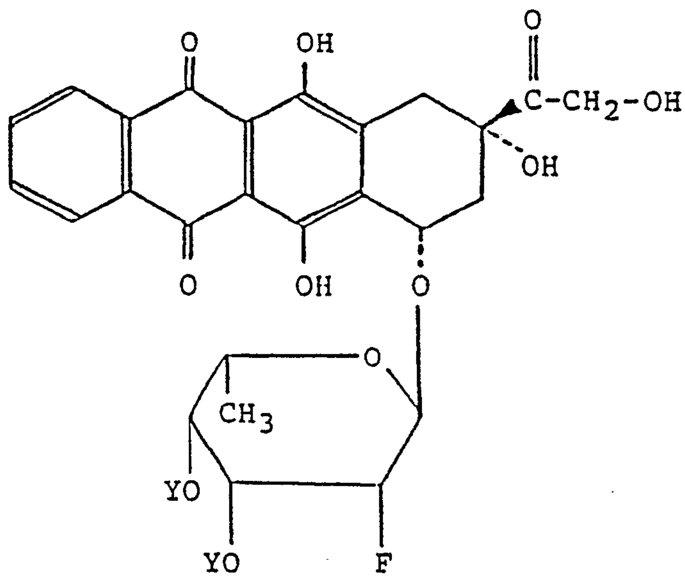

(I'-b-1)

wherein Y is as defined above, and where the compound of the formula (I'-b-1) contains the remaining hydroxyl-protecting groups (Y), then removing the hydroxyl-protecting groups (Y) from the compound of the formula (I'-b-1) in a known manner.

23. A process for the preparation of an ester derivative of a 4-demethoxyadriamycin analogue having the formula

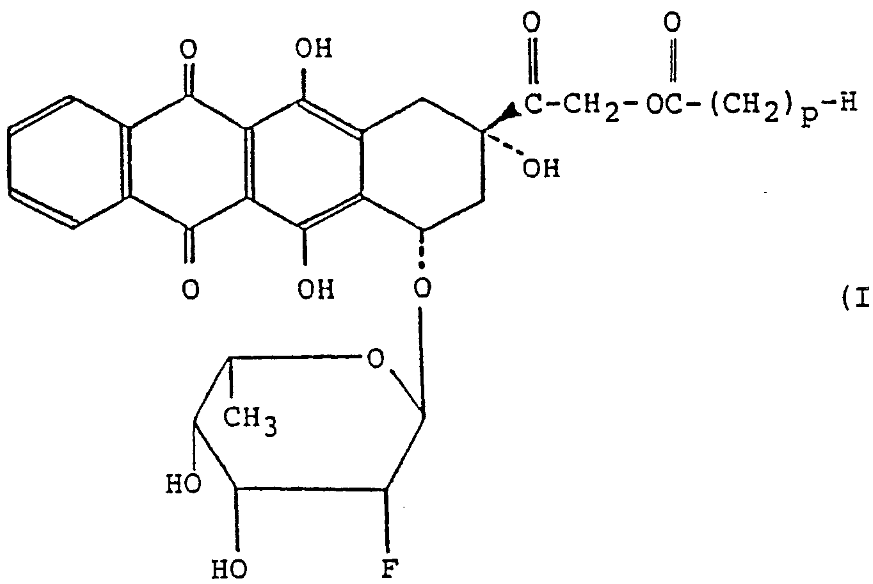

(I'-c)

wherein p denotes an integer of 1 to 6, which comprises reacting a 4-demethoxy-7-O-(2,6-dideoxy-2-fluoro-α-L-talopyranosyl)-14-halodaunomycinone derivative having the formula

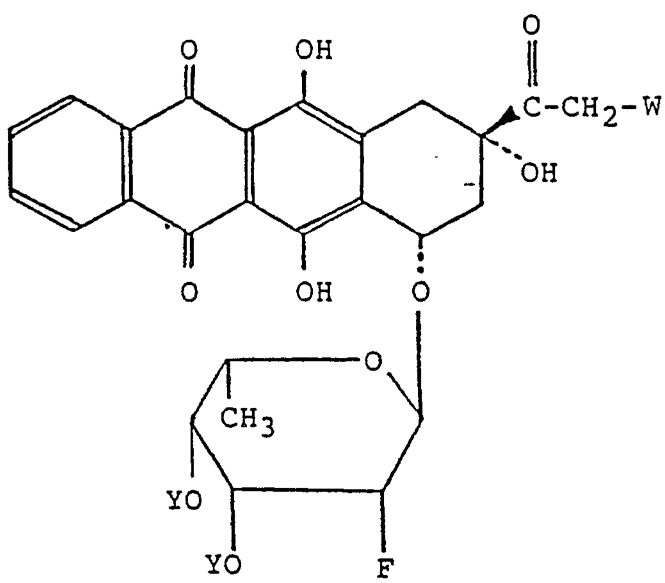

(I'-e)

wherein W is a bromine, chlorine or iodine atom and Y is a hydrogen atom or a hydroxyl-protecting group, with an alkali metal salt of an aliphatic carboxylic acid having the formula

$A\text{-}OOC\text{-}(CH_2)_p\text{-}H$ (IV')

wherein A is an alkali metal atom and p denotes an integer of 1 to 6, to produce a compound of the formula

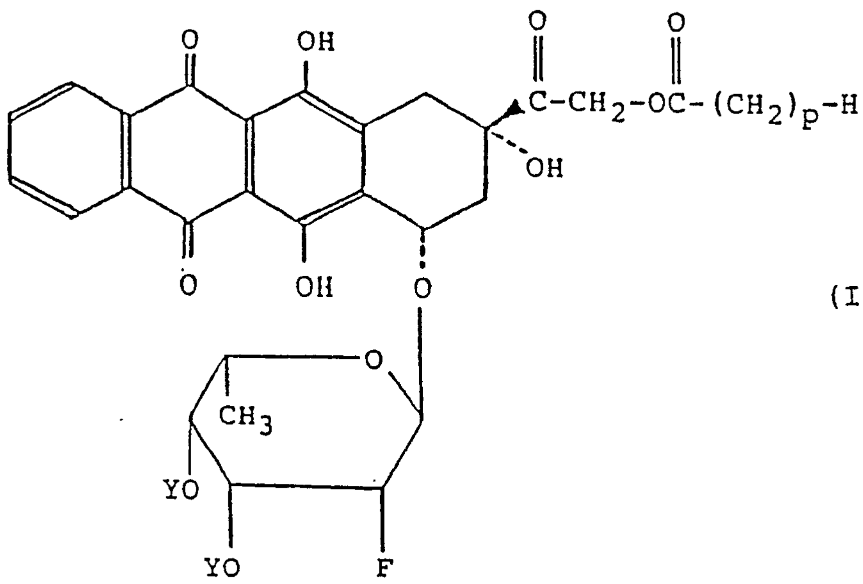

(I'-c-1)

wherein Y and p are as defined above, and where the compound of the formula (I'-c-1) contains the remaining hydroxyl-protecting groups (Y), then removing the hydroxyl-protecting groups (Y) from the compound of the formula (I'-c-1) in a known manner.

24. A process for the preparation of a half-ester derivative of 4-demethoxyadriamycin analogue having the formula

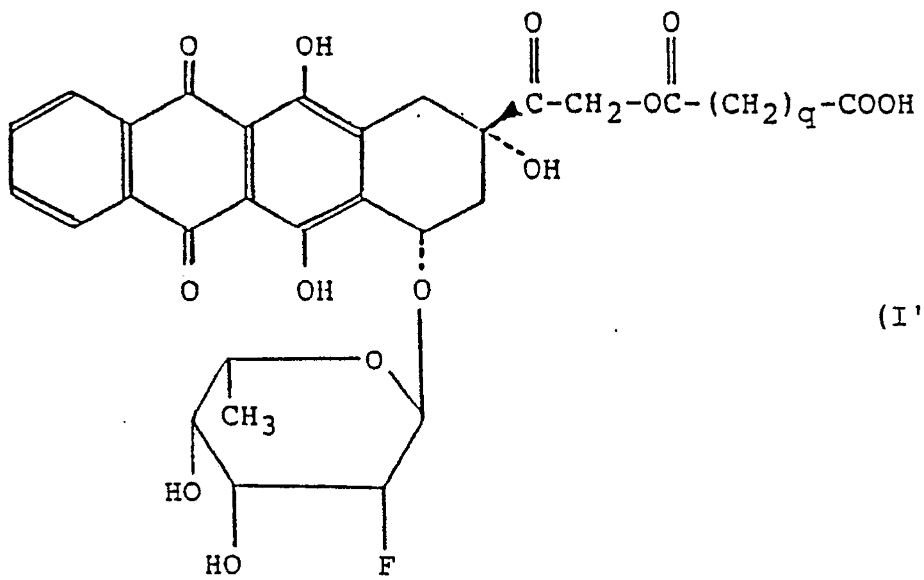

(I'-d)

wherein q denotes an integer of 1 to 6, which comprises reacting a 4-demethoxy-7-O-(2,6-dideoxy-2-fluoro-α-L-talopyranosyl)-14-halodaunomycinone derivative having the formula

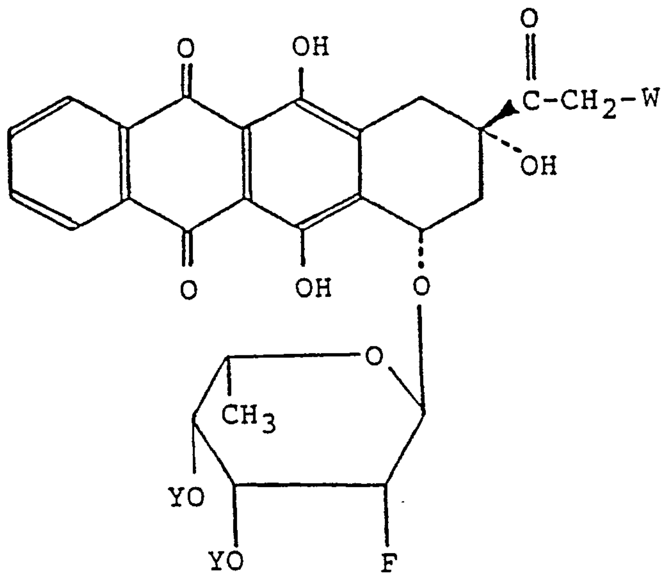

(I'-e)

wherein W is a bromine, chlorine or iodine atom and Y is a hydrogen atom or a hydroxyl-protecting group, with a mono-alkali metal salt of an aliphatic dicarboxylic acid having the formula

$A\text{-}OOC\text{-}(CH_2)_q\text{-}COOH$ (IV″)

wherein A is an alkali metal atom and q denotes an integer of 1 to 6, to produce a compound of the formula

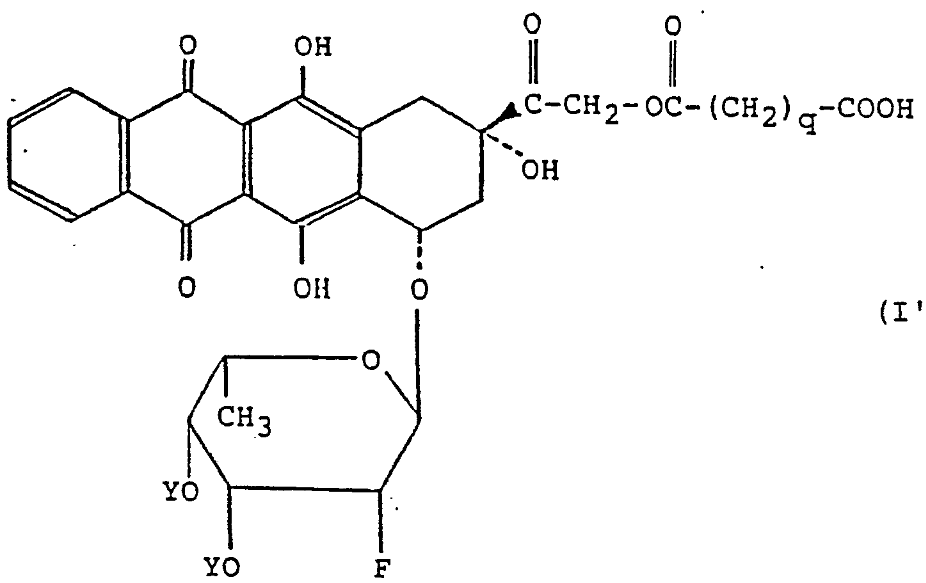

(I'-d-1)

wherein Y and q are as defined above, and where the compound of the formula (I′-d-1) contains the remaining hydroxyl-protecting groups (Y), then removing the hydroxyl-protecting groups (Y) from the compound of the formula (I′-d-1) in a known manner.

25. A pharmaceutical composition comprising an anthracycline derivative having the formula (I′) as claimed and defined in Claim 13, or a salt thereof, as the active ingredient.

26. A method of treating a tumor cell in a mammal, including human, which comprises administering an amount of a compound of the formula (I) or a compound of the formula (I′) as defined hereinbefore to a mammal bearing the tumor cell, with the amount of the compound administered being effective to suppress the growth of the tumor cell.